(19) Europäisches Patentamt
European Patent Office
Office européen des brevets

(11) EP 2 687 515 A1

(12) **EUROPEAN PATENT APPLICATION**
published in accordance with Art. 153(4) EPC

(43) Date of publication:
**22.01.2014 Bulletin 2014/04**

(21) Application number: **12758005.8**

(22) Date of filing: **15.03.2012**

(51) Int Cl.:
***C07D 231/20*** (2006.01) ***C07C 241/02*** (2006.01)
***C07C 243/16*** (2006.01)

(86) International application number:
**PCT/JP2012/056763**

(87) International publication number:
**WO 2012/124781 (20.09.2012 Gazette 2012/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2011 JP 2011058817**

(71) Applicant: **Shionogi & Co., Ltd.**
**Osaka-shi, Osaka 541-0045 (JP)**

(72) Inventors:
- **UENAKA, Masaaki**
  **Toyonaka-shi**
  **Osaka 561-0825 (JP)**
- **YONEZAWA, Shuji**
  **Toyonaka-shi**
  **Osaka 561-0825 (JP)**
- **IIDA, Akira**
  **Amagasaki-shi**
  **Hyogo 660-0813 (JP)**
- **TAKAKI, Mutsumi**
  **Amagasaki-shi**
  **Hyogo 660-0813 (JP)**
- **OGAWA, Tomoyuki**
  **Toyonaka-shi**
  **Osaka 561-0825 (JP)**
- **GODA, Satoshi**
  **Amagasaki-shi**
  **Hyogo 660-0813 (JP)**
- **HIROSE, Yoshikatsu**
  **Amagasaki-shi**
  **Hyogo 660-0813 (JP)**
- **FUKUDA, Tomohiro**
  **Toyonaka-shi**
  **Osaka 561-0825 (JP)**

(74) Representative: **HOFFMANN EITLE**
**Patent- und Rechtsanwälte**
**Arabellastrasse 4**
**81925 München (DE)**

# (54) METHOD FOR PRODUCING PYRAZOLECARBOXYLIC ACID DERIVATIVE

(57) Disclosed is a process for producing a pyrazole carboxylic acid derivative which is useful as a significant intermediate of an 11βHSD-1 inhibitor.

A compound represented by the Formula (XI):

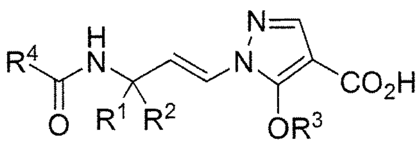

(XI)

is useful as a significant intermediate of an 11βHSD-1 inhibitor,
wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

EP 2 687 515 A1

## Description

[Field of the Invention]

The present invention relates to a process for producing a pyrazole carboxylic acid derivative and an 11βHSD-1 inhibitor (11β-hydroxysteroid dehydrogenase type I inhibitor) using the above derivative.

[Background Art]

A pyrazole carboxylic acid derivative is a useful compound as a pharmaceutical synthesis material or an intermediate. For example, the derivative can be used as a synthetic intermediate of a compound represented by the Formula (XIII):

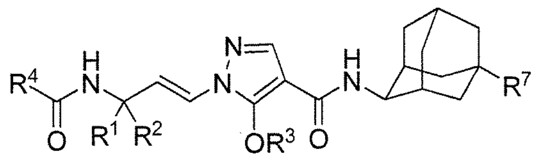

(XIII)

, its salt, or a solvate thereof.

The compound represented by the Formula (XIII) has 11βHSD-1 inhibitory activity, and a pharmaceutical composition comprising the compound is known to be useful as a therapeutic agent for type II diabetes (Patent Document 1 and Patent Document 2).

Example 30 of Patent Document 1 discloses the following process as a method for producing a side chain at the 1-position of the pyrazole ring of the above compound.

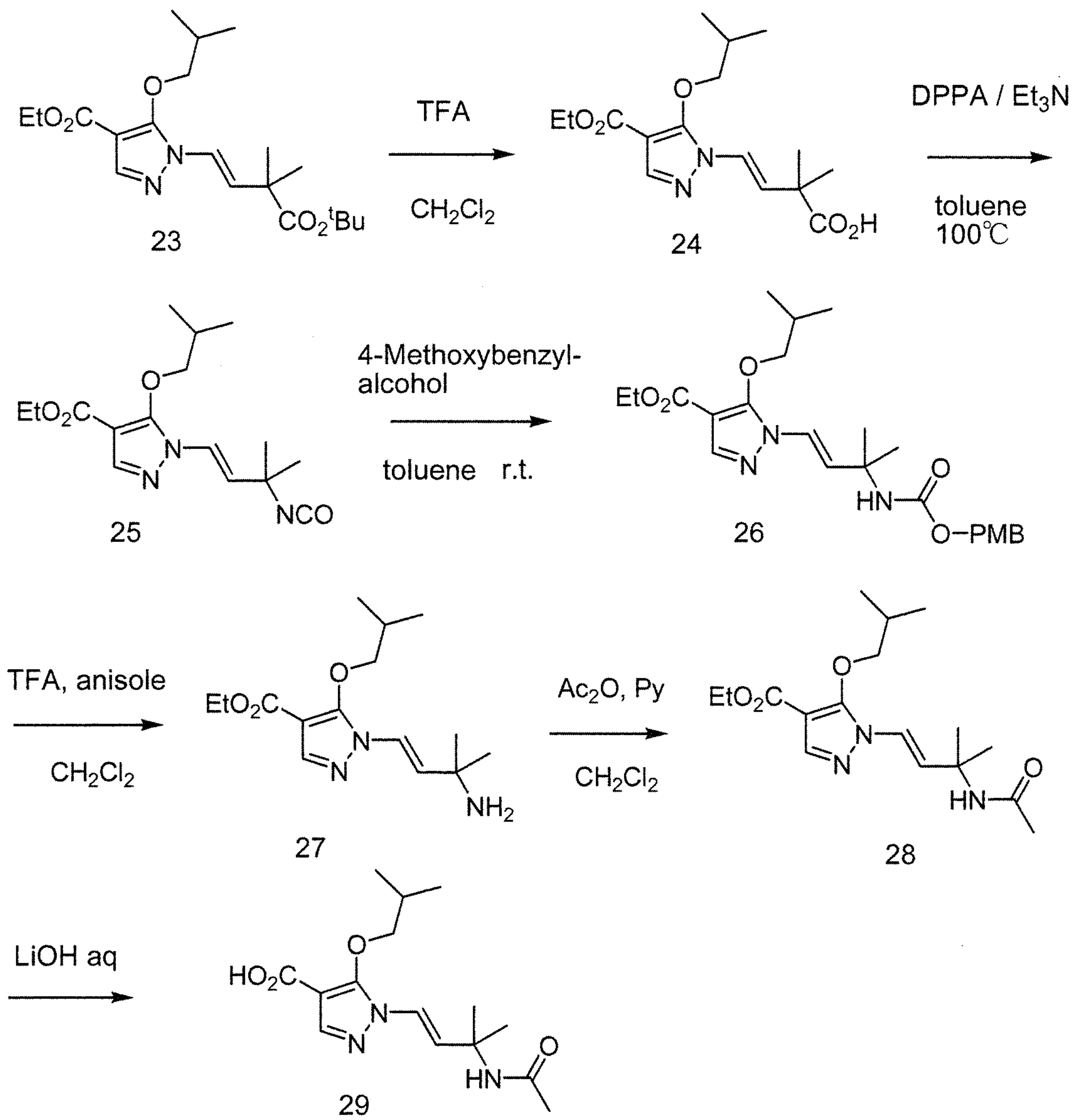


23 24 25 26 27 28 29

Example 29 of Patent Document 1 discloses the following process as a method for producing a side chain at the 1-position of the pyrazole ring of the above compound 23.

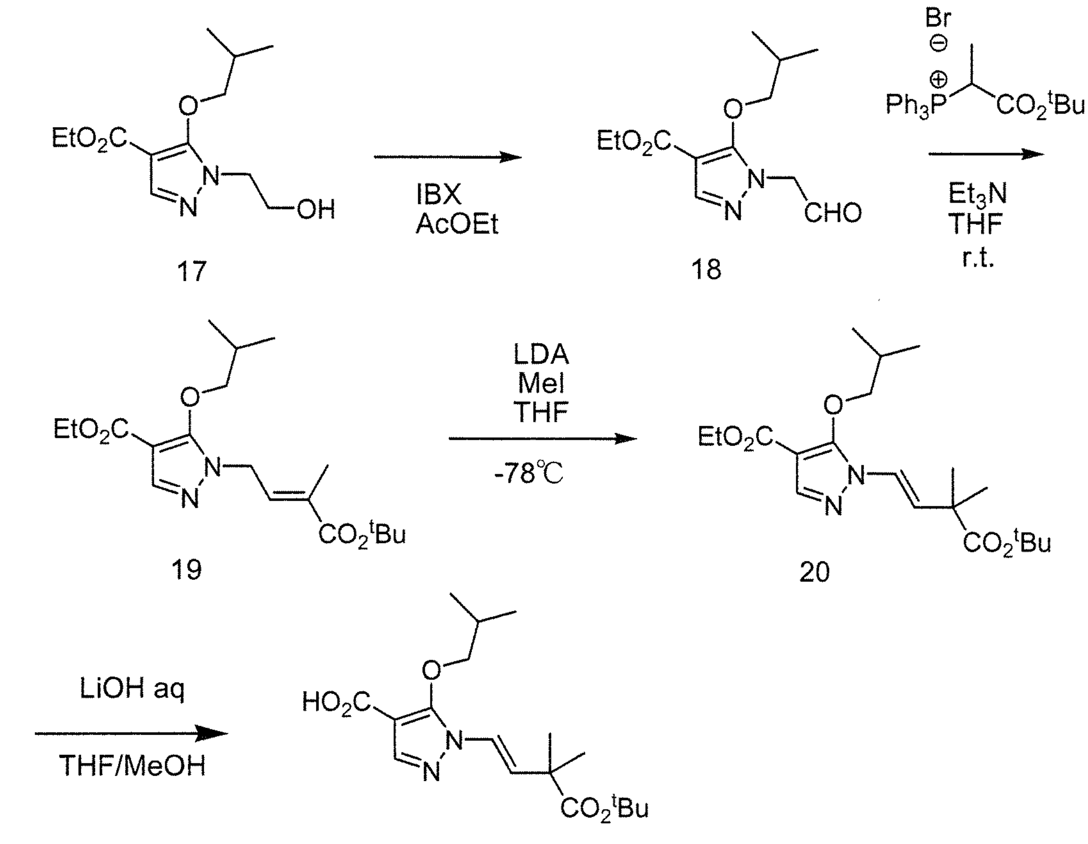


17 18 19 20 21

The method described in Patent Document 1 requires multisteps for producing a side chain at the 1-position of the pyrazole ring of the above compound represented by the Formula (XIII). Moreover, the method requires three times purification with column chromatography and low-temperature processing for producing a side chain, and requires 2-iodoxybenzoic acid (IBX) which is explosive, trifluoroacetic acid (TFA) which is corrosive and the like as a reagent, and thus the method is industrially difficult to apply.

Example 86 of Patent Document 2 discloses the following process as a method for producing a side chain at the 1-position of the pyrazole ring of the above compound represented by the Formula (XIII).

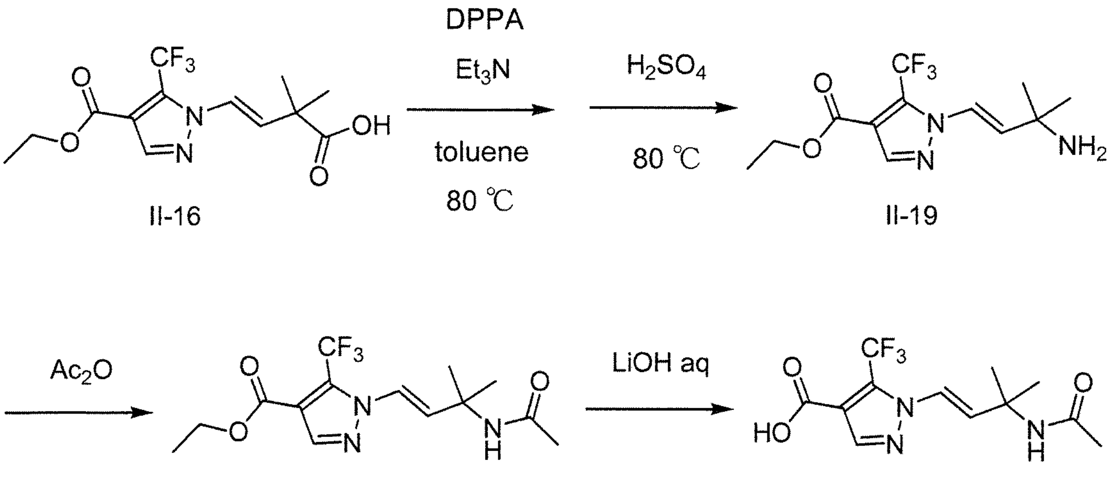


II-16 II-19 II-20 II-21

Furthermore, example 8 of Patent Document 1 discloses the following process as a method for producing a pyrazole ring.

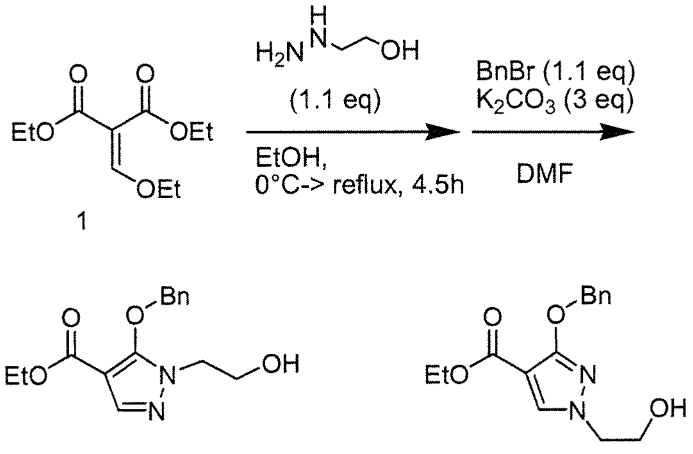


1

8

8'

Non Patent Document 1 discloses the following process as a method for the haloamidation of cyclohexene.

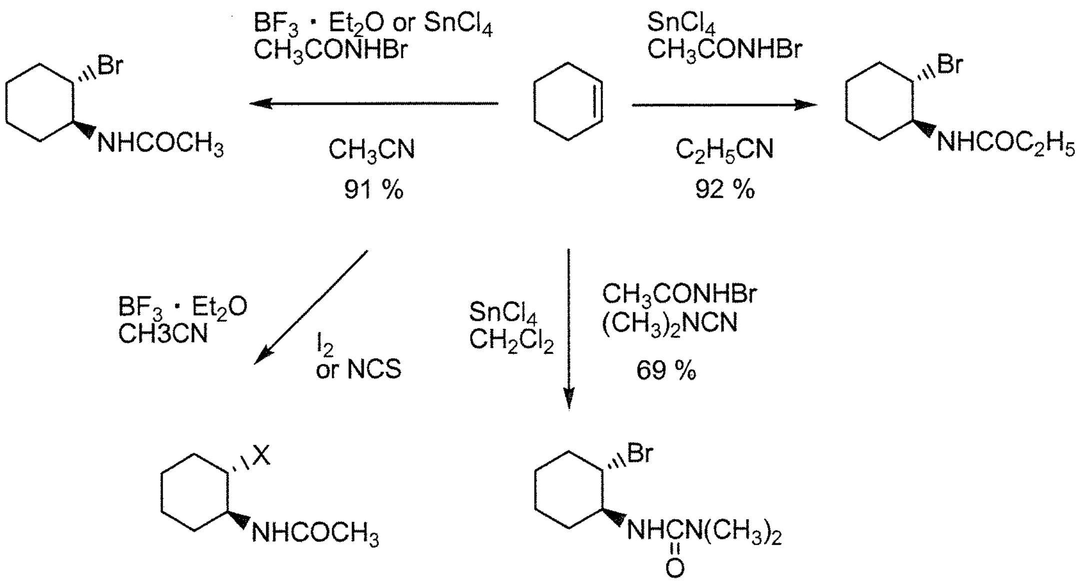


X = I or Cl

Moreover, Non Patent Document 1 discloses a process for producing an oxazoline ring by the haloamidation of cyclohexene, then reacting with triethylamine.

However, any documents do not describe a process for preparing a pyrazole carboxylic acid derivative of the present invention and a compound represented by the Formula (XIII) using the above derivative.

[Prior Art Document]

[Patent Document]

Patent Document 1: WO2007/058346
Patent Document 2: WO2008/142986

[Non-patent Document]

Non Patent Document 1: Journal of the American Chemical Society 2006, 128, 9644-9645

[Disclosure of Invention]

[Problems to be solved by the Invention]

The present invention provides an efficient process for producing a pyrazole carboxylic acid derivative represented by the Formula (XI):

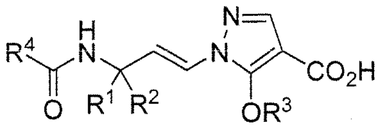

(XI)

, which is useful as a pharmaceutical synthetic material or an intermediate.

More specifically, the present invention provides a process for producing a pyrazole carboxylic acid derivative which is a useful intermediate in efficiently preparing a compound represented by the Formula (XIII):

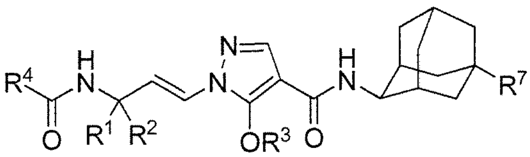

(XIII)

or its salt.

Moreover, the present invention provides a process for producing a compound represented by the Formula (XIII) or its salt using the intermedi ate.

[Means for Solving the Problem]

The inventors of the present invention found that, as an efficient process for producing a pyrazole carboxylic acid derivative, a pyrazole carboxylic acid derivative represented by the Formula (XI):

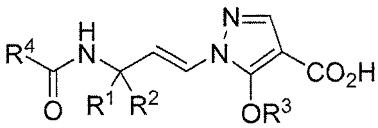

(XI)

could be efficiently produced, by way of the haloamidation of a compound represented bv the Formula (VI):

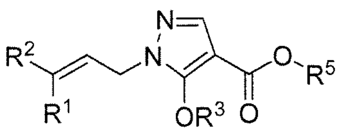

(VI)

, followed by reacting with a base to obtain a compound represented by the  Formula (IX):

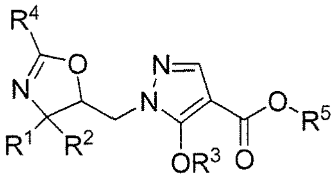

(IX)

, followed by reacting the obtained compound represented by the Formula (IX) with a base to obtain a compound represented by the Formula (X):

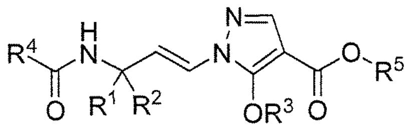

(X)

, followed by hydrolyzing.

Moreover, the inventors of the present invention found that a compound represented by the Formula (X-1) was obtained as a by-product in a step for obtaining a compound represented by the Formula (X) by reacting a compound represented by the Formula (IX) with a base as shown below.

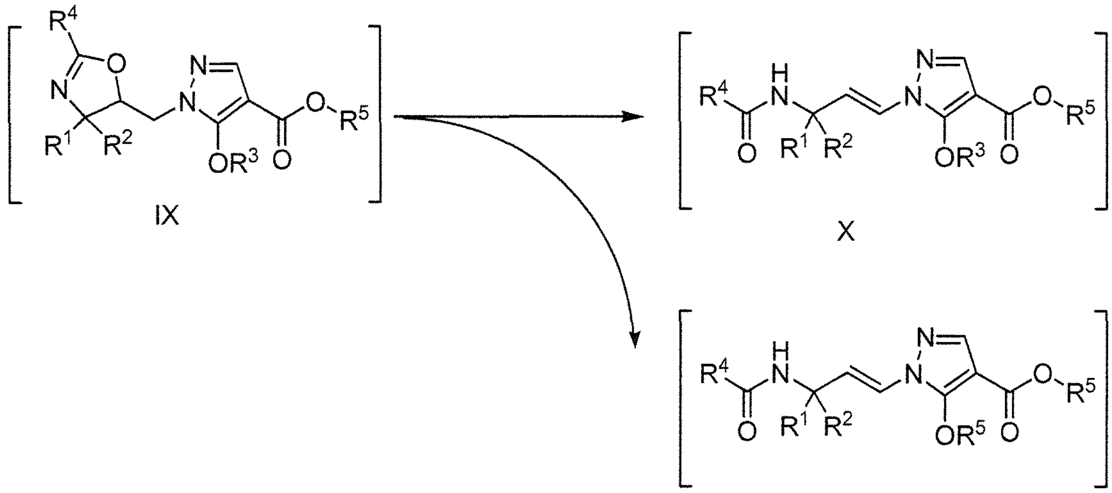

IX

X

X-1

Furthermore, the inventors of the present invention found that a compound represented by the Formula (XI) could be efficiently produced by making $R^3$ and $R^5$ into the same group in the above step. By making $R^3$ and $R^5$ into the same group, even if $-OR^3$ group is replaced with $-OR^5$ group at the 5-position of the pyrazole ring, the obtained compound represented by the Formula (X-1) can be set to the same compound as a compound represented by the Formula (X).

Concretely, a compound represented by the Formula (VI):

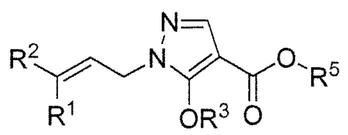

(VI)

, wherein $R^3$ is the same group as $R^5$, can be produced by hydrolyzing a compound represented by the Formula (VI):

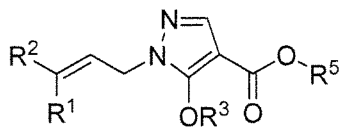

(VI)

, wherein $R^3$ is not the same group as $R^5$, to obtain a compound represented by the Formula (XV):

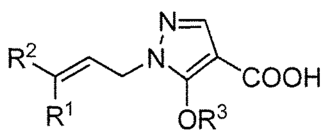

(XV)

, followed by reacting the obtained compound represented by the Formula (XV) with a compound represented by the Formula (XVII): $R^5$-OH, wherein $R^3$ is the same group as $R^5$.

Furthermore, a compound represented by the Formula (XI):

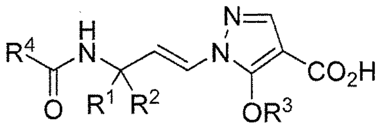

(XI)

can be produced, by way of the haloamidation of a compound represented by the Formula (VI):

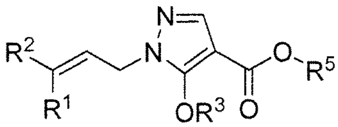

(VI)

, wherein $R^3$ is the same group as $R^5$,
followed by reacting with a base to obtain a compound represented by the Formula (IX):

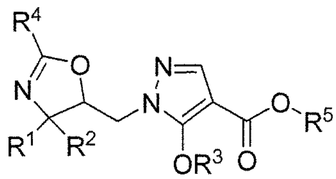

(IX)

, wherein $R^3$ is the same group as $R^5$,
followed by reacting the obtained compound represented by the Formula (IX) with a base to obtain a compound represented by the Formula (X):

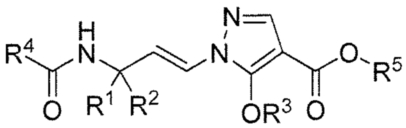

(X)

, wherein $R^3$ is the same group as $R^5$,
followed by hydrolyzing.

Furthermore, the present inventors have found that a compound represented by the Formula (XIII):

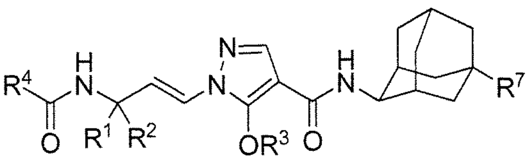

(XIII)

can be efficiently produced by reacting a compound represented by the Formula (XI):

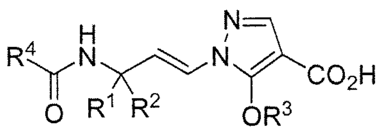

(XI)

with a compound represented by the Formula (XII):

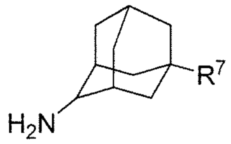

(XII)

.

The process described in the prior art document is to produce a compound represented by the Formula (XVIII):

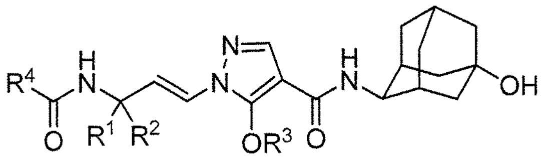

(XVIII)

followed by modifying a hydroxyl group on an adamantane skeleton.

This process requires further reaction of a compound represented by the Formula (XVIII) produced by multisteps as material, thus it may prove to be uneconomical depending on the yield of the modifying reaction to be followed and the number of steps involved to prepare the end object.

Moreover, a compound represented by the Formula (XVIII) has low solubility and solvents that can be used are limited. Tetrahydrofuran (THF) is an example of a solvent capable of dissolving a compound represented by the Formula (XVIII), but it was discovered that there was a problem that chlorosulfonyl isocyanate (CSI) used to modify the hydroxyl group on the adamantane skeleton reacted with THF to form a polymer.

The inventors found that the compound represented by the Formula (XIII) can be efficiently produced by first synthesizing an adamantane amine derivative having an intended group, followed by reacting the derivative with a compound represented by the Formula (XI):

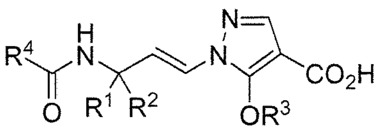

(XI)

as described in the present invention.

A compound represented by the Formula (XII):

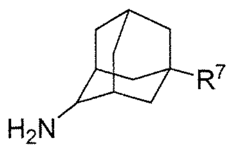

(XII)

can be synthesized according to a method described in WO2011/078101 and WO2012/020724.

The inventors of the present invention completed the following invention.

(1) A process for producing a compound represented by the Formula (VIII):

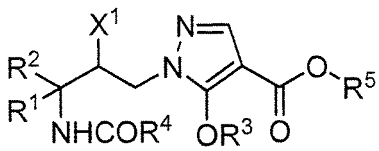

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $X^1$ is halogen;
which comprises reacting a compound represented by the Formula (VI):

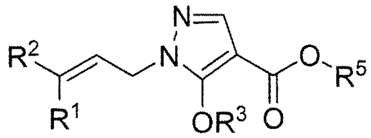
(VI)

wherein $R^1$, $R^2$, $R^3$ and $R^5$ are as defined above; with a compound represented by the Formula (VII): $R^4$-CN, wherein $R^4$ is as defined above; in the presence of a halogenating agent.
(2) The process according to the above (1), wherein the halogenating agent is N-halogenosuccinimide, N-halogenoacetamide or halogen.
(3) The process according to the above (2), wherein the halogenating agent is N-bromosuccinimide, N-bromoacetamide, N-chlorosuccinimide or $I_2$.
(4) The process according to any one of the above (1) to (3), wherein the reaction is performed in the presence of a Lewis acid.
(5) The process according to the above (4), wherein the Lewis acid is boron trifluoride ether complex or metal halide.
(6) The process according to the above (5), wherein the Lewis acid is boron trifluoride n-butyl ether complex, boron trifluoride diethyl ether complex or SnCl4.
(7) A process for producing a compound represented by the Formula (IX):

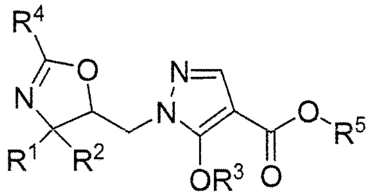
(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (VIII):

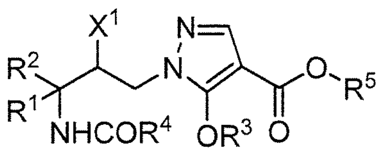
(VIII)

wherein $R^1$, $R^2$, $R^3$, $R^4$ and $R^5$ are as defined above and $X^1$ is halogen; with a base.
(8) The process according to the above (7), wherein the base is an organic base.
(9) The process for producing the compound represented by the Formula (IX) according to the above (7) or its salt, wherein the process comprises a process according to any one of the above (1) to (6).
(10) A process for producing a compound represented by the Formula (X):

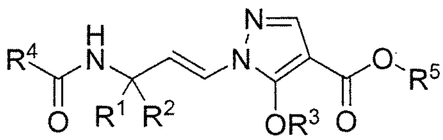
(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (IX):

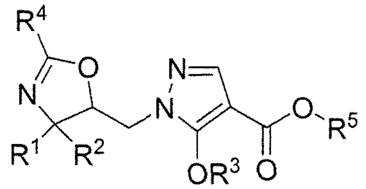
(IX)

wherein $R^1$, $R^2$, $R^3$, $R^4$ and $R^5$ are as defined above; with a base.
(11) The process according to the above (10), wherein the base is a metal alkoxide.
(12) The process for producing the compound represented by the Formula (X) according to the above (10) or its salt, wherein the process comprises a process according to any one of the above (1) to (9).
(13) A process for producing a compound represented by the Formula (XI):

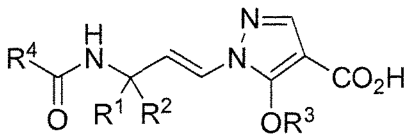
(XI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (X):

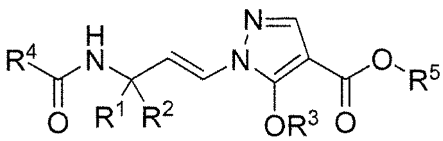
(X)

wherein $R^1$, $R^2$, $R^3$ and $R^4$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.
(14) A process for producing a compound represented by the Formula (XV):

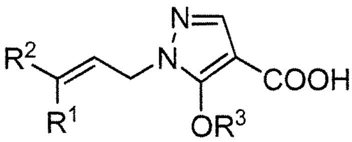
(XV)

or its salt, wherein $R^1$ and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (VI):

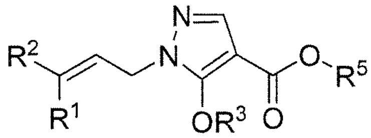

(VI)

wherein $R^1$, $R^2$ and $R^3$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, provided that $R^3$ and $R^5$ are not the same group.
(15) A process for producing a compound represented by the Formula (VI):

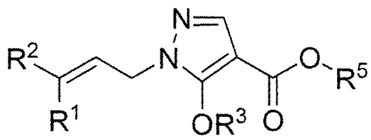

(VI)

or its salt, wherein $R^1$ and $R^2$ are each independently hydrogen or substituted or unsubstituted alkyl and $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (XV):

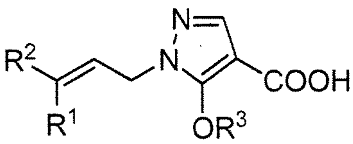

(XV)

wherein $R^1$, $R^2$ and $R^3$ are as defined above; with a compound represented by the Formula (XVII): $R^5$-OH, wherein $R^5$ is the same group as $R^3$.
(16) The process according to the above (15), which comprises a step of producing a compound represented by the formula (XVI):

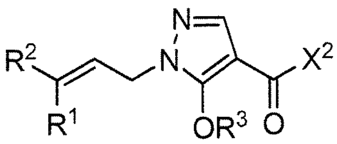

(XVI)

or its salt, wherein $R^1$, $R^2$ and $R^3$ are as defined in the above (15) and $X^2$ is halogen; by reacting a compound represented by the formula (XV):

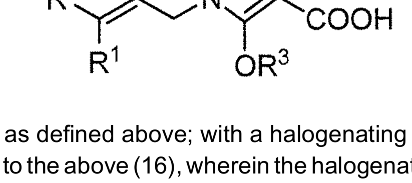

(XV)

wherein $R^1$, $R^2$ and $R^3$ are as defined above; with a halogenating agent.
(17) The process according to the above (16), wherein the halogenating agent is selected from phosphorus oxyhalide, phosphorus pentahalide, oxalyl halide and thionyl halide.
(18) The process for producing the compound represented by the Formula (VI) according to the above (15) or its

salt, wherein the process comprises a process according to the above (14).

(19) The process for producing the compound represented by the Formula (XI) according to the above (13) or its salt, wherein the process comprises a process according to any one of the above (1) to (18).

(20) A process for producing a compound represented by the Formula (III):

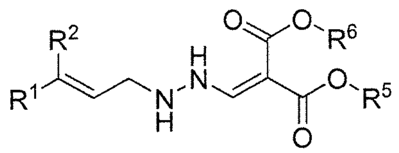

(III)

or its salt, wherein $R^1$ and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (I):

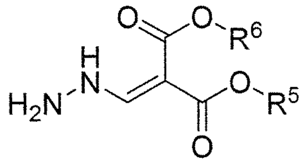

(I)

wherein $R^5$ and $R^6$ are as defined above; with a compound represented by the Formula (II):

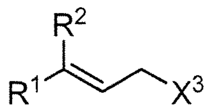

(II)

wherein $R^1$ and $R^2$ are as defined above and $X^3$ is a leaving group.

(21) A process for producing a compound represented by the Formula (IV):

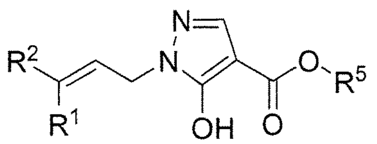

(IV)

or its salt, wherein $R^1$ and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (III):

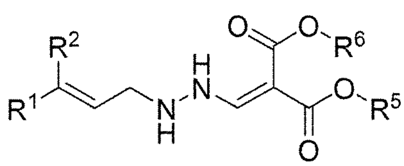

(III)

wherein $R^1$, $R^2$ and $R^5$ are as defined above and $R^6$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl; with a base.

(22) The process for producing the compound represented by the Formula (IV) according to the above (21) or its

salt, wherein the process comprises a process according to the above (20).

(23) A process for producing a compound represented by the Formula (VI):

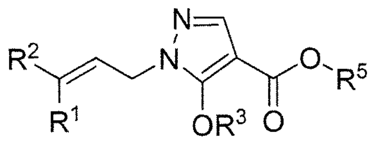

(VI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

which comprises reacting a compound represented by the Formula (IV):

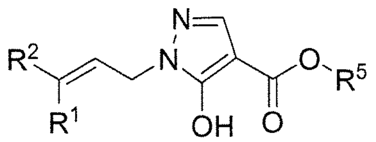

(IV)

wherein $R^1$, $R^2$ and $R^5$ are as defined above; with a compound represented by the Formula (V): $R^3$-$X^4$, wherein $R^3$ is as defined above and $X^4$ is a leaving group; in the presence of a base.

(24) The process for producing the compound represented by the Formula (VI) according to the above (23) or its salt, wherein the process comprises a process according to any one of the above (20) to (22).

(25) The process according to the above (19), wherein the process comprises a process according to the above (24).

(26) The process according to any one of the above (1) to (25), wherein $R^1$ and $R^2$ are substituted or unsubstituted alkyl.

(27) The process according to any one of the above (1) to (19) or (23) to (25), wherein $R^3$ is substituted or unsubstituted alkyl.

(28) The process according to any one of the above (1) to (19) or (23) to (25), wherein $R^3$ and $R^5$ are the same substituted or unsubstituted alkyl.

(29) A process for producing a compound represented by the Formula (XIII):

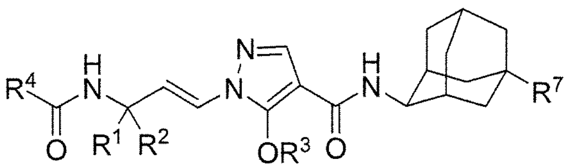

(XIII)

or its salt, wherein $R^1$, $R^2$, $R^3$ and $R^4$ are as defined in the above (13), $R^7$ is

a group represented by the Formula: -$OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

wherein the process comprises a process according to any one of the above (1) to (28).

(30) A process for producing a compound represented by the Formula (XIII):

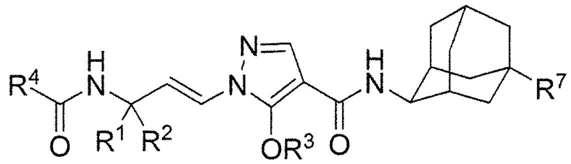

(XIII)

or its salt, wherein $R^1$, $R^2$, $R^3$ and $R^4$ are as defined in the above (13), $R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

which comprises obtaining a compound represented by the Formula (XI):

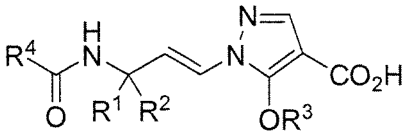

(XI)

wherein $R^1$, $R^2$, $R^3$ and $R^4$ are as defined above; by the process according to any one of the above (1) to (28), followed by reacting the obtained compound represented by the Formula (XI) with a compound represented by the Formula (XII):

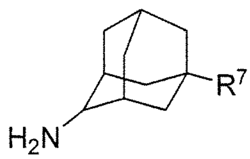

(XII)

wherein $R^7$ is as defined above.

(31) The process according to the above (30), wherein the reaction is performed in the presence of a condensing agent.

(32) The process according to the above (31), wherein the condensing agent is one or more condensing agent(s) selected from N,N'-dicyclohexylcarbodiimide, N,N'-diisopropylcarbodiimide and 1-ethyl-3-(3-dimethylamino propyl) carbodiimide hydrochloride.

(33) The process according to the above (31) or (32), wherein the reaction is performed in the presence of one or more additive agent(s) selected from 1-hydroxybenzotriazole and N-hydroxy succinimide.

(34) The process according to the above (30), which comprises a step of producing a compound represented by the formula (XIV):

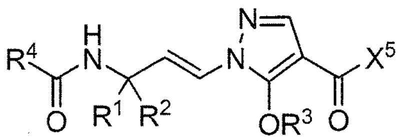

(XIV)

or its salt, wherein $R^1$, $R^2$, $R^3$ and $R^4$ are as defined in the above (13) and $X^5$ is halogen; by reacting a compound represented by the formula (XI):

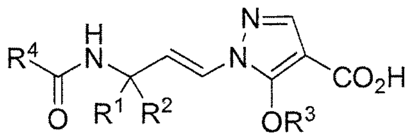

(XI)

wherein $R^1$, $R^2$, $R^3$ and $R^4$ are as defined above; with a halogenating agent.

(35) The process according to the above (34), wherein the halogenating agent is selected from phosphorus oxyhalide, phosphorus pentahalide, oxalyl halide and thionyl halide.

(36) A compound represented by the Formula (III):

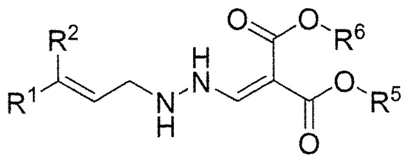

(III)

or its salt, wherein $R^1$ and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(36-1) The compound according to the above (36) or its salt, wherein $R^1$ and $R^2$ are methyl, and $R^5$ and $R^6$ are each independently alkyl.

(36-2) The compound according to the above (36-1) or its salt, wherein $R^5$ and $R^6$ are ethyl.

(37) A compound represented by the Formula (IV):

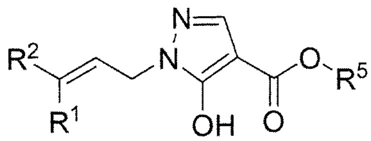

(IV)

or its salt, wherein $R^1$ and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(37-1) The compound according to the above (37) or its salt, wherein $R^1$ and $R^2$ are methyl, and $R^5$ is alkyl.

(37-2) The compound according to the above (37-1) or its salt, wherein $R^5$ is ethyl.

(37-3) The crystal of a compound according to the above (37-2) or its salt, wherein the values of 2θ of the powder X-ray diffraction have two or more 2θ selected from 8.2 ± 0.2, 12.4 ± 0.2, 18.3 ± 0.2, 24.5 ± 0.2 and 25.6 ± 0.2 degrees.

(38) A compound represented by the Formula (VI):

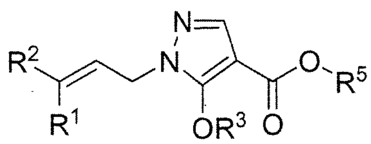

(VI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(38-1) The compound according to the above (38) or its salt, wherein $R^1$ and $R^2$ are methyl, and $R^3$ and $R^5$ are each

independently alkyl.

(38-2) The compound according to the above (38-1) or its salt, wherein $R^3$ is ethyl, n-propyl, isopropyl or isobutyl.

(38-3) The compound according to the above (38-1) or (38-2), or its salt, wherein $R^5$ is ethyl.

(38-4) The compound according to the above (38-1) or (38-2), or its salt, wherein $R^5$ is isopropyl.

(39) A compound represented by the Formula (VIII):

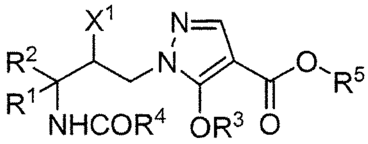

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $X^1$ is halogen.

(39-1) The compound according to the above (39) or its salt, wherein $R^1$, $R^2$ and $R^4$ are methyl, and $R^3$ and $R^5$ are each independently alkyl.

(39-2) The compound according to the above (39-1) or its salt, wherein $R^3$ is ethyl, n-propyl, isopropyl or isobutyl.

(39-3) The compound according to the above (39-1) or (39-2) or its salt, wherein $R^5$ is ethyl.

(39-4) The compound according to the above (39-1) or (39-2), or its salt, wherein $R^5$ is isopropyl.

(40) A compound represented by the Formula (IX):

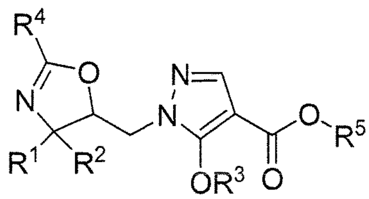

(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(40-1) The compound according to the above (40) or its salt, wherein $R^1$, $R^2$ and $R^4$ are methyl, and $R^3$ and $R^5$ are each independently alkyl.

(40-2) The compound according to the above (40-1) or its salt, wherein $R^3$ is ethyl, n-propyl, isopropyl or isobutyl.

(40-3) The compound according to the above (40-1) or (40-2), or its salt, wherein $R^5$ is ethyl.

(40-4) The compound according to the above (40-1) or (40-2), or its salt, wherein $R^5$ is isopropyl.

(41) A compound represented by the Formula (X):

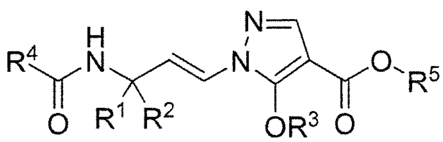

(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is sub-

stituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

(41-1) The compound according to the above (41) or its salt, wherein $R^1$, $R^2$ and $R^4$ are methyl, $R^3$ is isopropyl and $R^5$ is alkyl.

(41-2) The compound according to the above (41-1) or its salt, wherein $R^5$ is isopropyl.

(41-3) The crystal of a compound according to the above (41-2) or its salt, wherein the values of 2θ of the powder X-ray diffraction have two or more 2θ selected from 12.7 ± 0.2, 13.9 ± 0.2, 15.2 ± 0.2, 15.5 ± 0.2, 17.2 ± 0.2, 18.2 ± 0.2, 18.6 ± 0.2, 19.9 ± 0.2 and 20.2 ± 0.2 degrees.

(41-4) The compound according to the above (41-1) or its salt, wherein $R^5$ is ethyl.

(42) A compound represented by the Formula (XI):

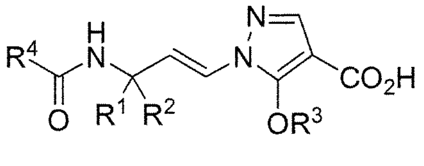

(XI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

(42-1) The compound according to the above (42) or its salt, wherein $R^1$, $R^2$ and $R^4$ are methyl, and $R^3$ is ethyl.

(42-2) The crystal of a compound according to the above (42-1) or its salt, wherein the values of 2θ of the powder X-ray diffraction have two or more 2θ selected from 9.0 ± 0.2, 13.0 ± 0.2, 13.9 ± 0.2, 15.0 ± 0.2, 15.6 ± 0.2, 18.9 ± 0.2, 22.0 ± 0.2, 22.3 ± 0.2, 23.1 ± 0.2, 24.3 ± 0.2, 25.0 ± 0.2 and 25.4 ± 0.2 degrees.

(42-3) The compound according to the above (42) or its salt, wherein $R^1$, $R^2$ and $R^4$ are methyl, and $R^3$ is n-propyl.

(42-4) The crystal of a compound according to the above (42-3) or its salt, wherein the values of 2θ of the powder X-ray diffraction have two or more 2θ selected from 8.5 ± 0.2, 11.4 ± 0.2, 14.2 ± 0.2, 18.1 ± 0.2, 19.4 ± 0.2, 21.1 ± 0.2, 23.9 ± 0.2 and 26.4 ± 0.2 degrees.

(42-5) The compound according to the above (42) or its salt, wherein $R^1$, $R^2$ and $R^4$ are methyl, and $R^3$ is isopropyl.

(42-6) The crystal of a compound according to the above (42-5) or its salt, wherein the values of 2θ of the powder X-ray diffraction have two or more 2θ selected from 8.7 ± 0.2, 11.3 ± 0.2, 14.3 ± 0.2, 15.0 ± 0.2, 17.5 ± 0.2, 19.4 ± 0.2, 21.1 ± 0.2, 22.7 ± 0.2, 24.3 ± 0.2, 24.8 ± 0.2 and 26.0 ± 0.2 degrees.

(42-7) The crystal of a compound according to the above (42-5) or its salt, wherein the values of 2θ of the powder X-ray diffraction have two or more 2θ selected from 12.6 ± 0.2, 13.7 ± 0.2, 15.3 ± 0.2, 17.9 ± 0.2, 20.1 ± 0.2, 20.6 ± 0.2, 21.2 ± 0.2, 23.5 ± 0.2, 25.4 ± 0.2 and 31.0 ± 0.2 degrees.

(42-8) The crystal of a compound according to the above (42-5) or its salt, wherein the values of 2θ of the powder X-ray diffraction have two or more 2θ selected from 8.7 ± 0.2, 9.4 ± 0.2, 11.3 ± 0.2, 14.3 ± 0.2, 16.3 ± 0.2, 18.9 ± 0.2, 20.2 ± 0.2, 23.1 ± 0.2, 27.7 ± 0.2 and 30.5 ± 0.2 degrees.

(42-9) The compound according to the above (42) or its salt, wherein $R^1$, $R^2$ and $R^4$ are methyl, and $R^3$ is isobutyl.

(42-10) The crystal of a compound according to the above (42-9) or its salt, wherein the values of 2θ of the powder X-ray diffraction have two or more 2θ selected from 10.6 ± 0.2, 11.5 ± 0.2, 17.6 ± 0.2, 18.0 ± 0.2, 18.6 ± 0.2, 19.5 ± 0.2, 19.9 ± 0.2, 23.1 ± 0.2, 24.8 ± 0.2, 27.5 ± 0.2 and 27.9 ± 0.2 degrees.

(43) A compound represented by the Formula (XV):

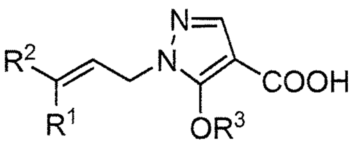

(XV)

or its salt, wherein $R^1$ and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(43-1) The compound according to the above (43) or its salt, wherein $R^1$ and $R^2$ are methyl, and $R^3$ is isopropyl.

(43-2) The crystal of a compound according to the above (43-1) or its salt, wherein the values of 2θ of the powder X-ray diffraction have 2θ of 12.0 ± 0.2 and 20.3 ± 0.2 degrees.

(44) A process for producing a crystal of a non-solvate of a compound represented by the Formula (XIII):

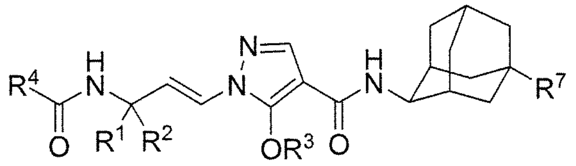

(XIII)

or a crystal of a non-solvate of a salt of the compound, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

which comprises crystallizing a compound represented by the Formula (XIII):

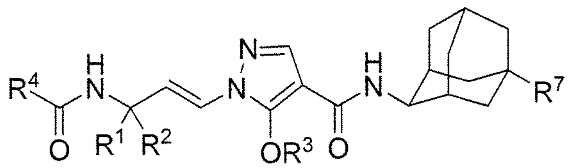

(XIII)

or its salt, wherein $R^1$, $R^2$, $R^3$ , $R^4$ and $R^7$ are as defined above; by using a solvent that is substantially free of methanol.

(45) A process for producing a crystal of a non-solvate of a compound represented by the Formula (XIII):

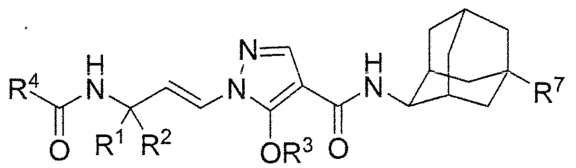

(XIII)

or a crystal of a non-solvate of a salt of the compound, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or
a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;
which comprises transforming a crystal of a methanol solvate of a compound represented by the Formula (XIII):

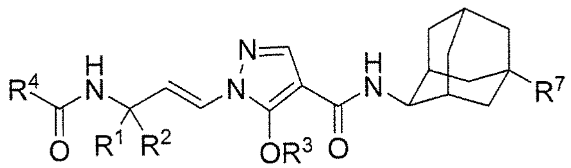

(XIII)

or a crystal of a methanol solvate of a salt of the compound, wherein $R^1$, $R^2$, $R^3$, $R^4$ and $R^7$ are as defined above; by using a solvent that is free of methanol of more than 30%(V/V).

Further, the inventors of the present invention completed the following invention.

(1A) A process for producing a compound represented by the Formula (III):

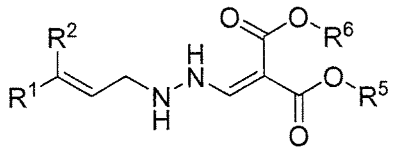

(III)

or its salt, wherein $R^1$ and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (I):

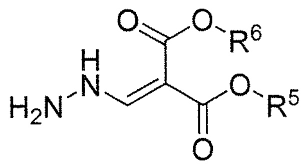

(I)

wherein $R^5$ and $R^6$ are as defined above; with a compound represented by the Formula (II):

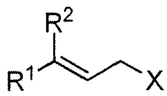

(II)

wherein $R^1$ and $R^2$ are as defined above and X is a leaving group.
(2A) A process for producing a compound represented by the Formula (IV):

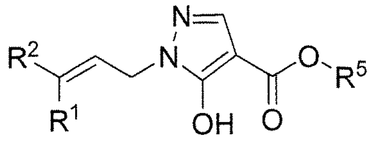

(IV)

or its salt, wherein $R^1$ and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (III):

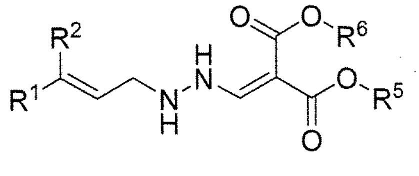

(III)

wherein $R^1$, $R^2$ and $R^5$ are as defined above and $R^6$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl; with a base.
(3A) A process for producing a compound represented by the Formula (VI):

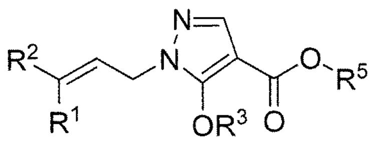

(VI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (IV):

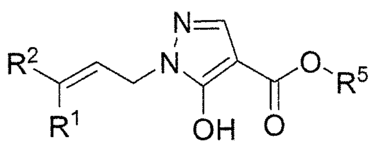

(IV)

wherein $R^1$, $R^2$ and $R^5$ are as defined above; with a compound represented by the Formula (V): $R^3$-X, wherein $R^3$ is as defined above and X is a leaving group; in the presence of a base.
(4A) A process for producing a compound represented by the Formula (III):

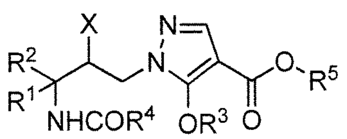

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and X is halogen;
which comprises reacting a compound represented by the Formula (VI):

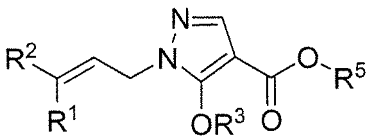

(VI)

wherein $R^1$, $R^2$, $R^3$ and $R^5$ are as defined above; with a compound represented by the Formula (VII): $R^4$-CN, wherein $R^4$ is as defined above; in the presence of a halogenating agent.
(5A) The process according to the above (4A), wherein the halogenating agent is N-halogenosuccinimide, N-halogenoacetamide or halogen.
(6A) The process according to the above (5A), wherein the halogenating agent is N-bromosuccinimide, N-bromoacetamide, N-chlorosuccinimide or $I_2$.
(7A) The process according to any one of the above (4A) to (6A), wherein the reaction is performed in the presence of a Lewis acid.
(8A) The process according to the above (7A), wherein the Lewis acid is boron trifluoride ether complex or metal halide.
(9A) The process according to the above (8A), wherein the Lewis acid is boron trifluoride n-butyl ether complex, boron trifluoride diethyl ether complex or SnCl4.
(10A) A process for producing a compound represented by the Formula (IX):

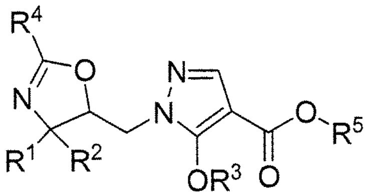

(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (VIII):

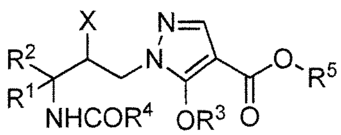

(VIII)

wherein $R^1$, $R^2$, $R^3$, $R^4$ and $R^5$ are as defined above and X is halogen; with a base.
(11A) The process according to the above (10A), wherein the base is an organic base.
(12A) A process for producing a compound represented by the Formula (X):

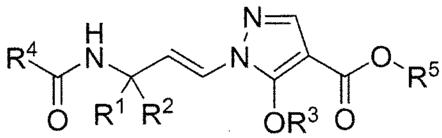

(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (IX):

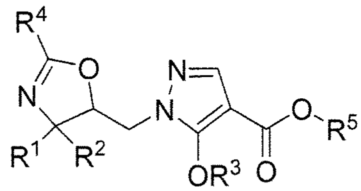

(IX)

wherein $R^1$, $R^2$, $R^3$, $R^4$ and $R^5$ are as defined above; with a base.
(13A) The process according to the above (12A), wherein the base is a metal alkoxide.
(14A) A process for producing a compound represented by the Formula (XI):

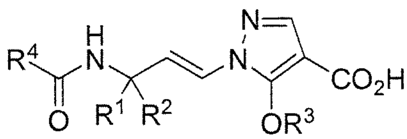

(XI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (X):

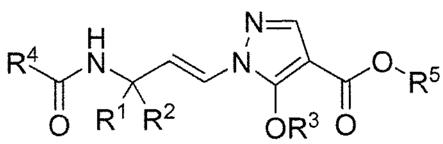

(X)

wherein $R^1$, $R^2$, $R^3$ and $R^4$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.
(15A) The process according to any one of the above (1A) to (14A), wherein $R^1$ and $R^2$ are substituted or unsubstituted alkyl.
(16A) The process according to any one of the above (3A) to (15A), wherein $R^3$ is substituted or unsubstituted alkyl.
(17A) The process according to any one of the above (3A) to (16A), wherein $R^3$ and $R^5$ are the same substituted or unsubstituted alkyl.
(18A) A process for producing a compound represented by the Formula (XV):

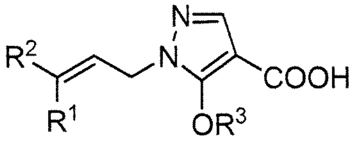

(XV)

or its salt, wherein $R^1$ and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (VI):

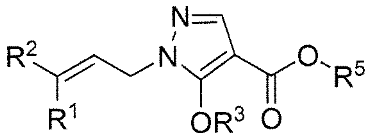

(VI)

wherein $R^1$, $R^2$ and $R^3$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, provided that $R^3$ and $R^5$ are not the same group.
(19A) A process for producing a compound represented by the Formula (VI):

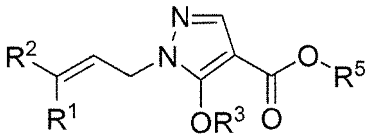

(VI)

or its salt, wherein $R^1$ and $R^2$ are each independently hydrogen or substituted or unsubstituted alkyl, and $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (XV):

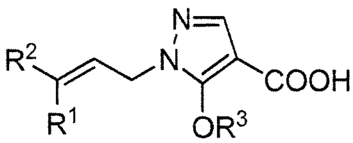

(XV)

wherein $R^1$, $R^2$ and $R^3$ are as defined above; with a compound represented by the Formula (XVII): $R^5$-OH, wherein $R^5$ is the same group as $R^3$.
(20A) The process according to the above (19A), which comprises a step of producing a compound represented by the formula (XVI):

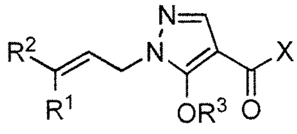

(XVI)

or its salt, wherein $R^1$, $R^2$ and $R^3$ are as defined in the above (19A) and X is halogen; by reacting a compound represented by the formula (XV):

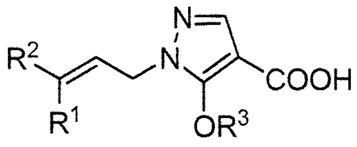

(XV)

wherein $R^1$, $R^2$ and $R^3$ are as defined above; with a halogenating agent.

(21A) The process according to the above (20A), wherein the halogenating agent is selected from phosphorus oxyhalide, phosphorus pentahalide, oxalyl halide and thionyl halide.

(22A) A process for producing a compound represented by the Formula (XIII):

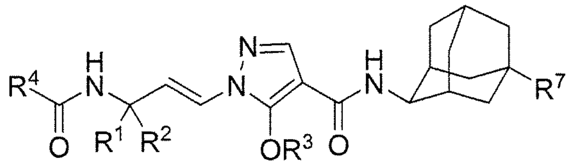

(XIII)

or its salt, wherein $R^1$, $R^2$, $R^3$ and $R^4$ are as defined in the above (14A), $R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ and $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

wherein the process comprises a process according to any one of the above (1A) to (21A).

(23A) A process for producing a compound represented by the Formula (XIII):

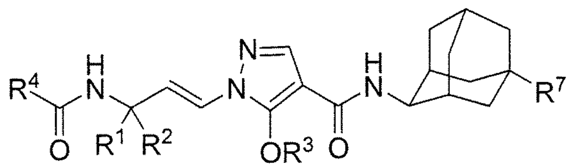

(XIII)

or its salt, wherein $R^1$, $R^2$, $R^3$ and $R^4$ are as defined in the above (14A), $R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ and $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

which comprises obtaining a compound represented by the Formula (XI):

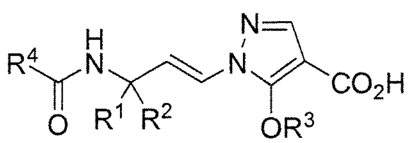

(XI)

wherein $R^1$, $R^2$, $R^3$ and $R^4$ are as defined above; by the process according to any one of the above (1A) to (21A), followed by reacting the obtained compound represented by the Formula (XI) with a compound represented by the Formula (XII):

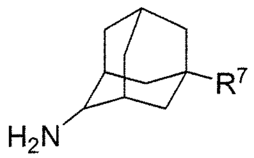

(XII)

wherein $R^7$ is as defined above.

(24A) The process according to the above (23A), wherein the reaction is performed in the presence of a condensing agent.

(25A) The process according to the above (24A), wherein the condensing agent is one or more condensing agent (s) selected from N,N'-dicyclohexylcarbodiimide, N,N'-diisopropylcarbodiimide and 1-ethyl-3-(3-dimethylaminopropyl) carbodiimide hydrochloride.

(26A) The process according to the above (24A) or (25A), wherein the reaction is performed in the presence of one or more additive agent(s) selected from 1-hydroxybenzotriazole and N-hydroxy succinimide.

(27A) The process according to the above (23A), which comprises a step of producing a compound represented by the formula (XIV):

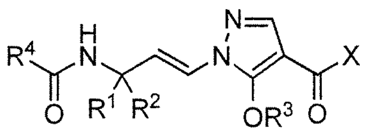

(XIV)

or its salt, wherein $R^1$, $R^2$, $R^3$ and $R^4$ are as defined in the above (14A) and X is halogen; by reacting a compound represented by the formula (XI):

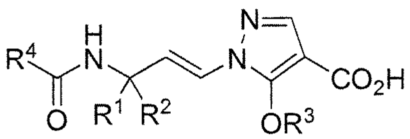

(XI)

wherein $R^1$, $R^2$, $R^3$ and $R^4$ are as defined above; with a halogenating agent.

(28A) The process according to the above (27A), wherein the halogenating agent is selected from phosphorus oxyhalide, phosphorus pentahalide, oxalyl halide and thionyl halide.

(29A) A compound represented by the Formula (III):

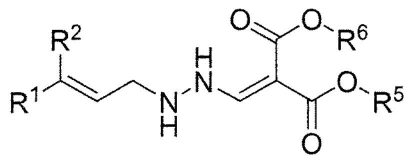

(III)

or its salt, wherein $R^1$ and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(29A-1) The compound according to the above (29A) or its salt, wherein $R^1$ and $R^2$ are methyl, and $R^5$ and $R^6$ are each independently alkyl.

(29A-2) The compound according to the above (29A-1) or its salt, wherein $R^5$ and $R^6$ are ethyl.

(30A) A compound represented by the Formula (IV):

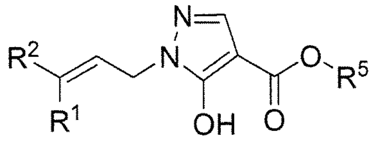

(IV)

or its salt, wherein $R^1$ and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(30A-1) The compound according to the above (30A) or its salt, wherein $R^1$ and $R^2$ are methyl, and $R^5$ is alkyl.

(30A-2) The compound according to the above (30A-1) or its salt, wherein $R^5$ is ethyl.

(30A-3) The crystal of a compound according to the above (30A-2) or its salt, wherein the values of $2\theta$ of the powder X-ray diffraction have two or more $2\theta$ selected from $8.2 \pm 0.2$, $12.4 \pm 0.2$, $18.3 \pm 0.2$, $24.5 \pm 0.2$ and $25.6 \pm 0.2$ degrees.

(31A) A compound represented by the Formula (VI):

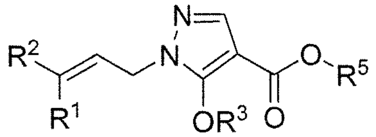

(VI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(31A-1) The compound according to the above (31A) or its salt, wherein $R^1$ and $R^2$ are methyl, and $R^3$ and $R^5$ are each independently alkyl.

(31A-2) The compound according to the above (31A-1) or its salt, wherein $R^3$ is ethyl, n-propyl, isopropyl or isobutyl.

(31A-3) The compound according to the above (31A-1) or (31A-2), or its salt, wherein $R^5$ is ethyl.

(31A-4) The compound according to the above (31A-1) or (31A-2), or its salt, wherein $R^5$ is isopropyl.

(32A) A compound represented by the Formula (VIII):

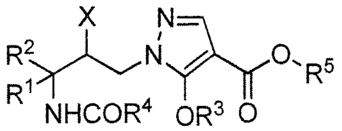

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and X is halogen.

(32A-1) The compound according to the above (32A) or its salt, wherein $R^1$, $R^2$ and $R^4$ are methyl, and $R^3$ and $R^5$ are each independently alkyl.

(32A-2) The compound according to the above (32A-1) or its salt, wherein $R^3$ is ethyl, n-propyl, isopropyl or isobutyl.

(32A-3) The compound according to the above (32A-1) or (32A-2), or its salt, wherein $R^5$ is ethyl.

(32A-4) The compound according to the above (32A-1) or (32A-2), or its salt, wherein $R^5$ is isopropyl.

(33A) A compound represented by the Formula (IX):

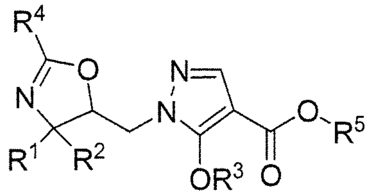

(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(33A-1) The compound according to the above (33A) or its salt, wherein $R^1$, $R^2$ and $R^4$ are methyl, and $R^3$ and $R^5$ are each independently alkyl.

(33A-2) The compound according to the above (33A-1) or its salt, wherein $R^3$ is ethyl, n-propyl, isopropyl or isobutyl.

(33A-3) The compound according to the above (33A-1) or (33A-2), or its salt, wherein $R^5$ is ethyl.

(33A-4) The compound according to the above (33A-1) or (33A-2), or its salt, wherein $R^5$ is isopropyl.

(34A) A compound represented by the Formula (X):

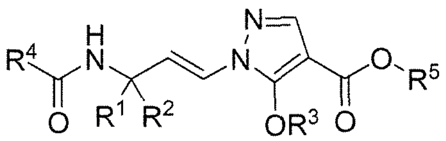

(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

(34A-1) The compound according to the above (34A) or its salt, wherein $R^1$, $R^2$ and $R^4$ are methyl, $R^3$ is isopropyl and $R^5$ is alkyl.

(34A-2) The compound according to the above (34A-1) or its salt, wherein $R^5$ is isopropyl.

(34A-3) The crystal of a compound according to the above (34A-2) or its salt, wherein the values of $2\theta$ of the powder X-ray diffraction have two or more $2\theta$ selected from $12.7 \pm 0.2$, $13.9 \pm 0.2$, $15.2 \pm 0.2$, $15.5 \pm 0.2$, $17.2 \pm 0.2$, $18.2 \pm 0.2$, $18.6 \pm 0.2$, $19.9 \pm 0.2$ and $20.2 \pm 0.2$ degrees.

(35A) A compound represented by the Formula (XI):

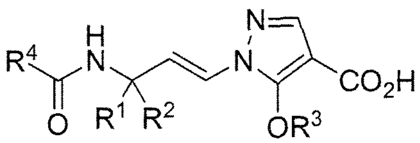

(XI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

(35A-1) The compound according to the above (35A) or its salt, wherein $R^1$, $R^2$ and $R^4$ are methyl, and $R^3$ is ethyl.

(35A-2) The crystal of a compound according to the above (35A-1) or its salt, wherein the values of 2θ of the powder X-ray diffraction have two or more 2θ selected from 9.0 ± 0.2, 13.0 ± 0.2, 13.9 ± 0.2, 15.0 ± 0.2, 15.6 ± 0.2, 18.9 ± 0.2, 22.0 ± 0.2, 22.3 ± 0.2, 23.1 ± 0.2, 24.3 ± 0.2, 25.0 ± 0.2 and 25.4 ± 0.2 degrees.

(35A-3) The compound according to the above (35A) or its salt, wherein $R^1$, $R^2$ and $R^4$ are methyl, and $R^3$ is n-propyl.

(35A-4) The crystal of a compound according to the above (35A-3) or its salt, wherein the values of 2θ of the powder X-ray diffraction have two or more 2θ selected from 8.5 ± 0.2, 11.4 ± 0.2, 14.2 ± 0.2, 18.1 ± 0.2, 19.4 ± 0.2, 21.1 ± 0.2, 23.9 ± 0.2 and 26.4 ± 0.2 degrees.

(35A-5) The compound according to the above (35A) or its salt, wherein $R^1$, $R^2$ and $R^4$ are methyl, and $R^3$ is isopropyl.

(35A-6) The crystal of a compound according to the above (35A-5) or its salt, wherein the values of 2θ of the powder X-ray diffraction have two or more 2θ selected from 8.7 ± 0.2, 11.3 ± 0.2, 14.3 ± 0.2, 15.0 ± 0.2, 17.5 ± 0.2, 19.4 ± 0.2, 21.1 ± 0.2, 22.7 ± 0.2, 24.3 ± 0.2, 24.8 ± 0.2 and 26.0 ± 0.2 degrees.

(35A-7) The compound according to the above (35A) or its salt, wherein $R^1$, $R^2$ and $R^4$ are methyl, and $R^3$ is isobutyl.

(35A-8) The crystal of a compound according to the above (35A-7) or its salt, wherein the values of 2θ of the powder X-ray diffraction have two or more 2θ selected from 10.6 ± 0.2, 11.5 ± 0.2, 17.6 ± 0.2, 18.0 ± 0.2, 18.6 ± 0.2, 19.5 ± 0.2, 19.9 ± 0.2, 23.1 ± 0.2, 24.8 ± 0.2, 27.5 ± 0.2 and 27.9 ± 0.2 degrees.

(36A) A compound represented by the Formula (XV):

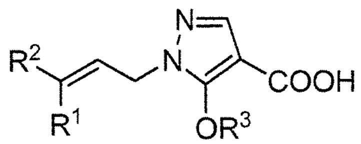

(XV)

or its salt, wherein $R^1$ and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

(36A-1) The compound according to the above (36A) or its salt, wherein $R^1$ and $R^2$ are methyl, and $R^3$ is isopropyl.

(36A-2) The crystal of a compound according to the above (36A-1) or its salt, wherein the values of 2θ of the powder X-ray diffraction have 2θ of 12.0 ± 0.2 and 20.3 ± 0.2 degrees.

For purposes of this description, reacting a compound with a compound includes reacting a compound, its salt, or a solvate thereof with a compound, its salt, or a solvate thereof.

[Effect of the Invention]

The novel process for producing a compound represented by the Formula (XI) of the present invention can be applied in industrial production as a process providing high yield and safety.

Moreover, a compound represented by the Formula (XI) is a useful compound as synthetic raw material or an intermediate for pharmaceuticals or the like. By using a compound represented by the Formula (XI), a compound represented by the Formula (XIII) can be efficiently produced.

[Brief Description of the Drawings]

Fig. 1 shows data of powder X-ray diffraction for compound (IV-1-1).
Fig. 2 shows data of powder X-ray diffraction for compound (XI-1-1).
Fig. 3 shows data of powder X-ray diffraction for compound (XI-1-2).
Fig. 4 shows data of powder X-ray diffraction for type I crystal of compound (XI-1-3).
Fig. 5 shows data of powder X-ray diffraction for compound (XI-1-4).
Fig. 6 shows data of powder X-ray diffraction for compound (XV-1-1).
Fig. 7 shows data of powder X-ray diffraction for compound (X-1-3').
Fig. 8 shows data of powder X-ray diffraction for type I crystal of compound (XIII-1-1).
Fig. 9 shows data of powder X-ray diffraction for type II crystal of compound (XI-1-3).
Fig. 10 shows data of powder X-ray diffraction for type III crystal of compound (XI-1-3).
Fig. 11 shows data of powder X-ray diffraction for methanol solvate of compound (XIII-1-1).

[Mode for Carrying Out the Invention]

In the following, meanings of terms used in the present specification will be explained. Each term has the same meaning when used alone or in combination with other term in this description.

"Halogen" includes fluorine, chlorine, bromine or iodine.

"Alkyl" means a C1 to C10 straight or branched alkyl group, and example includes methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, n-decyl or the like. Preferable is C1 to C6 or C1 to C4 alkyl, and example includes methyl, ethyl, n-propyl, isopropyl, n-butyl, isobutyl, sec-butyl, tert-butyl, n-pentyl, isopentyl, neopentyl, n-hexyl or isohexyl.

"Alkenyl" means C2 to C8 straight or branched alkenyl having one or more double bond(s) in the above "alkyl", and example includes vinyl, 1-propenyl, 2-propenyl, 1-butenyl, 2-butenyl, 3-butenyl, 1,3-butadienyl, 3-methyl-2-butenyl or the like.

"Alkynyl" means C2 to C8 straight or branched alkynyl having one or more triple bond(s) in the above "alkyl", and example includes ethynyl, propinyl, butynyl or the like. Furthermore, "Alkynyl" may have a double bond.

"Cycloalkyl" means a C3 to C15 cyclic saturated hydrocarbon group, and example includes cyclopropyl, cyclobutyl, cyclopentyl, cyclohexyl, cycloheptyl, cyclooctyl, bridged cyclic hydrocarbon group, spiro hydrocarbon group or the like. Preferable is cyclopropyl, cyclobutyl, cyclopentyl, cyclohexyl or bridged cyclic hydrocarbon group.

"Bridged cyclic hydrocarbon group" includes a group which is derived by excluding one hydrogen from a C5 to C8 aliphatic cycle which consists of two or more rings that share two or more atoms. Example includes bicyclo[2.1.0]pentyl, bicyclo[2.2.1]heptyl, bicyclo[2.2.2]octyl, bicyclo[3.2.1]octyl, tricyclo[2.2.1.0]heptyl or the like.

"Spiro hydrocarbon group" includes a group which is derived by excluding one hydrogen from a cycle which consists of two hydrocarbon rings that share one carbon atom. Example includes spiro[3.4]octyl or the like.

"Cycloalkenyl" means C3 to C10 cyclic unsaturated aliphatic hydrocarbon group, and example includes cyclopropenyl (e.g.: 1-cyclopropenyl), cyclobutenyl (e.g.: 1-cyclobutenyl), cyclopentenyl (e.g.: 1-cyclopenten-1-yl, 2-cyclopenten-1-yl or 3-cyclopenten-1-yl), cyclohexenyl (e.g.: 1-cyclohexen-1-yl, 2-cyclohexen-1-yl or 3-cyclohexen-1-yl), cycloheptenyl (e.g.: 1- cycloheptenyl), cyclooctenyl (e.g.: 1-cyclooctenyl) or the like. Preferable is cyclopropenyl, cyclobutenyl, cyclopentenyl or cyclohexenyl. Cycloalkenyl also includes bridged cyclic hydrocarbon group and spiro hydrocarbon group which have an unsaturated bond in the ring.

"Aryl" means a monocyclic aromatic hydrocarbon group (e.g.: phenyl) and a polycyclic aromatic hydrocarbon group (e.g.: 1-naphthyl, 2-naphthyl, 1-anthryl, 2-anthryl, 9-anthryl, 1-phenanthryl, 2-phenanthryl, 3-phenanthryl, 4-phenanthryl or 9-phenanthryl). Preferable is phenyl or naphthyl (1-naphthyl or 2-naphthyl).

"Heteroaryl" means a monocyclic aromatic heterocyclic group and a fused aromatic heterocyclic group.

The "monocyclic aromatic heterocyclic group" means a group which is induced from a 5 to 8-membered aromatic ring which has one or more, the same or different, hetero atoms optionally selected from oxygen, sulfur and nitrogen atoms in the ring, which group may have a bond at any substitutable position.

The "fused aromatic heterocyclic group" means a group in which a 5 to 8-membered aromatic ring which has one or more, the same or different, hetero atoms optionally selected from oxygen, sulfur and nitrogen atoms in the ring is fused with one to four 5 to 8-membered aromatic carbocyclic rings or another 5 to 8-membered aromatic hetero ring, which group may have a bond at any substitutable position.

Example of the "heteroaryl" includes furyl (e.g.: 2-furyl or 3-furyl), thienyl (e.g.: 2-thienyl or 3-thienyl), pyrrolyl (e.g.: 1-pyrrolyl, 2-pyrrolyl or 3-pyrrolyl), imidazolyl (e.g.: 1-imidazolyl, 2-imidazolyl or 4-imidazolyl), pyrazolyl (e.g.: 1-pyrazolyl, 3-pyrazolyl or 4-pyrazolyl), triazolyl (e.g.: 1,2,4-triazole-1-yl, 1,2,4-triazole-3-yl or 1,2,4-triazole-4-yl), tetrazolyl (e.g.: 1-tetrazolyl, 2-tetrazolyl or 5-tetrazolyl), oxazolyl (e.g.: 2-oxazolyl, 4-oxazolyl or 5-oxazolyl), isoxazolyl (e.g.: 3-isoxazolyl, 4-isoxazolyl or 5-isoxazolyl), thiazolyl (e.g.: 2-thiazolyl, 4-thiazolyl or 5-thiazolyl), thiadiazolyl, isothiazolyl (e.g.: 3-isothiazolyl, 4-isothiazolyl or 5-isothiazolyl), pyridyl (e.g.: 2-pyridyl, 3-pyridyl or 4-pyridyl), pyridazinyl (e.g.: 3-pyridazinyl or 4-pyridazinyl), pyrimidinyl (e.g.: 2- pyrimidinyl, 4- pyrimidinyl or 5-pyrimidinyl), furazanyl (e.g.: 3- furazanyl), pyrazinyl (e.g.: 2-pyrazinyl), oxadiazolyl (e.g.: 1,3,4-oxadiazole-2-yl), benzofuryl (e.g.: 2-benzo[b]furyl, 3-benzo[b]furyl, 4-benzo[b]furyl, 5-benzo[b]furyl, 6-benzo[b]furyl or 7-benzo[b]furyl), benzothienyl (e.g.: 2-benzo[b]thienyl, 3-benzo[b]thienyl, 4-benzo[b]thienyl, 5-benzo[b]thienyl, 6-benzo[b]thienyl or 7-benzo[b]thienyl), benzimidazolyl (e.g.: 1-benzimidazolyl, 2-benzimidazolyl, 4-benzimidazolyl or 5-benzimidazolyl), dibenzofuryl, benzoxazolyl, benzothiazolyl, quinoxalinyl (e.g.: 2-quinoxalinyl, 5-quinoxalinyl or 6-quinoxalinyl), cinnolinyl (e.g.: 3-cinnolinyl, 4-cinnolinyl, 5-cinnolinyl, 6-cinnolinyl, 7-cinnolinyl or 8-cinnolinyl), quinazolinyl (e.g.: 2-quinazolinyl, 4-quinazolinyl, 5-quinazolinyl, 6-quinazolinyl, 7-quinazolinyl or 8-quinazolinyl), quinolyl (e.g.: 2-quinolyl, 3-quinolyl, 4-quinolyl, 5-quinolyl, 6-quinolyl, 7-quinolyl or 8-quinolyl), phthalazinyl (e.g.: 1-phthalazinyl, 5-phthalazinyl or 6-phthalazinyl), isoquinolyl (e.g.: 1-isoquinolyl, 3-isoquinolyl, 4-isoquinolyl, 5-isoquinolyl, 6-isoquinolyl, 7-isoquinolyl or 8-isoquinolyl), puryl, pteridinyl (e.g.: 2-pteridinyl, 4-pteridinyl, 6-pteridinyl or 7-pteridinyl), carbazolyl, phenanthridinyl, acridinyl (e.g.: 1-acridinyl, 2-acridinyl, 3-acridinyl, 4-acridinyl or 9-acridinyl), indolyl (e.g.: 1-indolyl, 2-indolyl, 3-indolyl, 4-indolyl, 5-indolyl, 6-indolyl or 7-indolyl), isoindolyl, phenadinyl (e.g.: 1-phenadinyl or 2-phenadinyl), phenothiadinyl (e.g.: 1-phenothiadinyl, 2-phenothiadinyl, 3-phenothiadinyl or 4-phenothiadinyl) or the like.

"Heterocyclyl" means a non aromatic heterocyclic group, which may have a bond at any substitutable position of a ring which has at least one or more nitrogen, oxygen or sulfur atoms in the ring, or a ring in which such ring is fused with a cycloalkane (preferably 5 to 6-membered), a benzene ring and/or a ring which has at least one or more nitrogen, oxygen or sulfur atoms in the ring. "Nonaromatic heterocyclic group" can be saturated or unsaturated as long as it is non-aromatic. Preferable is a 5- to 8-membered ring. Example includes 1-pyrrolinyl, 2-pyrrolinyl, 3-pyrrolinyl, 1-pyrrolidinyl, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4- pyrazolinyl, 1-pyrazolidinyl, 3-pyrazolidinyl, 4-pyrazolidinyl, piperidino, 2-piperidinyl, 3-piperidinyl, 4-piperidinyl, 1-piperadinyl, 2-piperadinyl, 2-morpholinyl, 3-morpholinyl, morpholino, tetrahydropyranyl, 1,2,3,4-tetrahydroisoquinolinyl, 1,2,3,4-tetrahydroquinolinyl, 1,3-dihydro-2H-isoindol-5-yl or the like.

"Heterocyclyl" further contains a bridged group or a spiro ring forming group shown below.

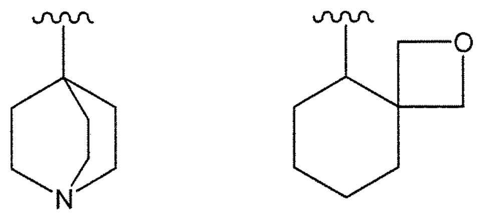

"Acyl" means formyl, substituted or unsubstituted alkylcarbonyl, substituted or unsubstituted alkenylcarbonyl, substituted or unsubstituted cycloalkylcarbonyl, substituted or unsubstituted cycloalkenylcarbonyl, substituted or unsubstituted arylcarbonyl, substituted or unsubstituted heteroarylcarbonyl or substituted or unsubstituted heterocyclylcarbonyl.

The alkyl part of "alkylcarbonyl", the alkenyl part of "alkenylcarbonyl", the cycloalkyl part of "cycloalkylcarbonyl", the cycloalkenyl part of "cycloalkenylcarbonyl", the aryl part of "arylcarbonyl", the heteroaryl part of "heteroarylcarbonyl" and the heterocyclyl part of "heterocyclylcarbonyl" respectively mean the above "alkyl", the above "alkenyl", the above "cycloalkyl", the above "cycloalkenyl", the above "aryl", the above "heteroaryl" and the above "heterocyclyl".

The alkyl part of "alkylsulfonyl" and "alkyloxycarbonyl" means the above "alkyl".

"Substituted alkyl", "substituted alkenyl", "substituted alkynyl", "substituted cycloalkyl", "substituted cycloalkenyl", "substituted aryl", "substituted heteroayl", "substituted heterocyclyl", "substituted acyl", "substituted alkylsulfonyl", "substituted alkyloxycarbonyl", "substituted carbamoyl" or "substituted sulfamoyl" may be substituted with 1 to 4 substituent(s) selected from a group consisting of, for example, halogen; hydroxy; carboxy; nitro; cyano;

substituted or unsubstituted alkyl (an example of a substituent of substituted alkyl includes halogen, hydroxy, carboxy, nitro, cyano, alkyl, alkenyl, alkynyl, aryl, heteroaryl, cycloalkyl, cycloalkenyl or heterocyclyl. e.g. methyl, ethyl, isopropyl, tert-butyl, $CF_3$ or benzyl);

substituted or unsubstituted alkenyl (an example of a substituent of substituted alkenyl includes halogen, hydroxy, carboxy, nitro, cyano, alkyl, alkenyl, alkynyl, aryl, heteroaryl, cycloalkyl, cycloalkenyl or heterocyclyl. e.g. vinyl);

substituted or unsubstituted alkynyl (an example of a substituent of substituted alkynyl includes halogen, hydroxy, carboxy, nitro, cyano, alkyl, alkenyl, alkynyl, aryl, heteroaryl, cycloalkyl, cycloalkenyl or heterocyclyl. e.g. ethynyl);

substituted or unsubstituted aryl (an example of a substituent of substituted aryl includes halogen, hydroxy, carboxy, nitro, cyano, alkyl, alkenyl, alkynyl, aryl, heteroaryl, cycloalkyl, cycloalkenyl or heterocyclyl. e.g. phenyl or naphthyl);

substituted or unsubstituted cycloalkyl (an example of a substituent of substituted cycloalkyl includes halogen, hydroxy, carboxy, nitro, cyano, alkyl, alkenyl, alkynyl, aryl, heteroaryl, cycloalkyl, cycloalkenyl or heterocyclyl. e.g. cyclopropyl or cyclobutyl.);

substituted or unsubstituted cycloalkenyl (an example of a substituent of substituted cycloalkenyl includes halogen, hydroxy, carboxy, nitro, cyano, alkyl, alkenyl, alkynyl, aryl, heteroaryl, cycloalkyl, cycloalkenyl or heterocyclyl. e.g. cyclopropenyl);

substituted or unsubstituted heteroaryl (an example of a substituent of substituted heteroaryl includes halogen, hydroxy, carboxy, nitro, cyano, alkyl, alkenyl, alkynyl, aryl, heteroaryl, cycloalkyl, cycloalkenyl or heterocyclyl. e.g. tetrazolyl, indolyl or pyrazolyl.);

substituted or unsubstituted heterocyclyl (an example of a substituent of substituted heterocyclyl includes halogen, hydroxy, carboxy, nitro, cyano, alkyl, alkenyl, alkynyl, aryl, heteroaryl, cycloalkyl, cycloalkenyl or heterocyclyl. e.g. pyrrolidinyl, morpholinyl, piperadinyl or piperidinyl.);

substituted or unsubstituted alkyloxy (an example of a substituent of substituted alkyloxy includes halogen, hydroxy, carboxy, nitro, cyano, alkyl, alkenyl, alkynyl, aryl, heteroaryl, cycloalkyl, cycloalkenyl or heterocyclyl. e.g. methoxy, ethoxy, propoxy, $OCF_3$ or butoxy.);

substituted or unsubstituted aryloxy (an example of a substituent of substituted aryloxy includes halogen, hydroxy,

carboxy, nitro, cyano, alkyl, alkenyl, alkynyl, aryl, heteroaryl, cycloalkyl, cycloalkenyl or heterocyclyl. e.g. phenyloxy); substituted or unsubstituted silyloxy;
substituted or unsubstituted amino (e.g. alkylamino (e.g. methylamino, ethylamino or dimethylamino.), acylamino (e.g. acetylamino or benzoylamino.), arylalkylamino (e.g. benzylamino or tritylamino.), hydroxyamino, alkyloxycarbonylamino, aryloxycarbonylamino, heteroaryloxycarbonylamino, alkylsulfonylamino, arylsulfonylamino, heteroarylsulfonylamino, arylamino, heteroarylamino or carbamoylamino.);
substituted or unsubstituted carbamoyl (an example of a substituent of substituted carbamoyl includes halogen, hydroxy, carboxy, nitro, cyano, alkyl, alkenyl, alkynyl, aryl, heteroaryl, cycloalkyl, cycloalkenyl or heterocyclyl. e.g. alkylcarbamoyl (e.g. methylcarbamoyl, ethylcarbamoyl, dimethylcarbamoyl or isopropylcarbamoyl.)); substituted or unsubstituted carbamoyloxy (an example of a substituent of substituted carbamoyloxy includes halogen, hydroxy, carboxy, nitro, cyano, alkyl, alkenyl, alkynyl, aryl, heteroaryl, cycloalkyl, cycloalkenyl or heterocyclyl.); substituted or unsubstituted acyl (an example of a substituent of substituted acyl includes halogen, hydroxy, carboxy, nitro, cyano, alkyl, alkenyl, alkynyl, aryl, heteroaryl, cycloalkyl, cycloalkenyl or heterocyclyl. e.g. alkylcarbonyl, arylcarbonyl, heteroarylcarbonyl, heterocyclylcarbonyl, formyl or acetyl.); substituted or unsubstituted alkylsulfonyl (an example of a substituent of substituted alkylsulfonyl includes halogen, hydroxy, carboxy, nitro, cyano, alkyl, alkenyl, alkynyl, aryl, heteroaryl, cycloalkyl, cycloalkenyl or heterocyclyl. e.g. methanesulfonyl or ethanesulfonyl.);
substituted or unsubstituted arylsulfonyl (an example of a substituent of substituted arylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heteroarylsulfonyl (an example of a substituent of substituted heteroarylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted cycloalkylsulfonyl (an example of a substituent of substituted cycloalkylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclylsulfonyl (an example of a substituent of substituted heterocyclylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted sulfamoyl (an example of a substituent of substituted sulfamoyl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted alkyloxycarbonyl (an example of a substituent of substituted alkyloxycarbonyl includes halogen, hydroxy, carboxy, nitro, cyano, alkyl, alkenyl, alkynyl, aryl, heteroaryl, cycloalkyl, cycloalkenyl or heterocyclyl. e.g. methoxycarbonyl, ethoxycarbonyl or tert-butoxycarbonyl.);
substituted or unsubstituted aryloxycarbonyl (an example of a substituent of substituted aryloxycarbonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heteroaryloxycarbonyl (an example of a substituent of substituted heteroaryloxycarbonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclyloxycarbonyl (an example of a substituent of substituted heterocyclyloxycarbonyl includes halogen, hydroxy, carboxy, nitro, cyano, alkyl, alkenyl, alkynyl, aryl, heteroaryl, cycloalkyl, cycloalkenyl or heterocyclyl.);
alkylsulfinyl; cycloalkylsulfinyl; arylsulfinyl; heteroarylsulfinyl;
heterocyclylsulfinyl;
nitroso;
alkenyloxy (e.g. vinyloxy or allyloxy.);
arylalkyloxy (e.g. benzyloxy);
azido;
isocyano; isocyanato; thiocyanato; isothiocyanato; mercapto;
alkylthio (e.g. methylthio);
formyloxy; haloformyl; oxalo; thioformyl; thiocarboxy; dithiocarboxy; thiocarbamoyl; sulfino; sulfo; sulfoamino; hydrazino; ureido; amidino; guanidino; phthalimido; oxo and the like.

Preferred example of a substituent of "substituted carbamoyl" or "substituted sulfamoyl" includes
halogen; hydroxy; carboxy; nitro; cyano;
substituted or unsubstituted alkyl (an example of a substituent of substituted alkyl includes halogen, hydroxy, carboxy, nitro, cyano, alkyl, alkenyl, alkynyl, aryl, heteroaryl, cycloalkyl, cycloalkenyl or heterocyclyl);
substituted or unsubstituted alkenyl (an example of a substituent of substituted alkenyl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted alkynyl (an example of a substituent of substituted alkynyl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted aryl (an example of a substituent of substituted aryl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted heteroaryl (an example of a substituent of substituted heteroaryl includes halogen, hydroxy, carboxy, nitro, cyano, alkyl, alkenyl, alkynyl, aryl, heteroaryl, cycloalkyl, cycloalkenyl or heterocyclyl.);

substituted or unsubstituted cycloalkyl (an example of a substituent of substituted cycloalkyl includes halogen, hydroxy, carboxy, nitro, cyano, alkyl, alkenyl, alkynyl, aryl, heteroaryl, cycloalkyl, cycloalkenyl or heterocyclyl);
substituted or unsubstituted cycloalkenyl (an example of a substituent of substituted cycloalkenyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclyl (an example of a substituent of substituted heterocyclyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted acyl (an example of a substituent of substituted acyl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted alkyloxycarbonyl (an example of a substituent of substituted alkyloxycarbonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted aryloxycarbonyl (an example of a substituent of substituted aryloxycarbonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heteroaryloxycarbonyl (an example of a substituent of substituted heteroaryloxycarbonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclyloxycarbonyl (an example of a substituent of substituted heterocyclyloxycarbonyl includes halogen, hydroxy, carboxy, nitro, cyano, alkyl, alkenyl, alkynyl, aryl, heteroaryl, cycloalkyl, cycloalkenyl or heterocyclyl.);
substituted or unsubstituted alkylsulfonyl (an example of a substituent of substituted alkylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted arylsulfonyl (an example of a substituent of substituted arylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heteroarylsulfonyl (an example of a substituent of substituted heteroarylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted cycloalkylsulfonyl (an example of a substituent of substituted cycloalkylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclylsulfonyl (an example of a substituent of substituted heterocyclylsulfonyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted alkylsulfinyl (an example of a substituent of substituted alkylsulfinyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted arylsulfinyl (an example of a substituent of substituted arylsulfinyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heteroarylsulfinyl (an example of a substituent of substituted heteroarylsulfinyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted cycloalkylsulfinyl (an example of a substituent of substituted cycloalkylsulfinyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted heterocyclylsulfinyl (an example of a substituent of substituted heterocyclylsulfinyl includes halogen, hydroxy, carboxy, nitro, cyano, alkyl, alkenyl, alkynyl, aryl, heteroaryl, cycloalkyl, cycloalkenyl or heterocyclyl.); substituted or unsubstituted amino (an example of a substituent of substituted amino includes halogen, hydroxy, carboxy, nitro, cyano, alkyl, alkenyl, alkynyl, aryl, heteroaryl, cycloalkyl, cycloalkenyl or heterocyclyl.)
or the like.

The alkyl part of "alkyloxy", "alkylamino", "arylalkylamino", "alkyloxycarbonylamino", "alkylsulfonylamino", "alkylcarbamoyl", "alkylsulfinyl", "arylalkyloxy" and "alkylthio" means the above-described "alkyl".

The alkenyl part of "alkenyloxy" means the above-described "alkenyl".

The aryl part of "arylalkylamino", "aryloxycarbonylamino", "arylsulfonylamino", "arylamino", "arylsulfonyl", "aryloxycarbonyl", "arylsulfinyl" and "arylalkyloxy" means the above-described "aryl".

The heteroaryl part of "heteroaryloxycarbonylamino", "heteroarylsulfonylamino", "heteroarylamino", "heteroarylsulfonyl", "heteroaryloxycarbonyl" and "heteroarylsulfinyl" means the above-described "heteroaryl".

The cycloalkyl part of "cycloalkylsulfonyl" and "cycloalkylsulfinyl" means the above-described "cycloalkyl".

The heterocyclyl part of "heterocyclylsulfonyl", "heterocyclyloxycarbonyl" and "heterocyclylsulfinyl" means the above-described "heterocyclyl".

Among the compounds of the present invention, the following embodiments are preferable.

$R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

Preferably, $R^5$ and $R^6$ are each independently substituted or unsubstituted alkyl.

$R^1$ and $R^2$ are each independently hydrogen or substituted or unsubstituted alkyl. Preferably, $R^1$ and $R^2$ are each independently substituted or unsubstituted alkyl.

$X^1$ is halogen. Preferable is bromine.

$X^2$ is halogen. Preferable is chloride.

$X^3$ is a leaving group. An example of a leaving group includes substituted or unsubstituted alkylsulfonyloxy (e.g., methanesulfonyloxy, trifluoromethanesulfonyloxy or the like.), substituted or unsubstituted benzene sulfonyloxy (e.g., paratoluene sulfonyloxy, orthonitrobenzene sulfonyloxy or the like.), halogen (iodine, bromine or chlorine) or the like. Preferable is halogen. More preferable is bromine.

The alkyl part of " alkylsulfonyloxy" means the above-described "alkyl".

$X^4$ is a leaving group. An example of a leaving group includes substituted or unsubstituted alkylsulfonyloxy (e.g., methanesulfonyloxy, trifluoromethanesulfonyloxy or the like.), substituted or unsubstituted benzene sulfonyloxy (e.g., paratoluene sulfonyloxy, orthonitrobenzene sulfonyloxy or the like.), halogen (iodine, bromine or chlorine) or the like. Preferable is halogen. More preferable is bromine.

$X^5$ is halogen. Preferable is chlorine.

X is a leaving group. Preferable is halogen. More preferable is chlorine or bromine.

$R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

Preferable is substituted or unsubstituted alkyl.

$R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

Preferable is substituted or unsubstituted alkyl.

$R^7$ is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{19}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above.

Preferable is a group represented by the Formula: $-OR^8$ or a group represented by the Formula: $-(CR^9R^{10})m-O-C(O)-NR^{13}-R^{14}$, wherein $R^8$, $R^{13}$, $R^{14}$, $R^9$, $R^{10}$ and m are as defined above.

More preferable is a group represented by the Formula: $-(CR^9R^{10})m-O-C(O)-NR^{13}-R^{14}$, wherein $R^{13}$, $R^{14}$, $R^9$, $R^{10}$ and m are as defined above.

$R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

Preferable is hydrogen or substituted or unsubstituted alkyl. More preferable is hydrogen.

$R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl.

Preferable is substituted or unsubstituted acyl, substituted or unsubstituted alkylsulfonyl or substituted or unsubstituted alkyloxycarbonyl.

$R^{11}$ is hydrogen or substituted or unsubstituted alkyl. Preferable is hydrogen.

$R^9$ are each independently hydrogen, substituted or unsubstituted alkyl or halogen. Preferable is hydrogen.

$R^{10}$ are each independently hydrogen, substituted or unsubstituted alkyl or halogen. Preferable is hydrogen.

m is an integer of 0 to 3. Preferable is 0 or 1. More preferable is 0.

$R^{13}$ and $R^{14}$ are each independently hydrogen or substituted or unsubstituted alkyl. Preferable is hydrogen.

One or more hydrogen, carbon and/or other atoms of the compound of the present invention can be replaced by an isotope of the hydrogen, carbon and/or other atoms, respectively.

Examples of isotopes that can be incorporated into the compound of formula (I) of the present invention include isotopes of hydrogen, carbon, nitrogen, oxygen, phosphorous, sulfur, fluorine, iodine and chlorine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$ and $^{36}Cl$, respectively. The compound of the present invention includes a compound replaced by an isotope. The compound replaced by an isotope is useful as a medicament and is encompassed by the present invention. The "radiolabeled" for producing the "radiolabeled form" is encompassed by the present invention and useful as a research and/or diagnostic tool in metabolism pharmacokinetic studies and in binding assays.

Radiolabeled compounds of the present invention can be prepared by methods known in the art. For example,

tritiated compounds can be prepared by introducing tritium into the particular compound of the present invention, for example, by catalytic dehalogenation with tritium. This method may include reacting a suitably halogen-substituted precursor of a compound of the present invention with tritium gas in the presence of a suitable catalyst such as Pd/C, in the presence or absence of a base. Other suitable methods for preparing tritiated compounds can be found in *Isotopes in the Physical and Biomedical Sciences, Viol. 1, Labeled Compounds (PartA),* Chapter 6 (1987). $^{14}C$-labeled compounds can be prepared by employing starting materials having a $^{14}C$ carbon.

As a pharmaceutically acceptable salt of the present compound, for example, the following salts can be included.

Example includes alkali metal salt such as lithium salt, sodium salt or potassium salt; alkaline earth metal salt such as calcium salt or barium salt; magnesium salt; transition metal salt such as zinc salt or iron salt; ammonium salt; organic base salt such as trimethylamine salt, triethylamine salt, dicyclohexylamine salt, ethanolamine salt, diethanolamine salt, triethanolamine salt, meglumine salt, diethanolamine salt, ethylenediamine salt, pyridine salt, picoline salt or quinolone salt; amino acid salt; inorganic acid salt such as hydrochloride, sulfate, nitrate, carbonate, hydrobromate, phosphate or hydroiodide; organic acid salt such as formate, acetate, propionate, trifluoroacetate, citrate, lactate, tartrate, oxalate, maleate, fumarate, mandelic acid salt, glutaric acid salt, malate, benzoate, phthalate, ascorbate, benzenesulfonate, p-toluenesulfonate, methanesulfonate or ethanesulfonate. Preferable is hydrochloride, sulfate, phosphate, tartrate or methanesulfonate. These salts can be prepared by methods known in the art.

The compound of the present invention or its pharmaceutically acceptable salt can form a solvate (e.g., alcohol solvate, hydrate or the like) and/or a crystal polymorph, and the present invention contains such solvates and crystal polymorph of various types. A solvate may be coordinated with an arbitrary number of solvent molecules (e.g., water molecules) in the compound of the present invention. The compound of the present invention or its pharmaceutically acceptable salt may be left in the atmosphere to absorb moisture, and a case where adsorbed water is attached or a case where hydrate is formed may arise. Moreover, the compound of the present invention or its pharmaceutically acceptable salt may be recrystallized to form their crystal polymorph.

A compound of the present invention or its pharmaceutically acceptable salt can form a prodrug, and the present invention also contains such various types of prodrug. The prodrugs are a derivative of a compound of the present invention, which has a chemically or metabolically decomposable group, and a compound which is changed into a compound of the present invention, which is pharmaceutically active, by solvolysis or in vivo under physiological conditions. The prodrugs contain a compound which is converted into a compound of the present invention by enzymatic oxidation, reduction, hydrolysis and the like in living organisms under physiological conditions; a compound which is converted into a compound of the present invention by hydrolysis by e.g. gastric acid; and the like. A method for selecting and a method for producing a proper prodrug derivative are described in e.g. Design of Prodrugs, Elsevier, Amsterdam 1985. Prodrugs can have activity in theirself.

When a compound of the present invention or its pharmaceutically acceptable salt has a hydroxyl group, prodrugs such as an acyloxy derivative and a sulfonyloxy derivative are exemplified, which derivatives are produced, for example, by a reaction of a compound having a hydroxy group and a proper acyl halide, a proper acid anhydride, a proper sulfonyl chloride, a proper sulfonyl anhydride and a mixed anhydride, or a reaction using a condensing agent. Examples thereof include $CH_3COO$-, $C_2H_5COO$-, t-BuCOO-, $C_{15}H_{31}COO$-, PhCOO-, (m-NaOOCPh)COO-, $NaOOCCH_2CH_2COO$-, $CH_3CH(NH_2)COO$-, $CH_2N(CH_3)_2COO$-, $CH_3SO_3$-, $CH_3CH_2SO_3$-, $CF_3SO_3$-, $CH_2FSO_3$-, $CF_3CH_2SO_3$-, p-$CH_3$-O-$PhSO_3$-, $PhSO_3$-and p-$CH_3PhSO_3$-.

The term "inhibition" means that the compound of the present invention inhibits the action of 11βHSD-1.

The term "pharmaceutically acceptable" means preventively or therapeutically harmless.

A general method for producing a compound of the present invention will be illustrated below. For extraction, purification and the like, treatment which is carried out in common experiments in organic chemistry may be carried out.

A compound represented by the Formula (IV) can be synthesized as follows.

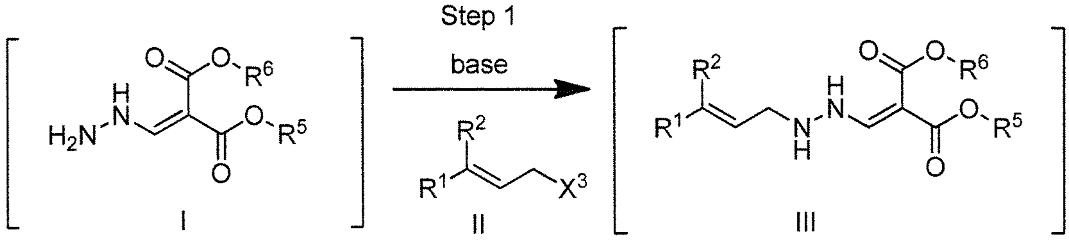


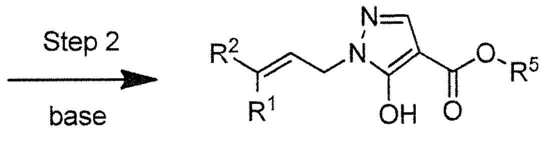


IV

wherein, each symbol has the same meaning as above, and as a compound represented by the formula (I), a known compound can be used and a compound which is derived from a known compound by a conventional method can be used.

Step 1

Step 1 is a step for producing a compound represented by the formula (III) by reacting a compound represented by the formula (I) with a compound represented by the formula (II).
As a solvent, example includes an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), saturated hydrocarbons (e.g., cyclohexane, hexane or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like), water, a mixed solvent thereof or the like.
Preferably, an aprotic polar solvent (e.g., N,N-dimethylformamide, 1-methyl-2-pyrrolidone, N,N-dimethylacetamide), dimethylsulfoxide, halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like), water or a mixed solvent thereof can be used. In particular, an aprotic polar solvent (e.g., N,N-dimethylformamide, 1-methyl-2-pyrrolidone, N,N-dimethylacetamide) is preferable.
As a base, example includes metal hydrides (e.g., sodium hydride or the like), metal hydroxides (e.g., sodium hydroxide, potassium hydroxide, lithium hydroxide, barium hydroxide or the like), metal carbonates (e.g., sodium carbonate, potassium carbonate, calcium carbonate, cesium carbonate or the like), metal alkoxides (e.g., sodium methoxide, sodium ethoxide, potassium t-butoxide or the like), sodium hydrogen carbonate, metal sodium, metal amide, organic amines (e.g., triethylamine, diisopropylethylamine, DBU, pyridine, 2,6-lutidine or the like), alkyllithiums (n-BuLi, sec-BuLi or tert-BuLi) or the like.
Preferably, metal hydrides (e.g., sodium hydride or the like), metal carbonates (e.g., sodium carbonate, potassium carbonate, calcium carbonate, cesium carbonate or the like), metal alkoxides (e.g., sodium methoxide, sodium ethoxide, potassium t-butoxide or the like), sodium hydrogen carbonate, metal amide or organic amines (e.g., triethylamine, diisopropylethylamine, DBU, pyridine, 2,6-lutidine or the like) can be used. In particular, organic amines (e.g., triethylamine, diisopropylethylamine, DBU, pyridine, 2,6-lutidine or the like) are preferable.
The reaction can be carried out at -40°C to a temperature at which a solvent being used is refluxed, preferably -10 to 25°C for 0.5 to 48 hours.
As a compound represented by the formula (II), example includes prenyl bromide or the like.

Step 2

Step 2 is a step for producing a compound represented by the formula (IV) from a compound represented by the formula (III).
As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g.,

toluene, benzene, xylene or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like), water or a mixed solvent thereof can be used. In particular, an aprotic polar solvent (e.g., N,N-dimethylformamide, 1-methyl-2-pyrrolidone, N,N-dimethylacetamide) is preferable.

As a base, a base described in the step 1 can be used. Preferably, metal hydrides (e.g. sodium hydride etc.), metal carbonates (e.g. sodium carbonate, calcium carbonate, cesium carbonate etc.), metal alkoxides (e.g. sodium methoxide, sodium ethoxide, potassium t-butoxide etc.), sodium hydrogen carbonate, metal amides or organic amines (e.g. triethylamine, diisopropylethylamine, DBU, 2,6-lutidine etc.) can be used.

Particularly preferable is metal carbonates (e.g. sodium carbonate, calcium carbonate, cesium carbonate etc.).

The reaction can be carried out at -40°C to a temperature at which a solvent being used is refluxed, preferably 40 to 70°C. for 0.5 to 24 hours.

A compound represented by the Formula (XI) can be synthesized as follows.

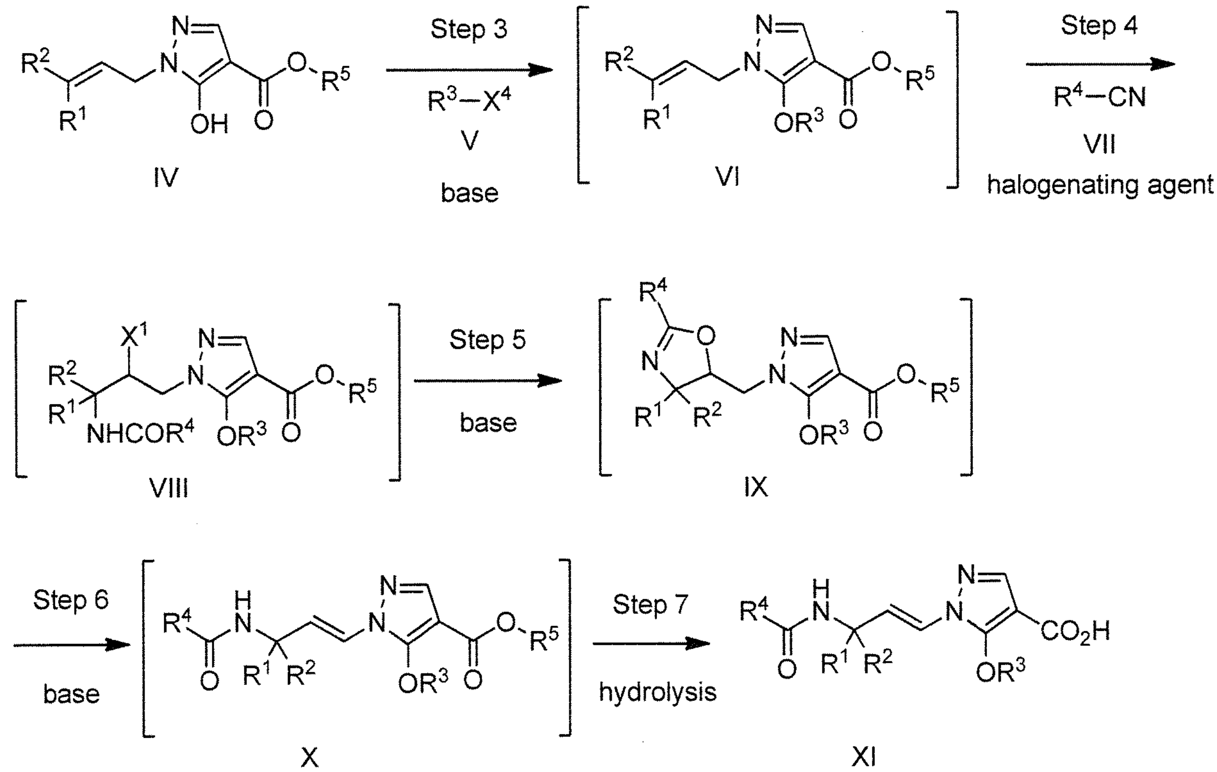


wherein, each symbol has the same meaning as above.

Step 3

Step 3 is a step for producing a compound represented by the formula (VI) by reacting a compound represented by the formula (IV) with a compound represented by the formula (V).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), water or a mixed solvent thereof can be used. Particularly preferable is an aprotic polar solvent (e.g., N,N-dimethylformamide, 1-methyl-2-pyrrolidone, N,N-dimethylacetamide).

As a base, a base described in the step 1 can be used. Preferably, metal hydrides (e.g., sodium hydride or the like), metal carbonates (e.g., sodium carbonate, potassium carbonate, calcium carbonate, cesium carbonate or the like), metal alkoxides (e.g., sodium methoxide, sodium ethoxide, potassium t-butoxide or the like), sodium hydrogen carbonate, metal amide, organic amines (e.g., triethylamine, diisopropylethylamine, DBU, pyridine, 2,6-lutidine or the like) can be used. Particularly preferable is metal carbonates (e.g., sodium carbonate, potassium carbonate, calcium carbonate, cesium carbonate or the like).

The reaction can be carried out at -40°C to a temperature at which a solvent being used is refluxed, preferably 10 to 100°C for 0.5 to 24 hours.
As a compound represented by the formula (V), example includes ethyl bromide, propyl bromide, isopropyl bromide, isobutyl bromide or the like.

Step 4

Step 4 is a step for producing a compound represented by the formula (VIII) by reacting a compound represented by the formula (VI) with a compound represented by the formula (VII) in the presence of a halogenating agent.
As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), saturated hydrocarbons (e.g., cyclohexane, hexane or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), water or a mixed solvent thereof can be used. Particularly preferable is an aprotic polar solvent (e.g., N,N-dimethylformamide, 1-methyl-2-pyrrolidone, N,N-dimethylacetamide) or nitriles (e.g., acetonitrile or the like).
As a halogenating agent, N-halogenosuccinimide (e.g., N-bromosuccinimide or the like), N-halogenoacetamide (e.g., N-bromoacetamide or the like), halogen (e.g., $I_2$ or the like) or the like can be used. Particularly preferable is N-bromosuccinimide.
Preferably, the reaction can be conducted in the presence of a Lewis acid.
As a Lewis acid, boron trifluoride ether complex(e.g., boron trifluoride n-butyl ether complex, boron trifluoride diethyl ether complex or the like), metal halide(e.g., $SnCl_4$, $AlCl_3$ or the like) or the like can be used. Particularly preferable is boron trifluoride n-butyl ether complex.
The reaction can be carried out at -78°C to a temperature at which a solvent being used is refluxed, preferably -40°C to room temperature for 0.5 to 12 hours.
As a compound represented by the formula (VII), example includes acetonitrile or the like.

Step 5

Step 5 is a step for producing a compound represented by the formula (IX) by reacting a compound represented by the formula (VIII) with a base.
As a solvent, a solvent described in the step 1 can be used. Pr eferably, an aprotic polar solvent (e.g., N,N-dimethylformamide, 1-methyl-2-pyr rolidone, N,N-dimethylacetamide), dimethylsulfoxide, aromatic hydrocarbons (e.g ., toluene, benzene, xylene or the like), halogenated hydrocarbons (e.g., dichlor omethane, chloroform, 1,2-dichloroethane or the like), ethers (e.g., tetrahydrofu ran, diethyl ether, dioxane, 1,2-dimethoxyethane or the like), esters (e.g., meth yl acetate, ethyl acetate or the like), ketones (e.g., acetone, methylethylketone or the like), nitriles (e.g., acetonitrile or the like), alcohols (e.g., methanol, eth anol, t-butanol or the like), water or a mixed solvent thereof can be used. Pa rticularly preferable is an aprotic polar solvent (e.g., N,N-dimethylformamide, 1-methyl-2-pyrrolidone, N,N-dimethylacetamide).
As a base, a base described in the step 1 can be used. Preferably, metal hydrides (e.g., sodium hydride or the like), metal carbonates (e.g., sodium carbonate, potassium carbonate, calcium carbonate, cesium carbonate or the like), sodium hydrogen carbonate, metal sodium, metal amide or organic amines (e.g., triethylamine, diisopropylethylamine, DBU, pyridine, 2,6-lutidine or the like) can be used. Particularly preferable is organic amines (e.g., triethylamine, diisopropylethylamine, DBU, pyridine, 2,6-lutidine or the like).
The reaction can be carried out at -40°C to a temperature at which a solvent being used is refluxed, preferably 45 to 75°C for 0.5 to 24 hours.

Step 6

Step 6 is a step for producing a compound represented by the formula (X) from a compound represented by the formula (IX).
As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), saturated hydrocarbons (e.g., cyclohexane, hexane or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), nitriles (e.g., acetonitrile or the like) or a mixed solvent thereof can be used. Particularly preferable is an aprotic polar solvent (e.g., N,N-dimethylformamide, 1-methyl-2-pyrrolidone, N,N-dimethylacetamide), aromatic hydrocarbons (e.g., toluene, benzene, xylene or the like) or a mixed solvent thereof.

As a base, a base described in the step 1 can be used. Preferably, metal hydrides (e.g., sodium hydride or the like), metal hydroxides (e.g., sodium hydroxide, potassium hydroxide, lithium hydroxide, barium hydroxide or the like), metal carbonates (e.g., sodium carbonate, potassium carbonate, calcium carbonate, cesium carbonate or the like), metal alkoxides (e.g., sodium methoxide, sodium ethoxide, potassium t-butoxide or the like), sodium hydrogen carbonate, metal sodium or metal amide can be used.

Particularly preferable is metal alkoxides (e.g., sodium methoxide, sodium ethoxide, potassium t-butoxide or the like).

The reaction can be carried out at -78°C to a temperature at which a solvent being used is refluxed, preferably -40°C to room temperature for 0.5 to 12 hours.

Step 7

Step 7 is a step for producing a compound represented by the formula (XI) by hydrolyzing a compound represented by the formula (X).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like), water or a mixed solvent thereof can be used. Particularly preferable is an aprotic polar solvent (e.g., N,N-dimethylformamide, 1-methyl-2-pyrrolidone, N,N-dimethylacetamide) or a mixed solvent of alcohols (e.g., methanol, ethanol, t-butanol or the like) and water.

As a base, a base described in the step 1 can be used. Preferably, metal hydroxides (e.g., sodium hydroxide, potassium hydroxide, lithium hydroxide, barium hydroxide or the like), metal carbonates (e.g., sodium carbonate, potassium carbonate, calcium carbonate, cesium carbonate or the like) or sodium hydrogen carbonate can be used.

Particularly preferable is metal hydroxides (e.g., sodium hydroxide, potassium hydroxide, lithium hydroxide, barium hydroxide or the like).

The reaction can be carried out at 0°C to a temperature at which a solvent being used is refluxed, preferably 40 to 60°C for 0.5 to 12 hours.

A compound represented by the Formula (XI) can be synthesized as follows.

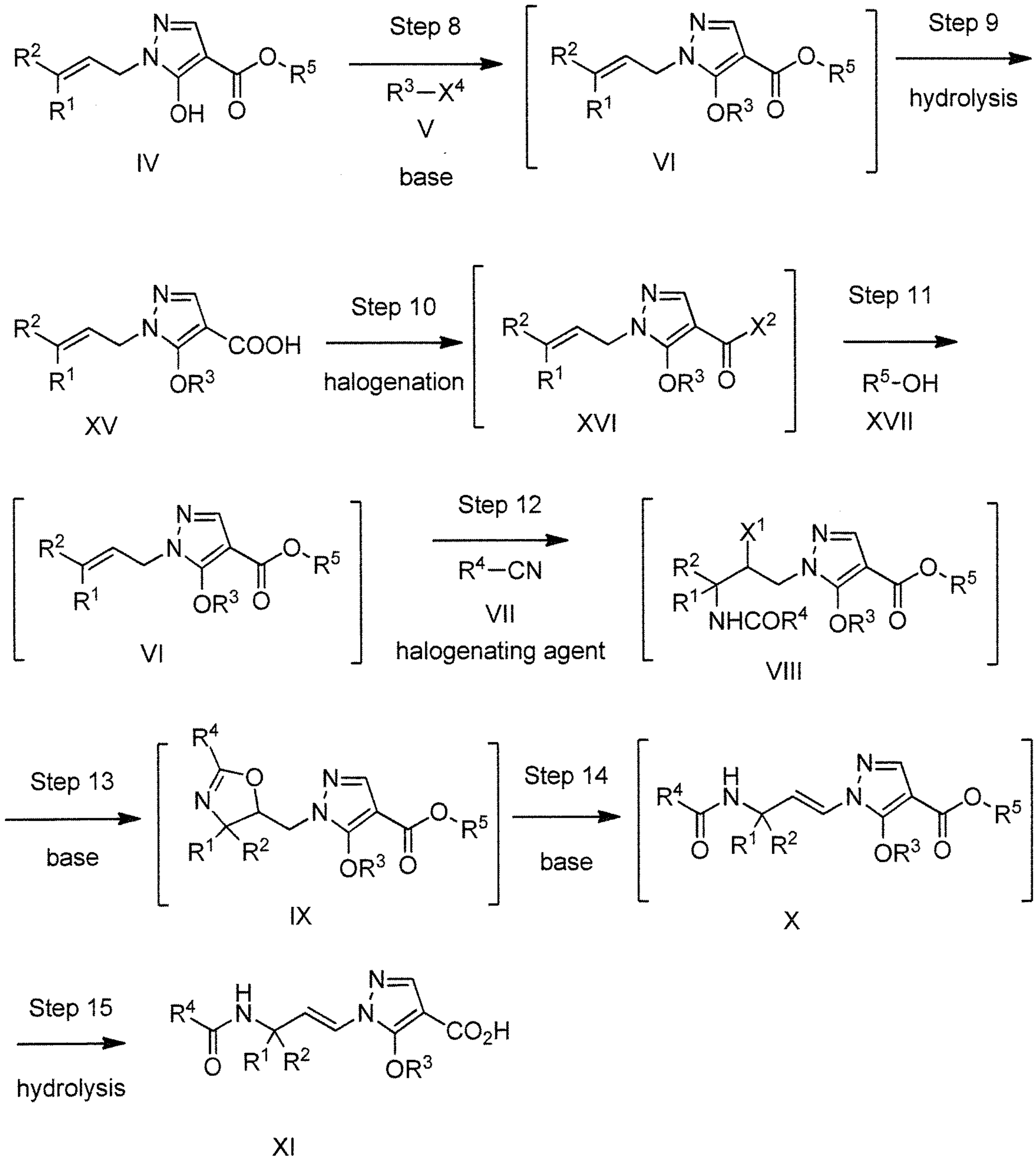


wherein, each symbol has the same meaning as above.

Step 8

Step 8 is a step for producing a compound represented by the formula (VI) by reacting a compound represented by the formula (IV) with a compound represented by the formula (V), provided that $R^3$ and $R^5$ are not the same group.

The reaction can be performed under the same conditions as the above Step 3.

Step 9

Step 9 is a step for producing a compound represented by the formula (XV) by hydrolyzing a compound represented by the formula (VI).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), ketones (e.g., acetone, methylethylketone or the like), nitriles (e.g., acetonitrile or the

like), alcohols (e.g., methanol, ethanol, t-butanol or the like), water or a mixed solvent thereof can be used. Particularly preferable is an aprotic polar solvent (e.g., N,N-dimethylformamide, 1-methyl-2-pyrrolidone, N,N-dimethylacetamide) or a mixed solvent of alcohols (e.g., methanol, ethanol, t-butanol or the like) and water.

As a base, a base described in the step 1 can be used. Preferably, metal hydroxides (e.g., sodium hydroxide, potassium hydroxide, lithium hydroxide, barium hydroxide or the like), metal carbonates (e.g., sodium carbonate, potassium carbonate, calcium carbonate, cesium carbonate or the like) or sodium hydrogen carbonate can be used.

Particularly preferable is metal hydroxides (e.g., sodium hydroxide, potassium hydroxide, lithium hydroxide, barium hydroxide or the like).

The reaction can be carried out at 0°C to a temperature at which a solvent being used is refluxed, preferably 60°C to a temperature at which a solvent being used is refluxed for 0.5 to 12 hours.

Step 10

Step 10 is a step for producing a compound represented by the formula (XVI) from a compound represented by the formula (XV).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like) or a mixed solvent thereof can be used. Particularly preferable is an aprotic polar solvent (e.g., N,N-dimethylformamide, 1-methyl-2-pyrrolidone, N,N-dimethylacetamide).

A compound represented by the formula (XV) can be reacted with a halogenating agent.

As a halogenating agent, phosphorous oxychloride, phosphorous pentachloride, oxalyl chloride, thionyl chloride, sulfuryl chloride, dichlorotriphenylphosphorane or the like can be used. The agent is preferably phosphorous oxychloride, phosphorous pentachloride, oxalyl chloride or thionyl chloride. Particularly preferable is thionyl chloride.

The reaction can be carried out at -40°C to a temperature at which a solvent being used is refluxed, preferably -10 to 40°C for 0.5 to 12 hours.

Step 11

Step 11 is a step for producing a compound represented by the formula (VI) by reacting a compound represented by the formula (XVI) with a compound represented by the formula (XVII), wherein $R^3$ is the same group as $R^5$.

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like) or a mixed solvent thereof can be used. Particularly preferable is an aprotic polar solvent (e.g., N,N-dimethylformamide, 1-methyl-2-pyrrolidone, N,N-dimethylacetamide).

The reaction can be carried out at 0°C to a temperature at which a solvent being used is refluxed, preferably 60°C to a temperature at which a solvent being used is refluxed for 0.5 to 12 hours.

As a compound represented by the formula (XVII), example includes ethanol, propanol, isopropyl alcohol, isobutyl alcohol or the like.

Step 12

Step 12 is a step for producing a compound represented by the formula (VIII) by reacting a compound represented by the formula (VI) with a compound represented by the formula (VII) in the presence of a halogenating agent, wherein $R^3$ is the same group as $R^5$.

The reaction can be performed under the same conditions as the above Step 4.

Step 13

Step 13 is a step for producing a compound represented by the formula (IX) by reacting a compound represented by the formula (VIII) with a base, wherein $R^3$ is the same group as $R^5$.

The reaction can be performed under the same conditions as the above Step 5.

Step 14

Step 14 is a step for producing a compound represented by the formula (X) from a compound represented by the formula (IX), wherein $R^3$ is the same group as $R^5$.

The reaction can be performed under the same conditions as the above Step 6.

Step 15

Step 15 is a step for producing a compound represented by the formula (XI) by hydrolyzing a compound represented by the formula (X), wherein $R^3$ is the same group as $R^5$.

The reaction can be performed under the same conditions as the above Step 7.

A compound represented by the Formula (XIII) can be synthesized as follows.

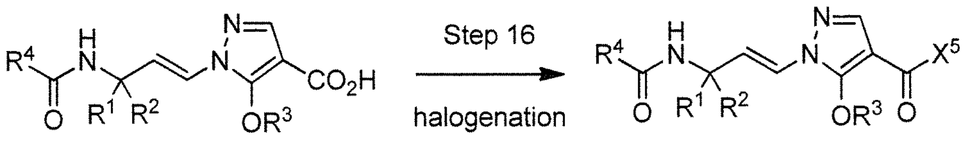


XI XIV

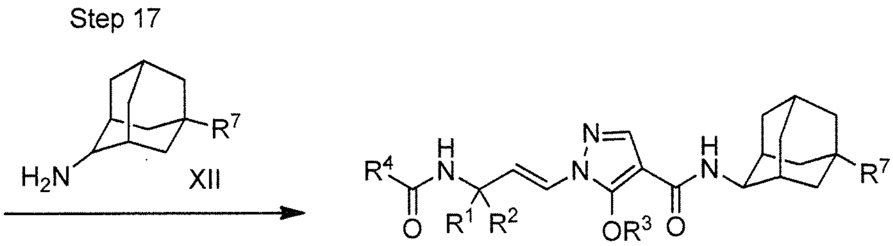


XIII

wherein, each symbol has the same meaning as above.

Step 16

Step 16 is a step for producing a compound represented by the formula (XIV) from a compound represented by the formula (XI).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), halogenated hydrocarbons (e.g., dichloromethane, chloroform, 1,2-dichloroethane or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like) or a mixed solvent thereof can be used. Particularly preferable is an aprotic polar solvent (e.g., N,N-dimethylformamide, 1-methyl-2-pyrrolidone, N,N-dimethylacetamide).

As a halogenating agent, phosphorous oxychloride, phosphorous pentachloride, oxalyl chloride, thionyl chloride, sulfuryl chloride, dichlorotriphenylphosphorane or the like can be used. The agent is preferably phosphorous oxychloride, phosphorous pentachloride, oxalyl chloride or thionyl chloride. Particularly preferable is thionyl chloride.

The reaction can be carried out at -40°C to a temperature at which a solvent being used is refluxed, preferably -20°C to room temperature for 0.5 to 12 hours.

Step 17

Step 17 is a step for producing a compound represented by the formula (XIII) by reacting a compound represented by the formula (XIV) with a compound represented by the formula (XII).

As a solvent, a solvent described in the step 1 can be used. Preferably, an aprotic polar solvent (e.g., N,N-dimethylformamide, 1-methyl-2-pyrrolidone, N,N-dimethylacetamide), dimethylsulfoxide, aromatic hydrocarbons (e.g., toluene, benzene, xylene or the like), halogenated hydrocarbons (e.g., dichloromethane, chloroform, 1,2-dichloroethane

or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), water or a mixed solvent thereof can be used. Particularly preferable is an aprotic polar solvent (e.g., N,N-dimethylformamide, 1-methyl-2-pyrrolidone, N,N-dimethylacetamide) or ethers (e.g., tetrahydrofuran, diethyl ether, dioxane, 1,2-dimethoxyethane or the like).

As a base, a base described in the step 1 can be used. Preferably, metal hydroxides (e.g., sodium hydroxide, potassium hydroxide, lithium hydroxide, barium hydroxide or the like), metal carbonates (e.g., sodium carbonate, potassium carbonate, calcium carbonate, cesium carbonate or the like), sodium hydrogen carbonate or organic amines (e.g., triethylamine, diisopropylethylamine, DBU, pyridine, 2,6-lutidine or the like) can be used. Particularly preferable is metal hydroxides (e.g., sodium hydroxide, potassium hydroxide, lithium hydroxide, barium hydroxide or the like).

The reaction can be carried out at -20°C to a temperature at which a solvent being used is refluxed, preferably -10°C to room temperature for 0.5 to 12 hours.

The compound represented by the Formula (XIV) and the compound represented by the Formula (XII) can be used in a form of a salt or a solvate.

The compound represented by the Formula (XII) can be produced according to a method described in WO2011/07810 and WO2012/020724.

In Step 16 stated above, by using a reagent other than a halogenating agent, a compound represented by the Formula (XIV) which has a wide variety of leaving group as $X^5$ can be synthesized. Then, the compound represented by the Formula (XIII) can be produced according to a method described in step 17. The methods are encompassed by the present invention. The "leaving group" is not limited as long as it is a substituent which leaves when carboxylic acid and amine are condensed. Example includes acyloxy (e.g., acetyloxy, benzoyloxy or the like), substituted or unsubstituted alkylsulfonyloxy (e.g., methanesulfonyloxy, trifluoromethanesulfonyloxy or the like), substituted or unsubstituted benzene sulfonyloxy (e.g., paratoluene sulfonyloxy, orthonitrobenzene sulfonyloxy or the like), N,N'-dicyclohexylcarbamimidoyloxy, N,N'-diisopropylcarbamimidoyloxy, (N-(3-(dimethylamino)propyl)-N'-ethylcarbamimidoyloxy or the like.

For example, by reacting acyl halide with a compound represented by the Formula (XI) in the presence of a base, a compound represented by the Formula (XIV) having acyloxy as a "leaving group" can be produced. By reacting alkylsulfonyl halide with a compound represented by the Formula (XI) in the presence of a base, a compound represented by the Formula (XIV) having alkylsulfonyloxy as a "leaving group" can be produced. The same holds true for others.

A compound represented by the Formula (XIII) can be synthesized as follows.

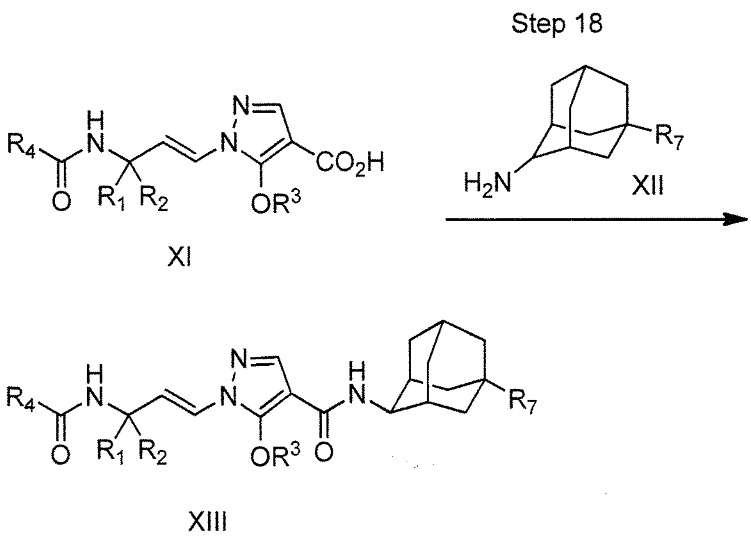


XI

XII

XIII

wherein, each symbol has the same meaning as above.

Step 18

Step 18 is a step for producing a compound represented by the formula (XIII) by reacting a compound represented by the formula (XI) with a compound represented by the formula (XII).

As a solvent, a solvent described in the step 1 can be used.

The process can be performed in the presence of a condensing agent.

As a condensing agent, an amide condensing agent used in condensation of carboxyl group and amino group can be used. For example, carbodiimides (e.g., N,N'-dicyclohexylcarbodiimide, N,N'-diisopropylcarbodiimide, 1-ethyl-3-(3-dimethylaminopropyl)carbodiimide hydrochloride or the like), diphenylphosphoryl azide, BOP reagents (e.g., BOP, PyBop, TBTU or the like), DMT-MM, 1,1'-carbonylbis-1H-imidazole, 2-chloro-1,3-dimethylimidazolium chloride, 4-(4,6-dimethoxy-1,3,5-triazin-2-yl)-4-methylmorpholinium chloride or the like can be used. Preferably, N,N'-dicyclohexylcarbodiimide, N,N'-diisopropylcarbodiimide or 1-ethyl-3-(3-dimethylaminopropyl)carbodiimide hydrochloride can be used. Particularly preferable is 1-ethyl-3-(3-dimethylaminopropyl)carbodiimide hydrochloride.

The step can be carried out in the presence of an additive.

As an additive, 1-hydroxybenzotriazole, N-hydroxysuccinimide or the like can be used. The additive is, in particular, preferably 1-hydroxybenzotriazole.

Unless otherwise stated, figures indicated in the description and in the claims are approximate. Variations of the figures are attributed to device calibrations, device errors, purity of substances, crystal sizes, sample sizes and other factors.

The "crystal" used in the description means the substance which possesses the long range of well-ordered molecular structures. The degree of crystallization of a crystal form can be measured by many techniques including for example, powder X-ray diffraction, moisture adsorption, differential scanning calorimetric analysis, solution colorimetric analysis and solubility property.

A compound represented by the Formula (III), a compound represented by the Formula (IV), a compound represented by the Formula (VI), a compound represented by the Formula (VIII), a compound represented by the Formula (IX), a compound represented by the Formula (X), a compound represented by the Formula (XI) and a compound represented by the Formula (XV) are useful as an intermediate to produce a compound represented by the Formula (XIII).

Among these intermediates, crystals described in the present description are, in particular, preferable. For example, crystals with two or more 2θ values selected from values of 2θ (± 0.2°) of powder X-ray diffraction presented in the present description, crystals with three or more of those 2θ values, crystals with four or more of those 2θ values, crystals with five or more of those 2θ values, and others are preferable.

Various types of substituent of the compounds of the present invention can be introduced by reference to (1) Alan R. Katriszly et al., Comprehensive Heterocyclic Chemistry, (2) Alan R. Katriszly et al., Comprehensive Heterocyclic Chemistry II, (3) RODD'S CHEMISTRY OF CARBON COMPOUNDS VOLUME IV HETEROCYCLIC COMPOUNDS or the like.

The present invention is further explained by the following Examples, which are not intended to limit the scope of the present invention.

NMR analysis obtained in each example was measured by 300 MHz, and measured using $CDCl_3$ or dimethylsulfoxide (d6-DMSO).

log k' is a value which means degree of the lipophilic character, and is calculated by the following formula.

$$\log k' = \log(t_R - t_0)/t_0$$

$t_R$ : retention time of compound under gradient condition

$t_0$ : retention time of standard material not retained in column

XTerra MS C18 5μm, 2.1×100mm column(made by Waters) was used for measurement. The elution was a straight line inclination of acetonitrile/pH6.8 buffer (5:95~95:5/20min) at flow velocity 0.25mL/min.

Powder X-ray diffraction measurements of crystals were conducted under one of the following measuring conditions in accordance with the powder X-ray diffraction measuring method described in General Tests of the Japanese Pharmacopoeia.

(Device 1)

TTR III manufactured by Rigaku
(Operation Procedures)
Samples were measured under the following conditions.
Measuring Method: Reflection Method
Type of Light Source: Cu Tube
Wavelength Used: CuKα X-ray
Tube Electric Current: 300 mA
Tube Electric Voltage: 50 Kv
Sample Plate: Aluminum
Scan Speed: 5.000°/min.

Scan Range: 4.000 to 40.0000°
Sampling Width: 0.0200°

(Device 2)

MiniFlex II manufactured by Rigaku
(Operation Procedures)
Samples were measured under the following conditions.
Measuring Method: Reflection Method
Type of Light Source: Cu Tube
Wavelength Used: CuKα X-ray
Tube Electric Current: 15 mA
Tube Electric Voltage: 30 Kv
Sample Plate: Aluminum
Scan Speed: 5.000°/min.
Scan Range: 4.000 to 40.0000°
Sampling Width: 0.0200°

Generally, a diffraction angle (2θ) in powder X-ray diffraction is susceptible to error in a range of ± 0.2°, thus the value of a diffraction angle includes a margin of error of about ± 0.2°. Accordingly, the present invention includes not only crystals whose diffraction angles at the peak of powder X-ray diffraction are perfectly same, but also crystals whose diffraction angles at the peak are almost same with a margin of error of ± 0.2°.
The melting points were all measured with a melting point measuring device.

(Measuring Conditions)
Device: BUCHI Melting Point B-545
Temperature Rising Rate: 1°C/min.

[Example 1]

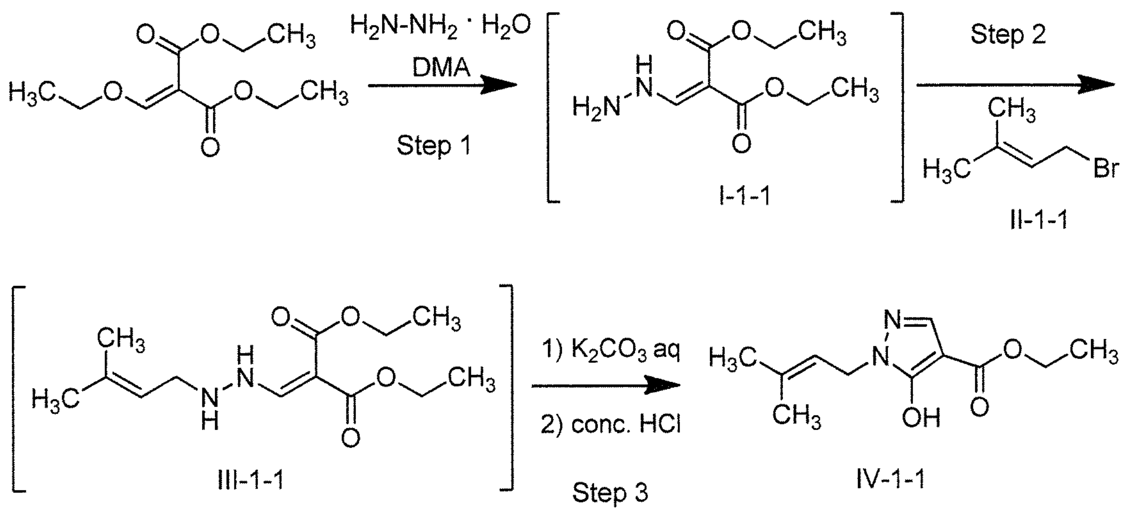


Step 1

A solution of Hydrazine hydrate (8.00 g, 159.8mmol) and N, N-dimethylacetoamide (96.8mL) was cooled to 10°C, and diethyl ethoxymethylene malonate (34.56 g, 159.8mmol) was added thereto for 1 hour. The reaction mixture was stirred for another 1 hour to obtain the reaction solution containing compound (I-1-1).

Step 2

The reaction solution obtained in Step 1 was cooled to 10°C, and diisopropyl ethylamine (30.98 g, 239.7 mmol) was added thereto. The reaction mixture was stirred for 10 minutes. To the reaction mixture was added prenyl bromide (11-1-1) (29.78 g, 199.8 mmol), and the reaction mixture was stirred for another 3 hours to obtain the reaction solution containing compound (III-1-1). The half of the reaction solution obtained in Step 2 was used at next process. $^1H$ NMR (d6-DMSO) δ(ppm) 1.18 (t, J=7.0Hz, 3H), 1.20 (t, J=7.1Hz, 3H), 1.57 ( s, 3H), 1.68 (s, 3H), 3.36 (brd, J=~7Hz, 2H), 4.02 (q, J=7.0Hz, 2H), 4.10 (q, J =7.1Hz, 2H), 5.15 (m, 1H), 5.61 (brs, 1H), 7.70 (d, J=12.3Hz, 1H), 9.77 (d, J=1 2.3Hz, 1H)

Step 3

The reaction solution (96.0g) obtained in Step 2 was warmed to 55°C, and 10%-potassium carbonate solution (138.06g) was added thereto. The reaction mixture was stirred for 3 and a half hour. The reaction solution was cooled to 25°C, and extracted with ethyl acetate (51.8mL). To the extracted solution were added ethanol (34mL) and concentrated hydrochloric acid (16.3g), and the mixture was concentrated under reduced pressure. The residue was crystallized at 0°C for one hour. The resulting crystal was filtered, and dried to obtain compound (IV-1-1) (2, 5.48g, 30.6%).
Melting point: 79.7~79.9°C.
$^1H$ NMR(d6-DMSO); δ(ppm) 1.24 (t, J=7.1Hz, 3H), 1.69 (brs, 3H), 1.72 (brs, 3 H),4.17 (q, J=7.1Hz, 2H), 4.47 (brd, J=~7Hz, 2H), 5.25 (m, 1H), 7.53 (s, 1H) Powder X-ray Diffraction 2θ (°); 8.2, 12.4, 18.3, 24.5, 25.6

Result of powder X-ray diffraction of Compound (IV-1-1) is shown in Fig. 1.

[Example2]

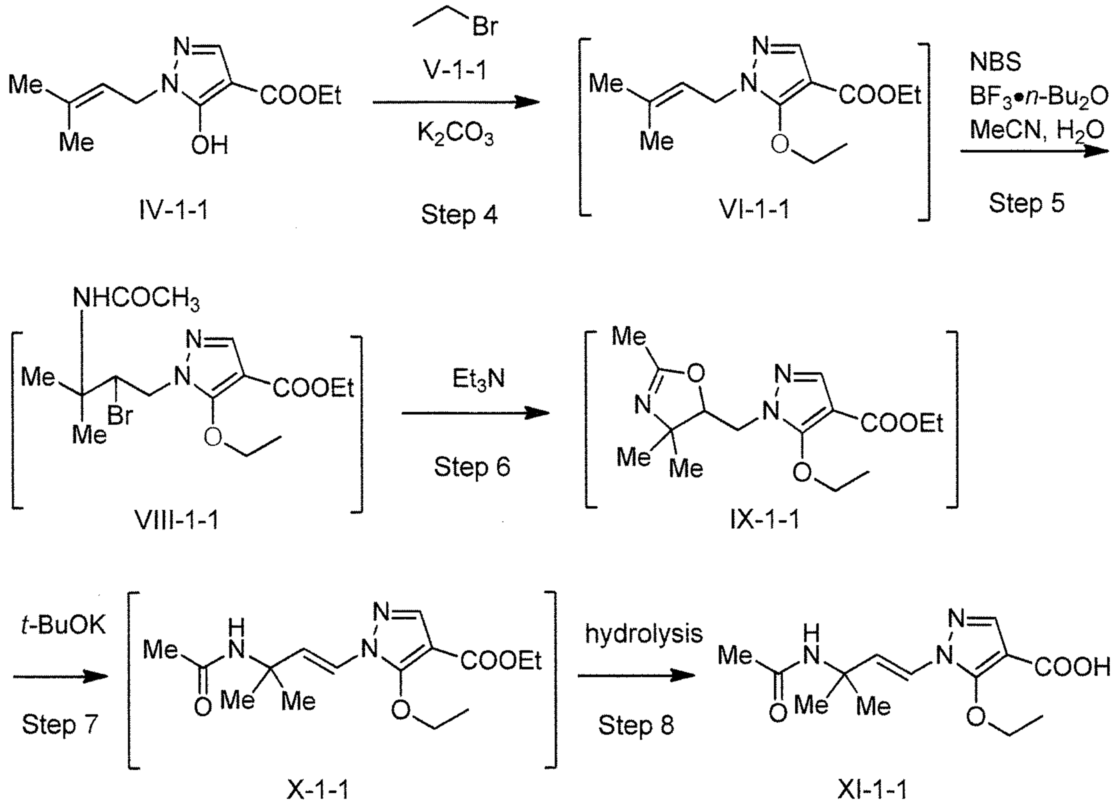


IV-1-1 VI-1-1 VIII-1-1 IX-1-1 X-1-1 XI-1-1

Step 4

A solution of Compound (IV-1-1) (44.8 g, 199.8 mmol), potassium carbonate (41.4 g, 299.5 mmol), N, N-dimethylacetoamide (197mL) was warmed to 80°C, and a solution of compound (V-1-1) (27.2 g, 249.6 mmol) in N, N-dimethylacetoamide (49mL) was added thereto. The reaction mixture was stirred for one hour. The reaction solution was cooled to room temperature, and water (448mL) was added thereto. The reaction solution was extracted with toluene

(224mL). The extracted solution was washed two times with water (45mL). The reaction solution was concentrated under reduced pressure to obtain the concentrated solution containing compound (VI-1-1).

Step 5

A solution of N-bromosuccinimide (39.2 g, 220.2 mmol), boron trifluoride n-butyl ether complex (138.4 g, 698.9 mmol) and acetonitrile (157mL) was cooled to -20°C, and a mixed solution of the concentrated solution obtained in Step 4, water (3.6 g, 199.8mmol) and acetonitrile (200mL) was added dropwise thereto. The reaction was performed for 2 hours.

Step 6

To the reaction solution containing the resulting compound (VIII-1-1) was added triethylamine (101.0 g, 998.1 mmol), and the reaction mixture was stirred at 60°C for 3.5 hours. The reaction solution was cooled to room temperature, and toluene (179mL) was added thereto. The reaction solution was stirred for 1 hour, and the precipitate was filtered. The residue was washed with toluene (224mL). The filtrate was cooled to 5°C, and extracted with 18%-sodium hydroxide aqueous solution (353.4g). The extracted solution was washed two times respectively with 5%-sodium hydroxide aqueous solution (159.8g) and water (268mL). The extracted solution was concentrated under reduced pressure to obtain the concentrated solution containing compound (IX-1-1).

Step 7

To the concentrated solution obtained in Step 6 was added N, N-dimethylacetoamide (134mL), and the reaction mixture was cooled to -20°C. A solution of tert-butoxy potassium (23.4 g, 208.5 mmol) in N, N-dimethylacetoamide (246mL) was added dropwise thereto, and the reaction mixture was stirred for 1.5 hours. To the reaction solution was added water (44.8mL) to obtain the reaction solution containing compound (X-1-1).

Step 8

The reaction solution obtained in Step 7 was warmed to 50°C, and 8%-sodium hydroxide aqueous solution (299.6g) was added thereto. The reaction mixture was stirred for 3.5 hours. The reaction solution was cooled to room temperature, and extracted with toluene (134mL). To lower layer of the extracted solution was added concentrated hydrochloric acid (137.6g), then extracted with ethyl acetate. The extracted solution was washed respectively with 20%-sodium chloride solution and water, and concentrated under reduced pressure. The concentrated solution was replaced with toluene, and was crystallized at 0°C for 1 hour. The precipitated crystal was filtered, and dried to obtain compound (XI-1-1) (26.82 g, 47.7%).
Melting point: 172.3~172.6°C
$^1$H NMR(d6-DMSO); δ(ppm) 1.30 (t, J=7.0Hz, 3H), 1.41 (s, 3H), 1.41 (s, 3H), 1 80 (s, 3H), 4.44 (q, J=7.0Hz, 2H), 6.42 (d, J=14.3Hz, 1H), 6.79 (d, J=14.3Hz, 1H), 7.78 (brs, 1H), 7.79 (brs, 1H), 12.45 (brs, 1H)
Powder X-ray Diffraction 2θ (°); 9.0, 13.0, 13.9, 15.0, 15.6, 18.9, 22.0, 22.3, 23.1, 24.3, 25.0, 25.4

Result of powder X-ray diffraction of Compound (XI-1-1) is shown in Fig. 2.

[Example3]

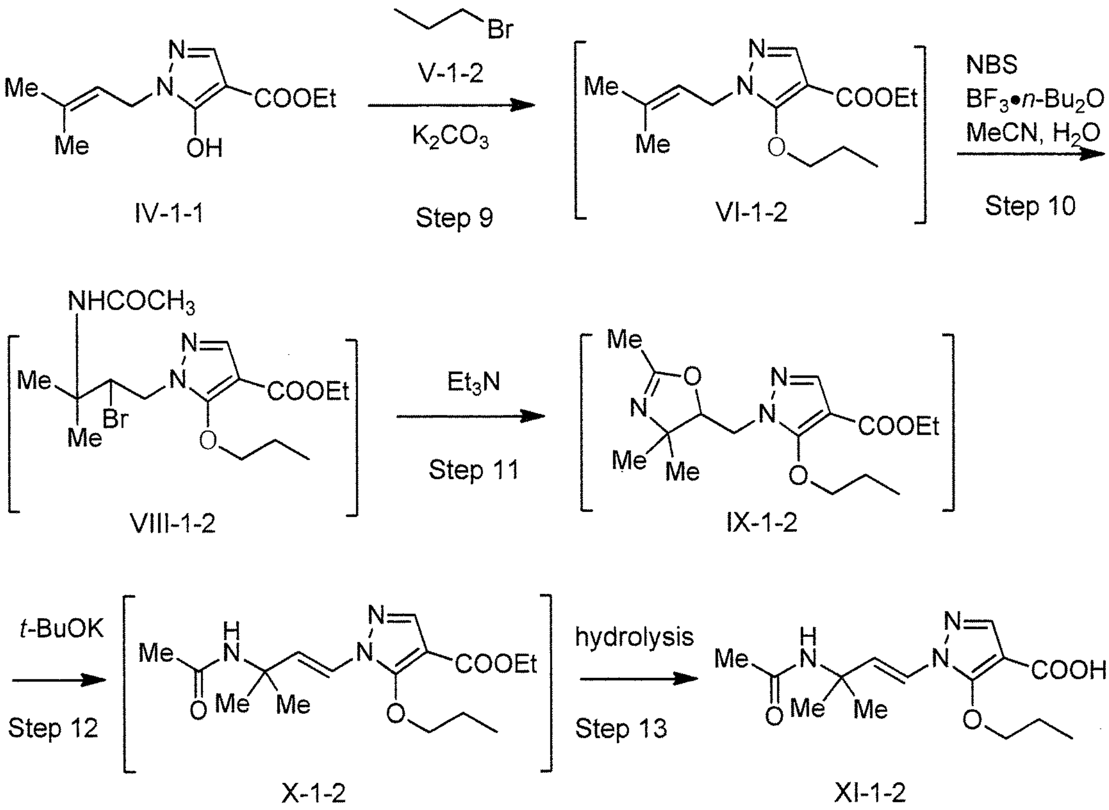


IV-1-1 VI-1-2 VIII-1-2 IX-1-2 X-1-2 XI-1-2

Step 9

A solution of Compound (IV-1-1) (44.8 g, 199.8 mmol), potassium carbonate (41.4 g, 299.5 mmol), N, N-dimethylacetoamide (197mL) was warmed to 80°C, and a solution of compound (V-1-2) (30.7 g, 249.6 mmol) in N, N-dimethylacetoamide (49mL) was added thereto. The reaction mixture was stirred for 1.5 hours. The reaction solution was cooled to room temperature, and water (448mL) was added thereto. The reaction solution was extracted with toluene (224mL). The extracted solution was washed two times with water (45mL). The extracted solution was concentrated under reduced pressure to obtain the concentrated solution containing compound (VI-1-2).

Step 10

A solution of N-bromosuccinimide (39.2 g, 220.2 mmol), boron trifluoride n-butyl ether complex (138.4 g, 698.9 mmol) and acetonitrile (155mL) was cooled to -20°C, and a mixed solution of the concentrated solution obtained in Step 9, water (3.6 g, 199.8mmol) and acetonitrile (200mL) was added dropwise thereto. The reaction was performed for 2 hours.

Step 11

To the reaction solution containing the resulting compound (VIII-1-2) was added triethylamine (101.0 g, 998.1 mmol), and the reaction mixture was stirred at 60°C for 3 hours. The reaction solution was cooled to room temperature, and toluene (180mL) was added thereto. The reaction solution was stirred for 1 hour, and the precipitate was filtered. The residue was washed with toluene (224mL). The filtrate was cooled to 5°C, and extracted with 18%-sodium hydroxide aqueous solution (353.2g). The extracted solution was washed two times respectively with 5%-sodium hydroxide aqueous solution (159.8g) and water (268mL). The extracted solution was concentrated under reduced pressure to obtain the concentrated solution containing compound (IX-1-2).

Step 12

To the concentrated solution obtained in Step 11 was added N, N-dimethylacetoamide (134mL), and the reaction mixture was cooled to -20°C. A solution of tert-butoxy potassium (21.4 g, 190.7 mmol) in N, N-dimethylacetoamide (245mL) was added dropwise thereto, and the reaction mixture was stirred for 1 hour. To the reaction solution was added water (44.8mL) to obtain the reaction solution containing compound (X-1-2).

Step 13

The reaction solution obtained in Step 12 was warmed to 50°C, and 8%-sodium hydroxide aqueous solution (299.6g) was added thereto. The reaction mixture was stirred for 1 hour. The reaction solution was cooled to room temperature, and extracted with toluene (134mL). To lower layer of the extracted solution was added concentrated hydrochloric acid (136.6g), then extracted with ethyl acetate. The extracted solution was washed respectively with 20%-sodium chloride solution and water, and concentrated under reduced pressure. The concentrated solution was replaced with toluene, and was crystallized at 0°C for 1 hour. The precipitated solid was filtered, and dried to obtain compound (XI-1-2) (28.75 g, 48.7%).
Melting point: 172.3~172.6°C
$^1$H NMR(d6-DMSO); δ(ppm) 0.98 (d, J=7.4Hz, 3H), 1.41 (s, 3H), 1.41 (s, 3H), 1.71 (m, 2H), 1.80 (s, 3H), 4.36 (t, J=6.4Hz, 2H), 6.41 (d, J=14.3Hz, 1H), 6.79 (d, J=14.3Hz, 1H), 7.78 (brs, 1H), 7.79 (brs, 1H), 12.44 (brs, 1H)
Powder X-ray Diffraction 2θ (°): 8.5, 11.4, 14.2, 18.1, 19.4, 21.1, 23.9, 26.4
Result of powder X-ray diffraction of Compound (XI-1-2) is shown in Fig. 3.

[Example4]

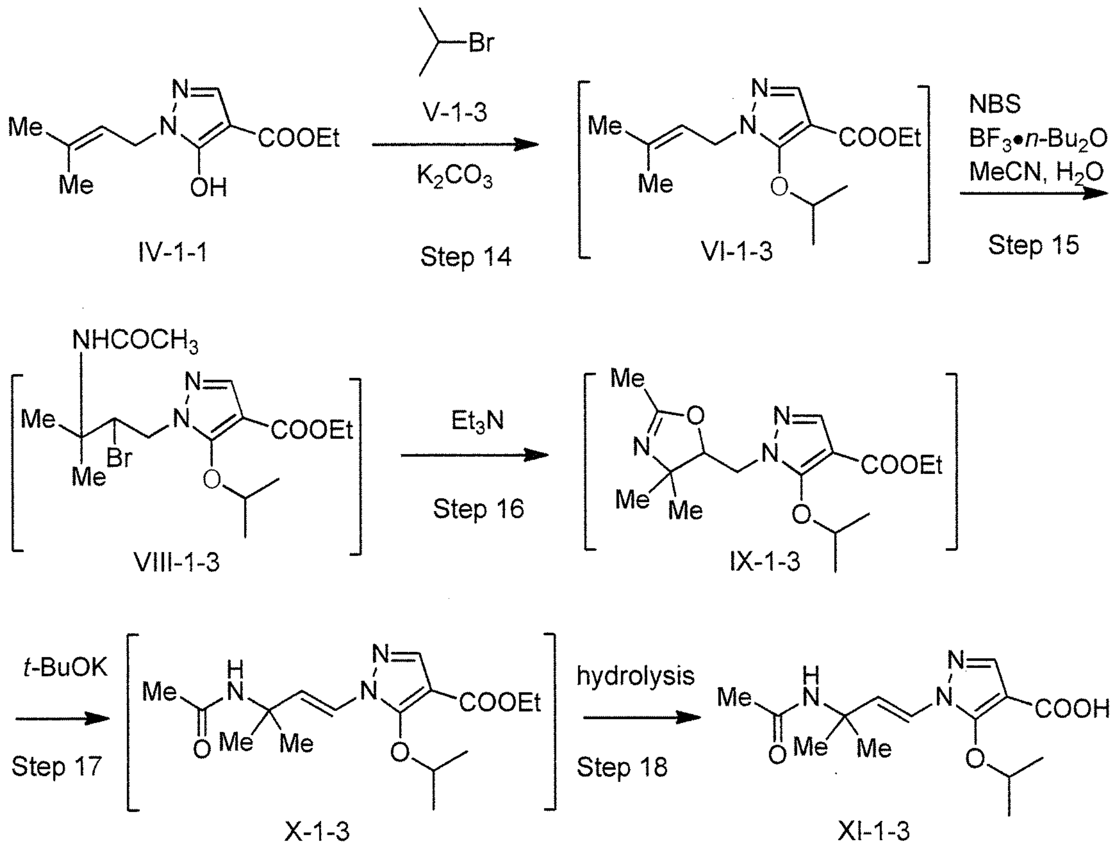


IV-1-1 VI-1-3 VIII-1-3 IX-1-3 X-1-3 XI-1-3

Step 14

A solution of Compound (IV-1-1) (22.4 g, 99.9 mmol), potassium carbonate (20.7 g, 149.8 mmol), N, N-dimethylacetoamide (98.6mL) was warmed to 80°C, and a solution of compound (V-1-3) (15.4 g, 124.9 mmol) in N, N-dimethylacetoamide (24.6mL) was added thereto. The reaction mixture was stirred for 3 hours. The reaction solution was cooled to room temperature, and water (224mL) was added thereto. The reaction solution was extracted with toluene (112mL). The extracted solution was washed two times with water (22mL). The reaction solution was concentrated under reduced pressure to obtain the concentrated solution containing compound (VI-1-3).

Step 15

A solution of N-bromosuccinimide (19.6 g, 110.1 mmol), boron trifluoride n-butyl ether complex (69.2 g, 349.7 mmol) and acetonitrile (89.6mL) was cooled to -20°C, and a mixed solution of the concentrated solution obtained in Step 14, water (1.8 g, 99.9mmol) and acetonitrile (89.6mL) was added dropwise thereto. The reaction was performed for 4 hours.

Step 16

To the reaction solution containing the resulting compound (VIII-1-3) was added triethylamine (50.5 g, 499.1 mmol), and the reaction mixture was stirred at 60°C for 3 hours. The reaction solution was cooled to room temperature, and toluene (89.6mL) was added thereto. The reaction solution was stirred for 1 hour, and the precipitate was filtered. The residue was washed with toluene (112mL). The filtrate was cooled to 5°C, and extracted with 18%-sodium hydroxide aqueous solution (176.7g). The extracted solution was washed two times respectively with 5%-sodium hydroxide aqueous solution (79.9g) and water (134mL). The extracted solution was concentrated under reduced pressure to obtain the concentrated solution containing compound (IX-1-3).
$^1$H NMR(d6-DMSO); δ(ppm) 1.13 (s, 3H), 1.20 (s, 3H), 1.27 (t, J=7.1Hz, 3H), 1 .28 (d, J=6.1Hz, 3H), 1.31 (d, J=6.1Hz, 3H), 1.79 (s, 3H), 4.12 (m, 2H), 4.20 ( q, J=7.1Hz, 2H), 4.45 (dd, J=5.2Hz, 7.8Hz, 1H), 5.00 (m, 1H), 7.78 (s, 1H)

Step 17

To the concentrated solution obtained in Step 16 was added N, N-dimethylacetoamide (67.2mL), and the reaction mixture was cooled to -20°C. A solution of tert-butoxy potassium (10.7 g, 94.9 mmol) in N, N-dimethylacetoamide (123.2mL) was added dropwise thereto, and the reaction mixture was stirred for 1 hour. To the reaction solution was added water (22.4mL) to obtain the reaction solution containing compound (X-1-3).
$^1$H NMR($CDCl_3$); δ(ppm) 1.35 (t, J=7.1Hz, 3H), 1.37 (d, J=6.1Hz, 6H), 1.54 (s, 6H), 1.95 (s, 3H), 4.27 (q, J=7.1Hz, 2H), 5.05 (qq, J=6.1Hz, 6.1Hz, 1H), 5.43 (brs, 1H), 6.40 (d, J=14.2Hz, 1H), 6.91 (dd, J=0.5Hz, 14.2Hz, 1H), 7.83 (d, J=0 . 5Hz, 1H)

Step 18

The reaction solution obtained in Step 17 was warmed to 50°C, and 8%-sodium hydroxide aqueous solution (149.8g) was added thereto. The reaction mixture was stirred for 1 hour. The reaction solution was cooled to room temperature, and extracted with toluene (67.2mL). To lower layer of the extracted solution was added concentrated hydrochloric acid (68.2g), then extracted with ethyl acetate. The extracted solution was washed respectively with 20%-sodium chloride solution and water, and concentrated under reduced pressure. The concentrated solution was replaced with toluene, and was crystallized at 0°C for 1 hour. The precipitated solid was filtered, and dried to obtain compound (XI-1-3) (type I crystal) (14.54 g, 49.3%).
Melting point: 183.9~184.4°C
$^1$H NMR(d6-DMSO); δ(ppm) 1.28 (d, J=6.2Hz, 3H), 1.28 (d, J=6.2Hz, 3H), 1.41 (s, 3H), 1.41 (s, 3H), 1.80 (s, 3H), 4.98 (q, J=6.2Hz, 1H), 6.39 (d, J=14.4Hz, 1H), 6.78 (d, J=14.3Hz, 1H), 7.78 (brs, 1H), 7.81 (brs, 1H), 12.43 (brs, 1H) Powder X-ray Diffraction 2θ (°): 8.7, 11.3, 14.3, 15.0, 17.5, 19.4, 21.1, 22.7, 24.3, 24.8, 26.0

[Example 5]

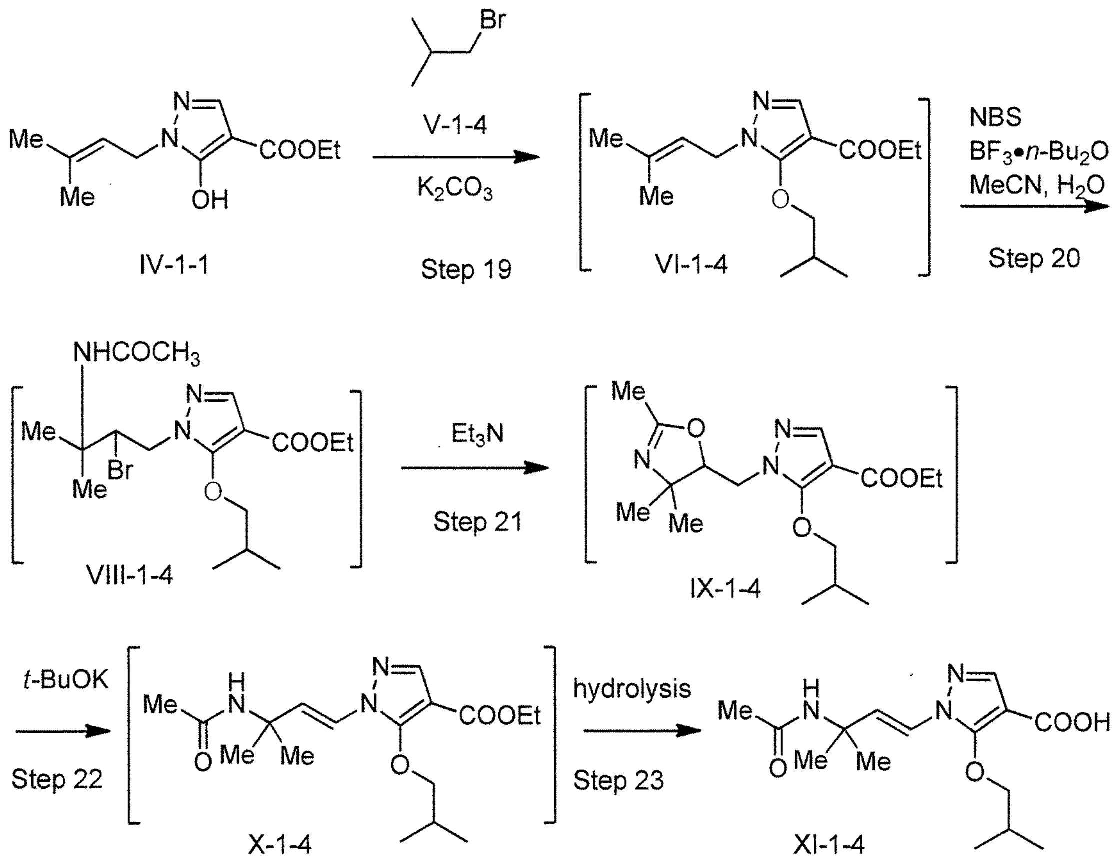

IV-1-1 VI-1-4 VIII-1-4 IX-1-4 X-1-4 XI-1-4

Step 19

A solution of Compound (IV-1-1) (22.4 g, 99.9 mmol), potassium carbonate (20.7 g, 149.8 mmol), N, N-dimethylacetoamide (99mL) was warmed to 80°C, and a solution of compound (V-1-4) (19.9 g, 144.9 mmol) in N, N-dimethylacetoamide (25mL) was added thereto. The reaction mixture was stirred for 7 hours. The reaction solution was cooled to room temperature, and water (224mL) was added thereto. The reaction solution was extracted with toluene (112mL). The extracted solution was washed two times with water (22mL). The extracted solution was concentrated under reduced pressure to obtain the concentrated solution containing compound (VI-1-4).

Step 20

A solution of N-bromosuccinimide (19.6 g, 110.1 mmol), boron trifluoride n-butyl ether complex (69.2 g, 349.4 mmol) and acetonitrile (79mL) was cooled to -20°C, and a mixed solution of the concentrated solution obtained in Step 19, water (3.6 g, 99.9mmol) and acetonitrile (100mL) was added dropwise thereto. The reaction was performed for 5 hours.

Step 21

To the reaction solution containing the resulting compound (VIII-1-4) was added triethylamine (50.5 g, 499.1 mmol), and the reaction mixture was stirred at 60°C for 3 hours. The reaction solution was cooled to room temperature, and toluene (90mL) was added thereto. The reaction solution was stirred for 1 hour, and the precipitate was filtered. The residue was washed with toluene (112mL). The filtrate was cooled to 5°C, and extracted with 18%-sodium hydroxide aqueous solution (176.7g). The extracted solution was washed two times respectively with 5%-sodium hydroxide aqueous solution (79.9g) and water (134mL). The extracted solution was concentrated under reduced pressure to obtain the concentrated solution containing compound (IX-1-4).

Step 22

To the concentrated solution obtained in Step 21 was added N, N-dimethylacetoamide (67mL), and the reaction mixture was cooled to -20°C. A solution of tert-butoxy potassium (10.7 g, 95.4 mmol) in N, N-dimethylacetoamide (123mL)

was added dropwise thereto, and the reaction mixture was stirred for 1.5 hours. To the reaction solution was added water (22.4mL) to obtain the reaction solution containing compound (X-1-4).

Step 23

The reaction solution obtained in Step 22 was warmed to 50°C, and 8%-sodium hydroxide aqueous solution (149.8g) was added thereto. The reaction mixture was stirred for 1 hour. The reaction solution was cooled to room temperature, and extracted with toluene (67mL). To lower layer of the extracted solution was added concentrated hydrochloric acid (67.9g), then extracted with ethyl acetate. The extracted solution was washed respectively with 20%-sodium chloride solution and water, and concentrated under reduced pressure. The concentrated solution was replaced with toluene, and was crystallized at 0°C for 1 hour. The precipitated solid was filtered, and dried to obtain compound (XI-1-4) (15.10 g, 48.9%).
Melting point: 166.3~166.5°C
$^1$H NMR(d6-DMSO); δ(ppm) 0.98 (d, J=6.7Hz, 3H), 0.98 (d, J=6.7Hz, 3H), 1.40 (s, 3H), 1.40 (s, 3H), 2.01 (m, 1H), 1.79 (s, 3H), 4.19 (d, J=6.2Hz, 2H), 6.39 ( d, J=14.3Hz, 1H), 6.80 (d, J=14.3Hz, 1H), 7.79 (brs, 1H), 7.79 (brs, 1H), 12.43 (brs, 1H)
Powder X-ray Diffraction 2θ (°): 10.6, 11.5, 17.6, 18.0, 18.6, 19.5, 19.9, 23.1, 2 4.8, 27.5, 27.9
Result of powder X-ray diffraction of Compound (XI-1-4) is shown in Fig. 5.

[Example6]

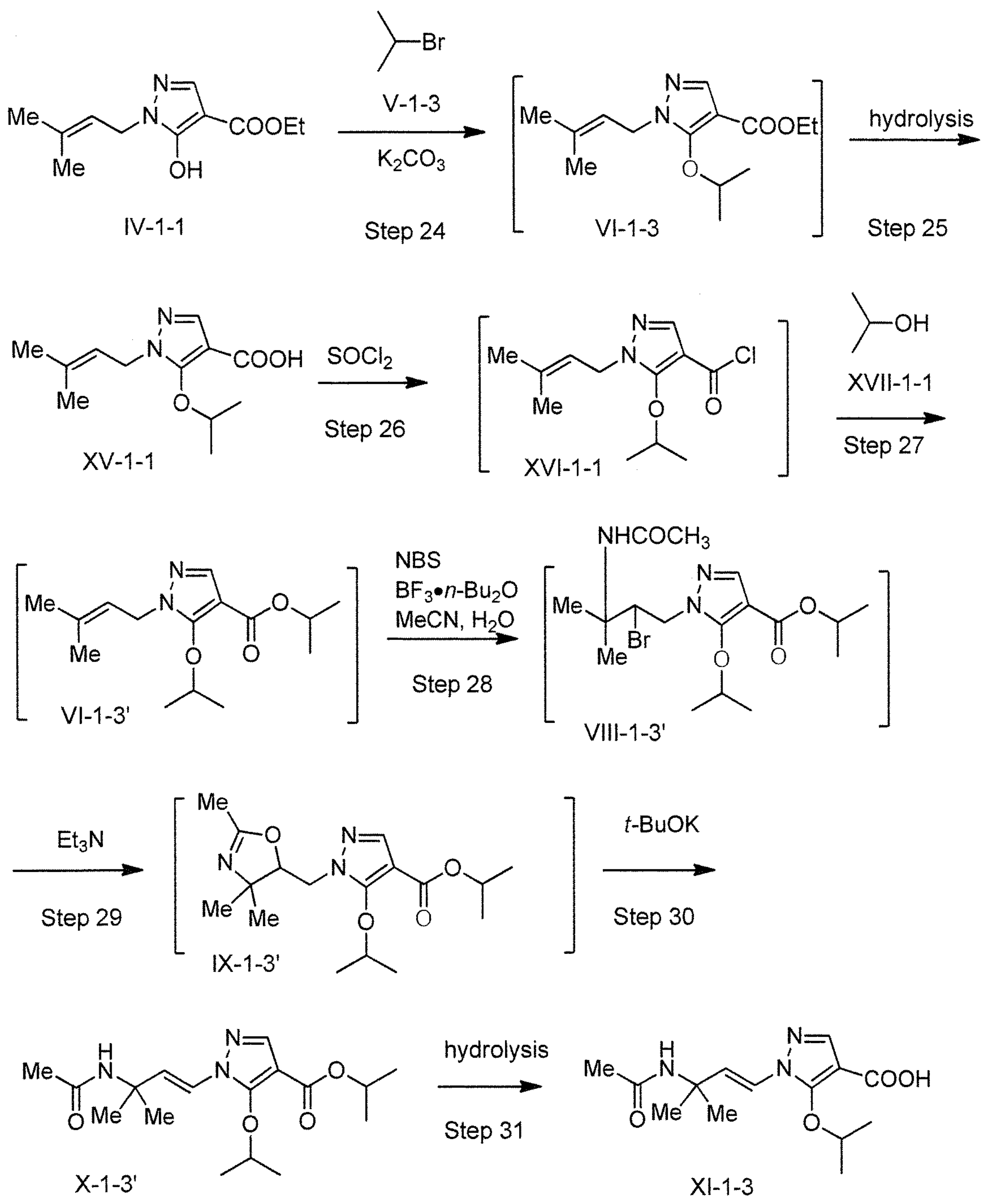


Step 24

A solution of Compound (IV-1-1) (130.0 g, 579.7 mmol), potassium carbonate (120.2 g, 869.5 mmol), N, N-dimethylacetoamide (377.0mL) was warmed to 80°C, and a solution of compound (V-1-3) (89.1 g, 724.6 mmol) in N, N-dimethylacetoamide (143.0mL) was added thereto. The reaction mixture was stirred for 3 hours. The reaction solution was cooled to room temperature, and water (1300mL) was added thereto. The reaction solution was extracted with toluene (650mL). The extracted solution was washed two times with water (130mL). The extracted solution was concentrated under reduced pressure to obtain the concentrated solution containing compound (VI-1-3).

Step 25

To the reaction solution obtained in Step 24 were added N, N-dimethylacetoamide (780.0 mL) and 20%-sodium hydroxide aqueous solution (579.7g), and the reaction mixture was warmed to 65°C. The reaction mixture was stirred

for 3 hours. The reaction solution was cooled to room temperature, and washed with toluene (780mL). To the extracted solution was added water (520mL), then washed with toluene (780mL) again. The extracted solution was adjusted to pH=2.0 with 20%-hydrochloric acid, followed by crystallizing at 0°C for 1 hour. The precipitated solid was filtered, and dried to obtain compound (XV-1-1) (129.4 g, 93.7%).
Melting point: 89.7~89.8°C
$^1$ H NMR (d6-DMSO); δ(ppm) 0.97 (d, J=6.0Hz, 6H), 1.39 (d, J=0.9Hz, 3H), 1.4 4 (d, J=1.2Hz, 3H), 4.22 (d, J=6.9Hz, 2H), 4.77 (sep, J=6.0Hz, 1H), 4.94 (m, 1 H), 7.37 (s, 1H), 11.89 (brs, 1H)
Powder X-ray Diffraction 2θ (°): 12.0, 20.3
Result of powder X-ray diffraction of Compound (XV-1-1) is shown in Fig. 6.

Step 26

A solution of compound (XV-1-1) (120.0 g, 503.6 mmol) in N, N-dimethylacetoamide (612mL) was cooled to 0°C, and thionyl chloride (65.2 g, 548.0mmol) was added thereto. The reaction mixture was stirred for 1 hour.

Step 27

To the reaction solution containing the resulting compound (XVI-1-1) was added compound (XVII-1-1) (151.3g, 2517.5mmol). The reaction mixture was stirred at room temperature for 1.5 hours, and then stirred at 80°C for 2 hours. The reaction solution was cooled to 0°C, and was added to 8%-sodium hydrogen carbonate solution (1305.9 g) and toluene (1200mL) under 15°C. After the aqueous layer was removed, the extracted solution was washed two times with water (1200mL). The extracted solution was concentrated under reduced pressure to obtain the concentrated solution containing compound (VI-1-3').

Step 28

A solution of N-bromosuccinimide (49.3 g, 277.2 mmol), boron trifluoride n-butyl ether complex (174.7 g, 822.0 mmol) and acetonitrile (210mL) was cooled to -20°C, and a mixed solution of the concentrated solution (222.2 g, equivalent to 252.2 mmol of compound (XV-1-1)) obtained in Step 27, water (4.56 g, 252.2mmol) and acetonitrile (270mL) was added dropwise thereto. The reaction was performed for 0.5 hours.
Compound (VIII-1-3'):
$^1$ H NMR ($CDCl_3$); δ(ppm) 1.31 (d, J=6.3Hz, 6H), 1.36 (d, J=5.9Hz, 3H), 1.38 ( d, J=5.9Hz, 3H), 1.56 (s, 3H), 1.57 (s, 3H), 1.91 (s, 3H), 4.34 (dd, J=8.6Hz, 14 .5Hz, 1H), 4.40 (dd, J=5.3Hz, 14.5Hz, 1H), 5.14 (qq, J=6.3Hz, 6.3Hz, 1H), 5.15 (dd, J=5.3Hz, 8.6Hz, 1H), 5.27 (qq, J=5.9Hz, 5.9Hz, 1H), 5.46 (brs, 1H), 7.78 (s, 1H)

Step 29

To the reaction solution containing the resulting compound (VIII-1-3') was added triethylamine (127.5 g, 1260.0 mmol), and the reaction mixture was stirred at 60°C for 3 hours. The reaction solution was cooled to room temperature, and toluene (240mL) was added thereto. The reaction solution was stirred for 1 hour, and the precipitate was filtered. The residue was washed with toluene (300mL). The filtrate was cooled to 5°C, and extracted with 18%-sodium hydroxide aqueous solution (448.0g). The extracted solution was washed two times respectively with 5%-sodium hydroxide aqueous solution (201.6g) and water (360mL). The extracted solution was concentrated under reduced pressure to obtain the concentrated solution (435.2 g) containing compound (IX-1-3').

Step 30

To the concentrated solution (114.4 g, equivalent to 120.0 mmol of compound (XV-1-1)) obtained in Step 29 was added N, N-dimethylacetoamide (85.8mL), and the reaction mixture was cooled to -20°C. A solution of tert-butoxy potassium (17.5 g, 156.0 mmol) in N, N-dimethylacetoamide (157mL) was added dropwise thereto, and the reaction mixture was stirred for 0.5 hours. To the reaction solution was added water (42.9mL) and toluene (286mL), and the reaction mixture was warmed to room temperature. The organic layer was washed with water (100mL), and the aqueous layer was back extracted with toluene (286mL). The organic layer was combined, and washed three times with water (57.2mL). The extracted solution was concentrated to 143g under reduced pressure. To the concentrated solution was heptane (428.9mL), followed by crystallizing at 0°C for 1 hour. The precipitated solid was filtered, and dried to obtain compound (X-1-3') (28.98 g, 71.6%).
Melting point: 120.0~120.1°C.
$^1$ H NMR (d6-DMSO); δ(ppm) 1.26 (d, J=6.3Hz, 6H), 1.28 (d, J=6.0Hz, 3H), 1.7 9 (s, 3H), 4.87 (sep, J=6.0Hz, 1H), 5.03

(sep, J=6.3Hz, 1H), 6.40 (d, J=14.1Hz, 1H), 6.80 (d, J=14.1Hz, 1H), 7.77 (brs, 1H), 7.82 (s, 1H)
Powder X-ray Diffraction 2θ (°): 12.7, 13.9, 15.2, 15.5, 17.2, 18.2, 18.6, 19.9, 2 0.2
Result of powder X-ray diffraction of Compound (X-1-3') is shown in Fig. 7.

Step 31

To a solution of compound (X-1-3') (1.012 g, 3mmol) in 2-propanol (5mL) was added 5%-sodium hydroxide aqueous solution (7.2g). The reaction mixture was warmed to 60°C, and stirred for 8 hours. The reaction solution was cooled to room temperature, and washed two times with toluene (5mL). The aqueous layer was adjusted to pH=2.0 with 12%-hydrochloric acid, followed by crystallizing at 0°C for 1 hour. The precipitated solid was filtered, and dried to obtain compound (XI-1-3) (0.59 g, 66.6%).
Melting point: 183.9~184.4°C
$^1$ H NMR (d6-DMSO); δ(ppm) 1.28 (d, J=6.2Hz, 3H), 1.28 (d, J=6.2Hz, 3H), 1.4 1(s, 3H), 1.41 (s, 3H), 1.80 (s, 3H), 4.98 (q, J=6.2Hz, 1H), 6.39 (d, J=14.4Hz, 1H), 6.78 (d, J=14.3Hz, 1H), 7.78 (brs, 1H), 7.81 (brs, 1H), 12.43 (brs, 1H) Type I crystal:
Powder X-ray Diffraction 2θ (°): 8.7, 11.3, 14.3, 15.0, 17.5, 19.4, 21.1, 22.7, 24. 3, 24.8, 26.0
Result of powder X-ray diffraction of type I crystal of Compound (XI-1-3) is shown in Fig. 4.
It has been also found that two kinds of different crystal forms from the above type I crystal exist as crystals of compound (XI-1-3) from the result of measurement of powder X-ray diffraction. Two kinds of the crystal forms are hereafter called respectively type II and type III. Type II and type III crystals are discerned with a characteristic peak obtained by powder X-ray diffraction, an absorption band of an infrared absorption spectrum or the like. Results of powder X-ray diffraction of each crystal forms are shown below.
Type II crystal:
Powder X-ray Diffraction 2θ (°): 12.6, 13.7, 15.3, 17.9, 20.1, 20.6, 21.2, 23.5, 2 5.4, 31.0
Result of powder X-ray diffraction of type II crystal of Compound (XI-1-3) is shown in Fig. 9.
Type III crystal:
Powder X-ray Diffraction 2θ (°): 8.7, 9.4, 11.3, 14.3, 16.3, 18.9, 20.2, 23.1, 27.7 , 30.5
Result of powder X-ray diffraction of type III crystal of Compou nd (XI-1-3) is shown in Fig. 10.

[Example7]

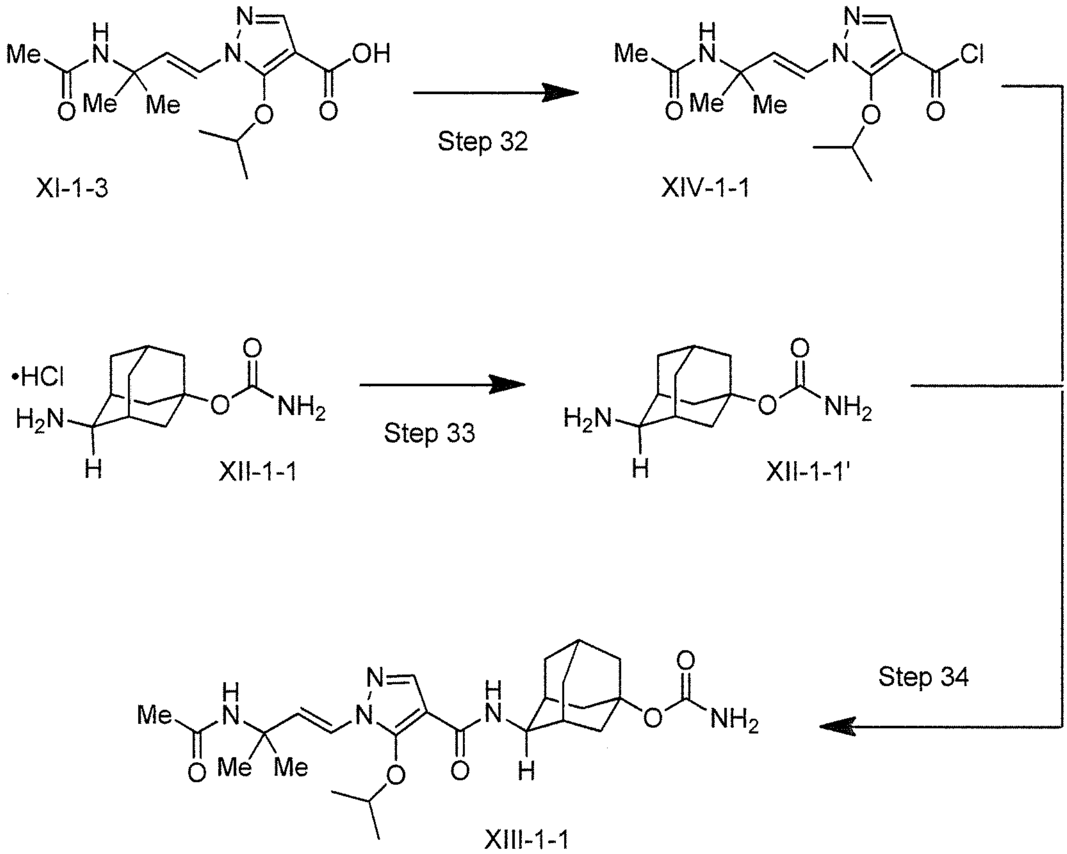


XI-1-3 XIV-1-1 XII-1-1 XII-1-1' XIII-1-1

Step 32

Butenylisopropoxypyrazole carboxylic acid (XI-1-3) (24.6 g, 83.3 mmol) was dissolved in N-methylpyrrolidone (94.6 mL) at 0°C, and thionyl chloride (10.9 g, 91.6 mmol) was added dropwise thereto. The reaction mixture was stirred for 0.5 hours to obtain the reaction solution containing butenyl isopropoxy pyrazole carbonyl chloride (XIV-1-1).

Step 33

Aminoadamantyl carbamate hydrochloride (XII-1-1) was synthesized according to a method described in WO2012/020724.

To the compound (XII-1-1) (22.6 g, 91.6 mmol) was added tetrahydrofuran (246mL), and the mixture was cooled to 0°C. 18%-Sodium hydroxide aqueous solution (101.8 g) was added thereto to obtain the slurry containing aminoadamantyl carbamate (XII-1-1').

Step 34

To the slurry obtained in Step 33 was added dropwise the reaction mixture obtained in Step 32 at 0°C over 2.5 hours. Water (221 mL) and 8% hydrochloric acid (40.0g) were added thereto, and the reaction mixture was warmed to 25°C. The reaction mixture was adjusted to pH=6.5 with 8% hydrochloric acid, and concentrated to 520 mL under reduced pressure under 50°C. To the obtained slurry was water (221mL), followed by crystallizing at 0°C for 1 hour. The precipitated solid was filtered, and dissolved in methanol (135mL) and ethyl acetate (135mL) at 55°C. Methanol (20mL) and ethyl acetate (172mL) were added thereto, the mixture was concentrated to 150mL under 50°C. Concentrated solution was cooled to 30°C. After checking that compound (XIII-1-1) had precipitated, the concentrated solution was stirred for 1 hour. To the obtained slurry was added ethyl acetate (315 mL), and the mixture was concentrated to 180 mL under 50°C. The obtained slurry was crystallized at -10°C for 1 hour. The precipitated solid was filtered, and dried to obtain crystals (type I crystals) of non-solvate of compound (XIII-1-1) (36.6 g, 90.0%).
Crystal (type I crystal) of non-solvate of compound (XIII-1-1):
Melting point: 196.9-203.5°C
$^1$H NMR (d6-DMSO); δ(ppm) 1.24 (d, J=6.3Hz, 6H), 1.34-1.48 (m, 8H), 1.79 (s, 3H), 1.87-1.99 (m, 2H), 2.00-2.14 (m,

9H), 3.92-3.98 (m, 1H), 4.88 (sep, J=6.3Hz, 1H), 6.19 (brs, 2H), 6.35 (d, J=14.4Hz, 1H), 6.74 (d, J=14.1Hz, 1H), 7.35 (d, J=6.6Hz, 1H), 7.76 (s, 1H), 8.00 (s, 1H)

Powder X-ray Diffraction 2θ (°): 8.2, 10.9, 12.3, 16.9, 19.5, 20.8, 24.6, 29.1

Result of powder X-ray diffraction of Crystal (type I crystal) of non-solvate of compound (XIII-1-1) is shown in Fig. 8.

When Step 34 was performed according to the same method as described above, a different crystal from the above type I crystal was obtained. The obtained crystal was a methanol solvate of compound (XIII-1-1).

Results of NMR analysis and powder X-ray diffraction of the methanol solvate are shown below. methanol solvate of compound (XIII-1-1):

$^1$H NMR (d6-DMSO); δ(ppm) 1.24 (d, J=6.3Hz, 6H), 1.34-1.48 (m, 8H), 1.79 (s, 3H), 1.87-1.99 (m, 2H), 2.00-2.14 (m, 9H), 3.16 (s, 3H), 3.92-3.98 (m, 1H), 4.8 8 (sep, J=6.3Hz, 1H), 6.19 (brs, 2H), 6.35 (d, J=14.4Hz, 1H), 6.74 (d, J=14.1Hz , 1H), 7.35 (d, J=6.6Hz, 1H), 7.76 (s, 1H), 8.00 (s, 1H)

Powder X-ray Diffraction 2θ (°): 7.4, 9.4, 11.9, 14.4, 18.5, 19.4, 15.1, 25.4, 29.5

Result of powder X-ray diffraction of methanol solvate of compound (XIII-1-1) is shown in Fig. 11.

A methanol solvate of compound (XIII-1-1) was obtained once, then even if Step 34 was performed according to the same method as described above, a methanol solvate of compound (XIII-1-1) was precipitated and Crystal (type I crystal) of non-solvate of compound (XIII-1-1) was not precipitated.

Moreover, even if the crude crystals of compound (XIII-1-1) were crystallized by the solvent containing methanol, it became clear that they could transform into methanol solvate.

However, since a methanol solvate of compound (XIII-1-1) contains methanol more than predetermined threshold of an ICH (International Conference on Harmonisation of Technical Requirements for Registration of Pharmaceuticals for Human Use) guideline, it is not desirable as a pharmaceutical agent.

The present inventors found out that Crystal (type I crystal) of non-solvate of compound (XIII-1-1) could be selectively obtained by crystallizing the crude crystals of compound (XIII-1-1) with a solvent that is substantially free of methanol.

"A solvent that is substantially free of methanol" means a solvent which does not contain methanol more than 10% (V/V). A solvent which does not contain methanol more than 5% (V/V) is preferable. A solvent which does not contain methanol more than 3% (V/V) is more preferable.

Example includes ethyl acetate which may contain methanol less than 10% (V/V);

ethyl acetate which may contain methanol less than 5% (V/V);
ethyl acetate which may contain methanol less than 3% (V/V);
a mixed solvent of one or more solvent(s) selected from acetone, 1-propanol, 2-propanol, ethanol, acetonitrile, dimethylsulfoxide, water and N, N-dimethylacetoamide and one or more solvent(s) selected from water and toluene;
a mixed solvent of acetone and water;
a mixed solvent of 1-propanol and water;
a mixed solvent of 2-propanol and water;
a mixed solvent of 2-propanol and toluene;
a mixed solvent of ethanol and water;
a mixed solvent of acetonitrile and water;
a mixed solvent of dimethylsulfoxide and water;
a mixed solvent of N, N-dimethylacetoamide and water, or the like.

[Example8]

Compound (XIII-1-1) (4.95 g, 10.16 mmol) was dissolved in 1-propanol (11.7 mL) and water (3.9 mL) at 55°C. The reaction mixture was cooled to 40°C, and type I seed crystal was added thereto. The reaction mixture was stirred at 25°C for 1 hour. To the obtained slurry was water (40.5 mL), followed by crystallizing at 0°C for 1 hour. The precipitated solid was filtered, and dried to obtain compound (XIII-1-1) (4.77 g, 96.4%). The obtained crystal was type I crystal from result of powder X-ray diffraction.

[Example9]

Compound (XIII-1-1) (9.91 g, 20.32 mmol) was dissolved in acetone (62.4mL) and water (15.6mL) at 55°C. The reaction mixture was cooled to 40°C, and type I seed crystal was added thereto. The reaction mixture was stirred at 25°C for 1 hour. The obtained slurry was concentrated to 36 mL under reduced pressure, and water (42.0 mL) was added thereto, followed by crystallizing at 0°C for 1 hour. The precipitated solid was filtered, and dried to obtain compound (XIII-1-1) (9.60 g, 96.9%). The obtained crystal was type I crystal from result of powder X-ray diffraction.

Type I seed crystal can be obtained by using acetone and water instead of methanol and ethyl acetate in the method of Step 34.

The present inventors found out that a crystal of a non-solvate of a compound (XIII-1-1) or a crystal of a non-solvate of a salt of the compound could be synthesized by transforming a crystal of a methanol solvate of a compound (XIII-1-1) or a crystal of a methanol solvate of a salt of the compound with a solvent which does not contain methanol more than 30% (V/V).

"Transformation" means that a crystal of a methanol solvate of a compound (XIII-1-1) or a crystal of a methanol solvate of a salt of the compound is transformed into a crystal of a non-solvate of a compound (XIII-1-1) or a crystal of a non-solvate of a salt of the compound by stirring a crystal of a methanol solvate of a compound (XIII-1-1) or a crystal of a methanol solvate of a salt of the compound in a suspension state.

"A solvent which does not contain methanol more than 30% (V/V)" means a solvent which may contain methanol less than 30% (V/V). A solvent which does not contain methanol more than 20% (V/V) is preferable. A solvent which does not contain methanol more than 10% (V/V) is more preferable. A solvent which does not contain methanol more than 5% (V/V) is particularly preferable.

"A solvent which does not contain methanol more than 30% (V/V)" means a solvent such as acetone, 1-propanol, 2-propanol, ethanol, acetonitrile, dimethylsulfoxide, N, N-dimethylacetoamide, N-methylpyrrolidone, dimethylformamide and tetrahydrofuran, water, toluene or a mixed solvent thereof which may contain methanol less than 30% (V/V).

Particularly, "a solvent which does not contain methanol more than 30% (V/V)" means a mixed solvent of one or more solvent(s) selected from acetone, 1-propanol, 2-propanol, ethanol, acetonitrile, dimethylsulfoxide, N, N-dimethylacetoamide, water, N-methylpyrrolidone, dimethylformamide and tetrahydrofuran and one or more solvent(s) selected from water and toluene which may contain methanol less than 30% (V/V). Particularly, "a solvent which may contain methanol less than 20% (V/V)", "a solvent which may contain methanol less than 10% (V/V)", "a solvent which may contain methanol less than 5% (V/V)" or the like is preferable.

[Example 10]

To 20% aqueous methanol solution (mixture of methanol (0.20mL) and water (0.80mL)), 50% aqueous methanol solution (mixture of methanol (0.50mL) and water (0.50mL)), 80% aqueous methanol solution (mixture of methanol (0.80mL) and water (0.20mL)) or 100% methanol (methanol (1.00mL)) was respectively added methanol solvate (0.30g, 0.58mmol) of compound (XIII-1-1). Their reaction mixtures were respectively stirred in a suspension state at 25°C for 6 hours.

The obtained solids were filtered, and Powder X-ray Diffraction was measured without drying the solids.

The solid obtained from 20% aqueous methanol solution was transformed into type I crystal of compound (XIII-1-1). On the other hand, each solid obtained from 50% aqueous methanol solution, 80% aqueous methanol solution and 100% methanol was still methanol solvate of compound (XIII-1-1).

[Example 11]

To 30% aqueous methanol solution (mixture of methanol (0.30mL) and water (0.70mL)), 40% aqueous methanol solution (mixture of methanol (0.40mL) and water (0.60mL)), 50% aqueous methanol solution (mixture of methanol (0.50mL) and water (0.50mL)) or 80% aqueous methanol solution (mixture of methanol (0.80mL) and water (0.20mL)) was respectively added type I crystal (0.15g, 0.31mmol) of compound (XIII-1-1).

To 30% aqueous methanol solution (mixture of methanol (0.30mL) and water (0.70mL)), 40% aqueous methanol solution (mixture of methanol (0.40mL) and water (0.60mL)), 50% aqueous methanol solution (mixture of methanol (0.50mL) and water (0.50mL)) or 80% aqueous methanol solution (mixture of methanol (0.80mL) and water (0.20mL)) was respectively added methanol solvate (0.15g 0.29mmol) of compound (XIII-1-1).

The suspensions were stirred at 25°C for 5 hours. The obtained solids were filtered, and Powder X-ray Diffraction was measured without drying the solids. Each of the obtained solid was methanol solvate of compound (XIII-1-1).

From these results, the present inventors found out that methanol solvate of compound (XIII-1-1) could be obtained by stirring in aqueous methanol solution whose methanol concentration is higher than 30% aqueous methanol solution. Furthermore, the present inventors found out that type I crystal of compound (XIII-1-1) could be obtained by stirring in 20% aqueous methanol solution.

[Example 12]

To 25% aqueous acetone solution (mixture of acetone (0.25mL) and water (0.75mL)), 38% aqueous acetone solution (mixture of acetone (0.38mL) and water (0.62mL)), 52% aqueous acetone solution (mixture of acetone (0.52mL) and water (0.48mL)) or 80% aqueous acetone solution (mixture of acetone (0.80mL) and water (0.20mL)) was respectively

added methanol solvate (0.50g, 0.96mmol) of compound (XIII-1-1). Their reaction mixtures were respectively stirred in a suspension state at 25°C for 5 hours. The obtained solids were filtered, and Powder X-ray Diffraction was measured without drying the solids. Each of the obtained solid was type I crystal of compound (XIII-1-1).

[Example 13]

To 80% aqueous acetone solution (mixture of acetone (0.80mL) and water (0.20mL)) was added methanol solvate (0.50g, 0.96mmol) of compound (XIII-1-1). The reaction mixture was stirred in a suspension state at 40°C for 5 hours. The obtained solid was filtered, and Powder X-ray Diffraction was measured without drying the solid. The obtained solid was type I crystal of compound (XIII-1-1).

[Example 14]

To 80% aqueous acetone solution (mixture of acetone (0.80mL) and water (0.20mL)) was added methanol solvate (0.50g, 0.96mmol) of compound (XIII-1-1). The reaction mixture was stirred in a suspension state at 55°C for 5 hours. The obtained solid was filtered, and Powder X-ray Diffraction was measured without drying the solid. The obtained solid was type I crystal of compound (XIII-1-1).

From these results, the present inventors found out that type I crystal of compound (XIII-1-1) could be obtained from methanol solvate of compound (XIII-1-1) by stirring in the water containing 25 to 80% of acetone.

[Example 15]

To 20% aqueous 2-propanol solution (mixture of 2-propanol (0.20mL) and water (0.80mL)), 50% aqueous 2-propanol solution (mixture of 2-propanol (0.50mL) and water (0.50mL)) or 75% aqueous 2-propanol solution (mixture of 2-propanol (0.75mL) and water (0.25mL)) was respectively added methanol solvate (0.30g 0.58mmol) of compound (XIII-1-1). Their reaction mixtures were respectively stirred in a suspension state at 25°C for 96 hours. The obtained solids were filtered, and Powder X-ray Diffraction was measured without drying the solids. Each of the obtained solid was type I crystal of compound (XIII-1-1).

From these results, the present inventors found out that type I crystal of compound (XIII-1-1) could be obtained from methanol solvate of compound (XIII-1-1) by stirring in the water containing 20 to 75% of 2-propanol.

[Example 16]

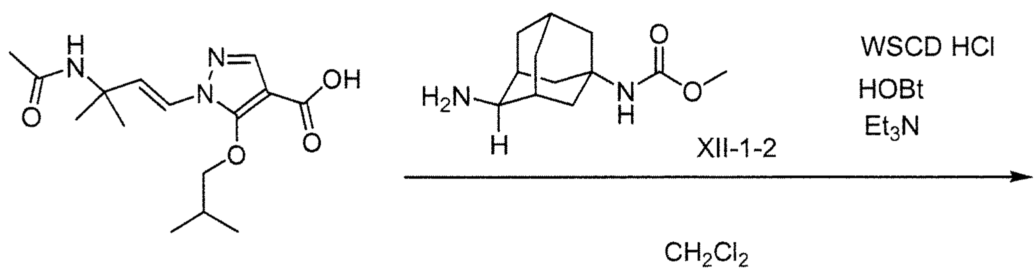


XI-1-4

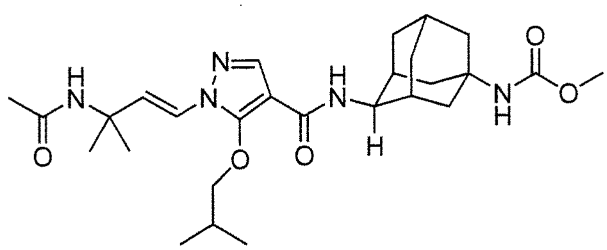

XIII-1-2

To a solution of Compound (XI-1-4) (80mg) in dichloromethane (2.0 ml) were added amine (XII-1-2) (64mg), 1-(3-dimethylaminopropyl)-3-ethylcarbodiimide hydrochloride (65mg), 1-hydroxybenzotriazole (11mg) and triethylamine (54 μl), then the reaction mixture was stirred at room temperature overnight. After the completion of the reaction, to 0.5N aqueous hydrochloric acid solution was added the reaction mixture, then extracted with dichloromethane. The organic layer was washed respectively with saturated sodium bicarbonate solution and brine, then dried over sodium sulfate. The solvent was removed under reduced pressure. The obtained residue was purified by column chromatography to give Compound (XIII-1-2) (100 mg, 75.0%).
logk'=0.886

[Example 17]

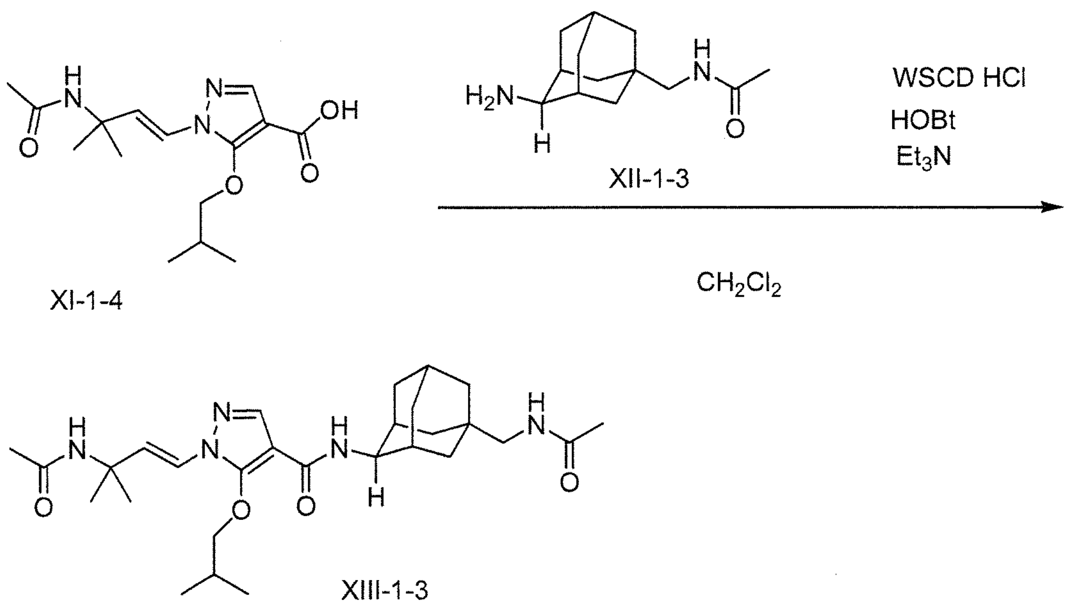

XI-1-4
XII-1-3
XIII-1-3

To a solution of Compound (XI-1-4) (80mg) in dichloromethane (3.2 ml) were added amine (XII-1-3) (74mg), 1-(3-dimethylaminopropyl)-3-ethylcarbodiimide hydrochloride (WSCD) (65mg), 1-hydroxybenzotriazole (HOBt) (11mg) and triethylamine (90 μl), then the reaction mixture was stirred at room temperature for 36 hours. After the completion of the reaction, to 0.5N aqueous hydrochloric acid solution was added the reaction mixture, then extracted with dichloromethane. The organic layer was washed respectively with saturated sodium bicarbonate solution and brine, then dried over sodium sulfate. The solvent was removed under reduced pressure. The obtained residue was purified by column chromatography to give Compound (XIII-1-3) (98 mg, 73.8%). logk'=0.838

A compound represented by the Formula (XII) can be synthesized as follows.

(Reference example 1)

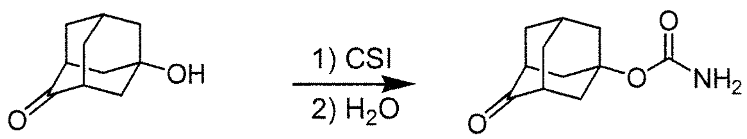

S-1
S-2

A solution of 5-hydroxy-2-adamantanone (S-1) (60.00 g, 360.97 mmol) in acetone (420 ml) was cooled to 0°C, and chlorosulfonyl isocyanate (CSI) (53.6 g, 378.72 mmol) was added thereto for 1 hour. The reaction mixture was stirred for 30 minutes. Then, the solution was kept at 5°C. To the solution was added water (60 ml) for 2 hours, and then the solution was heated to 15°C. The solution was stirred for another 7 hours. To the solution was added 7% ammonia

water to obtain pH 7.9. The solution was heated to 60°C, and then was separated. To the extraction solution in the upper layer was added water (240 ml), and the solution was concentrated. Crystallization was conducted for 1 hour at 25°C, and the precipitated crystal was filtered. The obtained crystal was dried to obtain compound (S-2) (64.43 g, 85.3%).
$^{1}$H NMR (d6-DMSO); δ (ppm) 1.82 (brd, J=13Hz, 2H), 1.96 (brd, J=13Hz, 2H), 2.24 (m, 1H), 2.24 (brs, 2H), 2.27-2.28 (m, 4H), 2.48 (brs, 2H), 6.30 (br, 2H)

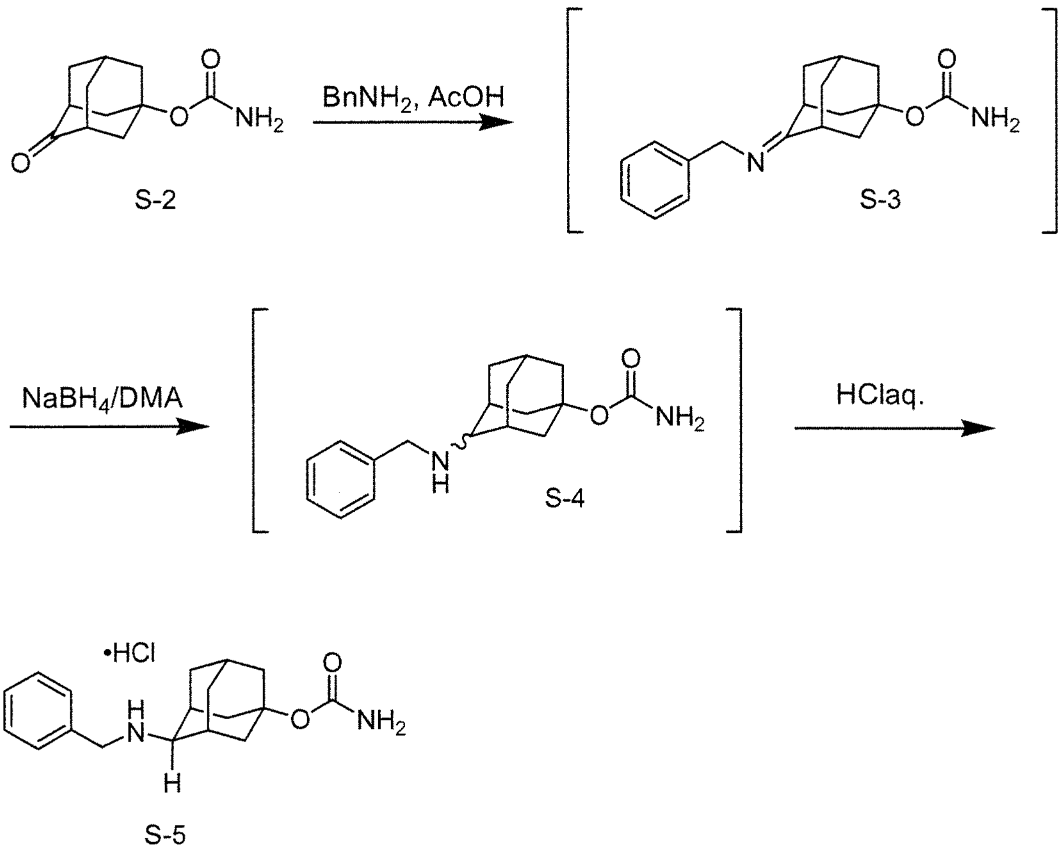


S-2 S-3 S-4 S-5

To a solution of Compound (S-2) (30.00g, 143.4 mmol) in dichloromethane (300 ml) were added benzylamine (15.36 g, 143.4 mmol) and acetic acid (8.61 g, 143.4 mmol), and the reaction mixture was cooled to 0°C.

To the reaction solution containing the resulting Compound (S-3) was added a solution of sodium borohydride (3.25 g, 83.7 mmol) in N,N-dimethylacetamide (45 ml) over 2 hours, and the reaction mixture was stirred for another 2 hours.

To the reaction solution containing the resulting Compound (S-4) was added hydrochloric acid (7%) to obtain pH 2, and the reaction mixture was heated to room temperature. To the reaction mixture were added water (150 ml) and 16% sodium hydroxide aqueous solution to obtain pH 8, and the reaction mixture was extracted. The extraction solution in the lower layer was cooled to 5°C, 4N-hydrochloric acid/ethyl acetate solution (35.85 ml, 143.4 mmol) was added thereto. Crystallization was conducted for 2 hours at 5°C. The precipitated crystal was filtered, and dried to obtain compound (S- 5) (27.48 g, 56.90%).

Compound (S-3): $^{1}$H NMR ($CDCl_3$); δ (ppm): 1.67 (brd, J=13Hz, 1H), 1.89 (m, 3H), 2.08 (brd, J=12Hz, 1H), 2.30 (m, 6H), 2.79 (m, 1H), 3.34 (m, 1H), 4.51 (br, 2H), 4.55 (d, J=3Hz, 2H), 7.24 (m, 2H), 7.30 (m, 3H)

Compound (S-5): $^{1}$H NMR (d6-DMSO); δ (ppm) 1.44 (brd, J=13Hz, 2H), 2.00 (m, 2H), 2.01 (brs, 2H), 2.07 (brd, J=13Hz, 2H), 2.09 (m, 1H), 2.15 (brd, J=13Hz, 2H), 2.40 (brs, 1H), 2.49 (brs, 1H), 3.15 (brs, 1H), 4.17 (brs, 2H), 6.23 (br, 2H), 7.42 (m, 3H), 7.67 (m, 2H), 9.42 (brs, 2H)

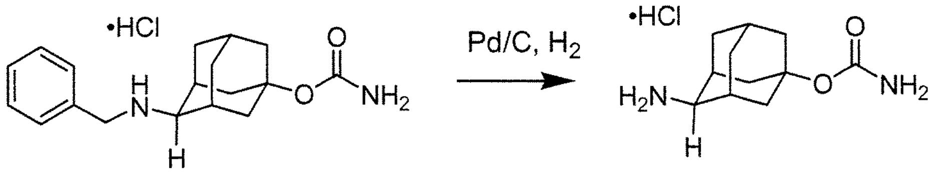


S-5 XII-1-1

A solution of compound (S-5) (20.00 g, 59.4 mmol) in methanol (200 ml) and 5% palladium carbon catalyst (M) (moisture 53.1%, 4.26 g) were heated to 30°C, and the reaction mixture was stirred for 5 hours under pressure of hydrogen of 0.2 MPa. The reaction solution was filtered to eliminate the palladium carbon catalyst, and then the filtrate was concentrated under reduced pressure. The concentrated solution was replaced with tetrahydrofuran, and was crystallized for 1 hour at room temperature. The precipitated crystal was filtered, and dried to obtain compound (XII-1-1) (12.78 g, 87.3%).

$^1$H NMR (d6-DMSO); δ (ppm) 1.45 (brd, J=13Hz, 2H), 1.98 (brd, J=13Hz, 2H), 2.04 (m, 1H), 2.05 (br, 1H), 2.07 (brd, J=11Hz, 2H), 2.12 (brd, J=11Hz, 2H), 2.20 (br, 2H), 3.29 (m, 1H), 6.23 (br, 2H), 8.32 (brs, 3H)

(Reference example 2)

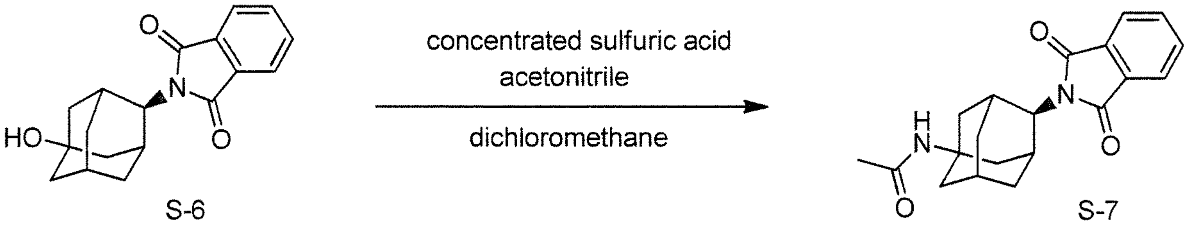


S-6 S-7

Compound (S-6) was synthesized according to a method described in W02007/114125.

To dichloromethane (6 ml) and acetonitrile (3 ml) was suspended Compound (S-6) (1.6g), then the reaction mixture was cooled to 0 °C. To the reaction mixture was added dropwise concentrated sulfuric acid (1.1 g), then the reaction mixture was stirred at 0 °C for 20 minutes. The reaction mixture was continuously stirred at room temperature for 14 hours. After the completion of the reaction, to cooled aqueous saturated sodium hydrogen carbonate solution was added the reaction mixture, then extracted with dichloromethane. The organic layer was washed with brine and dried over sodium sulfate. The solvent was removed under reduced pressure, then the obtained residue was purified by column chromatography to give Compound (S-7) (1.55 g, 85.1%).

NMR(d6-DMSO); 8(ppm)1.50-1.53 (m, 2H), 1.77 (s, 3H), 1.94-2.03 (m, 5H), 2.12-2.18 (m, 4H), 2.68 (br s, 2H), 4.19 (s, 1H), 7.39 (s, 1H), 7.82 (s, 4H).

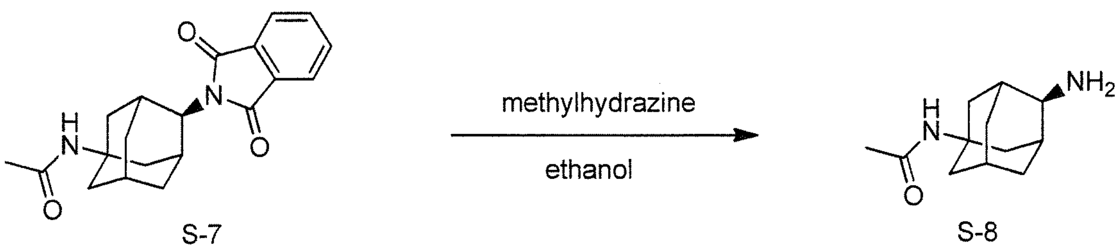


S-7 S-8

To a suspension of Compound (S-7) (1.55g) in ethanol (16ml) was added methylhydrazine (0.61ml), the reaction mixture was refluxed for 28 hours. After the completion of the reaction, the solvent was removed under reduced pressure. To the residue were added 2N aqueous hydrochloric acid solution and ethyl acetate, then the mixture was stirred. The insoluble residue was collected by filtration, then the filtrate was separated. The aqueous layer was washed with ethyl acetate, then concentrated under reduced pressure. The residue was alkalified with 5N aqueous sodium hydroxide and extracted with dichloromethane. The organic layer was washed with brine and dried over magnesium sulfate. The solvent was removed under reduced pressure to give amine (S-8). NMR(d6-DMSO); δ(ppm)1.24-1.28 (m, 2H), 1.69-1.99 (m,

14H), 2.86 (s, 1H), 7.25 (s, 1H)

(Reference example 3)

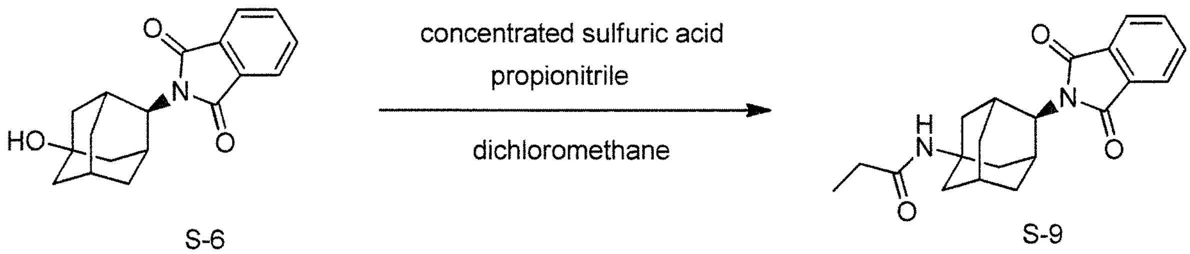


S-6 S-9

To dichloromethane (6 ml) and propionitrile (3 ml) was suspended Compound (S-6) (1.5g), then the reaction mixture was cooled to 0 °C. To the reaction suspension was added dropwise concentrated sulfuric acid (990mg), then the reaction mixture was stirred at room temperature for 18 hours. After the completion of the reaction, to cooled aqueous saturated sodium hydrogen carbonate solution was added the reaction mixture, then extracted with dichloromethane. The organic layer was washed with brine and dried over sodium sulfate. The solvent was removed under reduced pressure, then the obtained residue was purified by column chromatography to give Compound (S-9) (1.17 g, 65.8%).

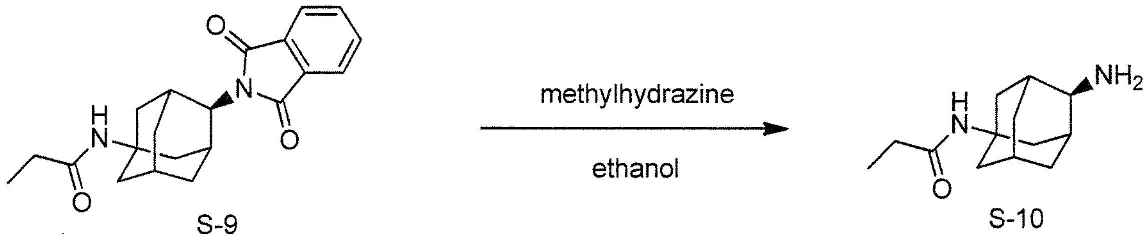


S-9 S-10

To a suspension of Compound (S-9) (1.17g) in ethanol (12ml) was added methylhydrazine (442μl), the reaction mixture was refluxed for 24 hours. After the completion of the reaction, the solvent was removed under reduced pressure. To the residue were added 2N aqueous hydrochloric acid solution and ethyl acetate, then the mixture was stirred. The insoluble residue was collected by filtration, then the filtrate was separated. The aqueous layer was washed with ethyl acetate, then concentrated under reduced pressure. The residue was alkalified with 2N aqueous sodium hydroxide and extracted with dichloromethane. The organic layer was washed with brine and dried over magnesium sulfate. The solvent was removed under reduced pressure to give amine (S-10) (660 mg, 89.4%). NMR(d6-DMSO); δ(ppm)0.93 (t, J = 7.6 Hz, 3H), 1.25-1.28 (m, 2H), 1.69 (br s, 2H), 1.87-2.02 (m, 11H), 2.86 (s, 1H), 7.16 (s, 1H)

(Reference example 4)

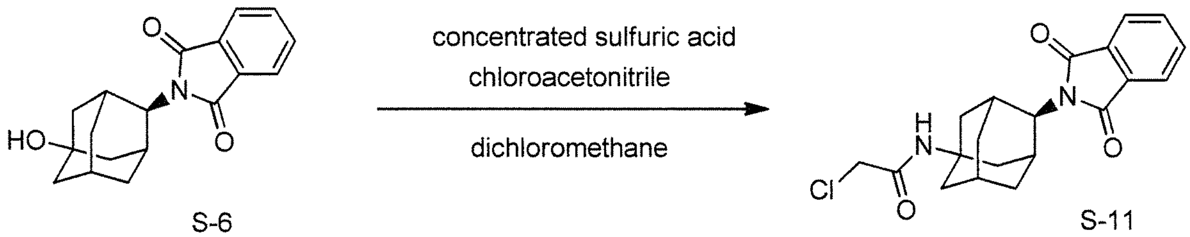


S-6 S-11

To dichloromethane (7 ml) and chloroacetonitrile (3.5ml) was suspended Compound (S-6) (2.0g), then the reaction mixture was cooled to 0 °C. To the reaction mixture was added dropwise concentrated sulfuric acid (990mg), then the reaction mixture was stirred at room temperature for 6.5 hours. After the completion of the reaction, to cooled

aqueous saturated sodium hydrogen carbonate solution was added the reaction mixture, then extracted with dichloromethane. The organic layer was washed with brine and dried over sodium sulfate. The solvent was removed under reduced pressure, then the obtained residue was purified by column chromatography to give Compound (S-11) (2.37 g, 94.5%).
NMR(d6-DMSO); δ(ppm)1.52-1.55 (m, 2H), 1.96-2.19 (m, 9H), 2.71 (brs, 2H), 3.98 (s, 2H), 4.20 (s, 1H), 7.77 (s, 1H), 7.82 (s, 4H)

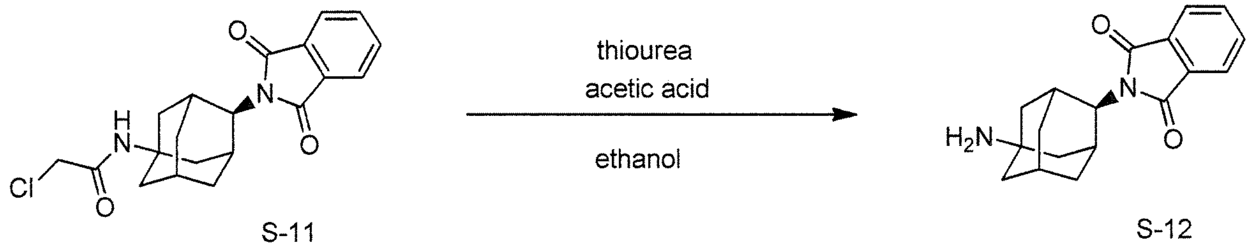


S-11 S-12

To a solution of Compound (S-11) (2.37g) in ethanol (13ml) were added acetic acid (2.6ml) and thiourea (581mg), the reaction mixture was refluxed for 14 hours. After the completion of the reaction, to the reaction mixture was added water. The insoluble residue was collected by filtration. The filtrate was alkalified with 5N aqueous sodium hydroxide and extracted with dichloromethane. The organic layer was washed with brine and dried over magnesium sulfate. The solvent was removed under reduced pressure to give Compound (S-12) (1.33g). The obtained product was used for the next reaction without further purification.

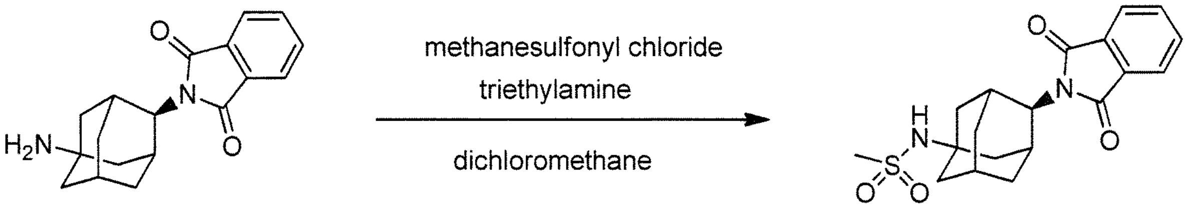


S-12 S-13

To a solution of Compound (S-12) (1.33g) in dichloromethane (15 ml) was added triethylamine (1.26ml), then the reaction mixture was cooled to 0 °C. To the reaction mixture was added methanesulfonyl chloride (418 μl), then the reaction mixture was stirred at room temperature for 1.5 hours. After the completion of the reaction, the organic layer was washed with 1N aqueous hydrochloric acid solution. The solvent was removed under reduced pressure, then the obtained residue was purified by column chromatography to give Compound (S-13) (907 mg, 53.7%).
NMR(d6-DMSO); δ(ppm)1.49-1.52 (m, 2H), 1.93-2.15 (m, 9H), 2.72 (brs, 2H), 3.34 (s, 3H), 4.16 (s, 1H), 6.93 (s, 1H), 7.82 (s, 4H)

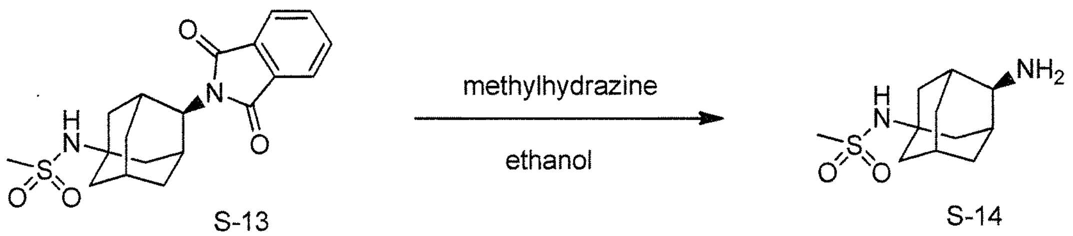


S-13 S-14

To a suspension of Compound (S-13) (907mg) in ethanol (20ml) was added methylhydrazine (322μl), the reaction mixture was refluxed overnight. After the completion of the reaction, the solvent was removed under reduced pressure. To the residue were added 2N aqueous hydrochloric acid solution and a mixed solution(1:1) of ethyl acetate and hexane, then the mixture was stirred. The insoluble residue was collected by filtration, then the filtrate was separated. The aqueous layer was washed with ether. The aqueous layer was alkalified with 5N aqueous sodium hydroxide and extracted with dichloromethane. The organic layer was washed with brine and dried over magnesium sulfate. The solvent was removed under reduced pressure to give amine (S-14) (321 mg, 54.2%). NMR(d6-DMSO); δ(ppm)1.23-1.26 (m, 2H), 1.71-1.99 (m, 11H), 2.84 (s, 1H), 2.92 (s, 3H), 6.76 (brs, 1H)

(Reference example 5)

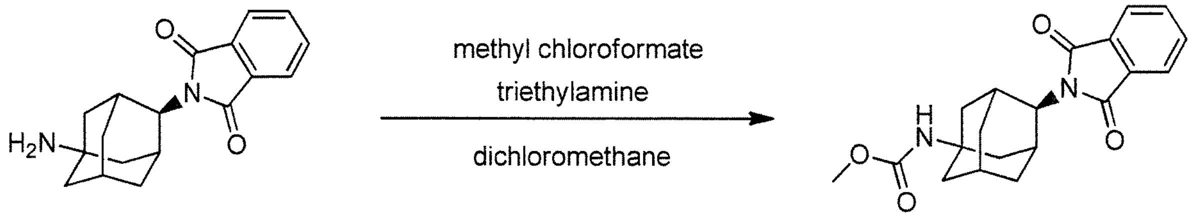


S-12 S-15

To a solution of Compound (S-12) (700mg) in dichloromethane (14 ml) was added triethylamine (0.99ml), then the reaction mixture was cooled to 0 °C. To the reaction mixture was added methyl chloroformate (272μl), then the reaction mixture was stirred at room temperature for 4 hours. After the completion of the reaction, to 0.5N aqueous hydrochloric acid solution was added the reaction mixture, then extracted with dichloromethane. The organic layer was washed with brine and dried over sodium sulfate. The solvent was removed under reduced pressure, then the obtained residue was purified by column chromatography to give Compound (S-15) (266 mg, 31.8%).
NMR(d6-DMSO); δ(ppm)1.49-1.52 (m, 2H), 1.89-2.16 (m, 9H), 2.68 (brs, 2H), 3.49 (s, 3H), 4.16 (s, 1H), 6.92 (s, 1H), 7.82 (s, 4H)

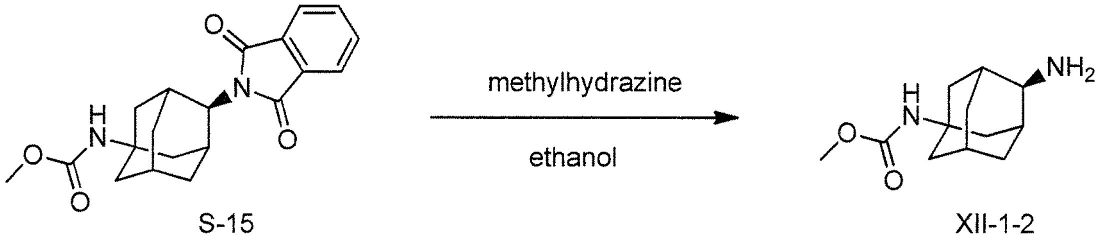


S-15 XII-1-2

To a suspension of Compound (S-15) (259mg) in ethanol (3ml) was added methylhydrazine (97μl), the reaction mixture was refluxed for 24 hours. After the completion of the reaction, the solvent was removed under reduced pressure. To the residue was added 2N aqueous hydrochloric acid solution. The insoluble residue was collected by filtration. The aqueous layer was washed with ethyl acetate. The aqueous layer was alkalified with 5N aqueous sodium hydroxide and extracted with dichloromethane. The organic layer was washed with brine and dried over magnesium sulfate. The solvent was removed under reduced pressure to give amine (XII-1-2) (135 mg, 82.4%).
NMR(d6-DMSO); δ(ppm)1.23-1.26 (m, 2H), 1.69-1.99 (m, 11H), 2.84 (s, 1H), 3.45 (s, 3H), 6.76 (s, 1H)

(Reference example 6)

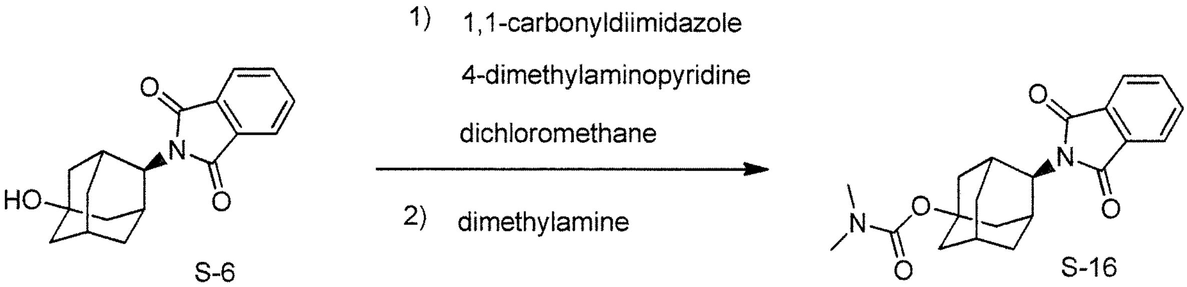


S-6 S-16

To a solution of Compound (S-6) (5.0g) in dichloromethane (50 ml) were added 1,1-carbonyldiimidazole (3.27g) and 4-dimethylaminopyridine (411mg), then the reaction mixture was stirred at room temperature for 14 hours. After confirming the disappearance of the starting material, to the reaction mixture was added dimethylamine (25.2 ml, 2M

tetrahydrofuran solution), then the reaction mixture was continuously stirred at room temperature for 10 hours. After the completion of the reaction, the reaction mixture was acidified with 2N aqueous hydrochloric acid solution and extracted with chloroform. The organic layer was washed with brine and dried over sodium sulfate. The solvent was removed under reduced pressure, then the obtained residue was purified by column chromatography to give Compound (S-16) (2.73 g, 44.1%).

NMR(d6-DMSO); δ(ppm) 1.48-1.57 (m, 2H), 2.060-2.29 (m, 9H), 2.72-2.86 (m, 8H), 4.18-4.22 (br, 1H), 7.82 (s, 4H)

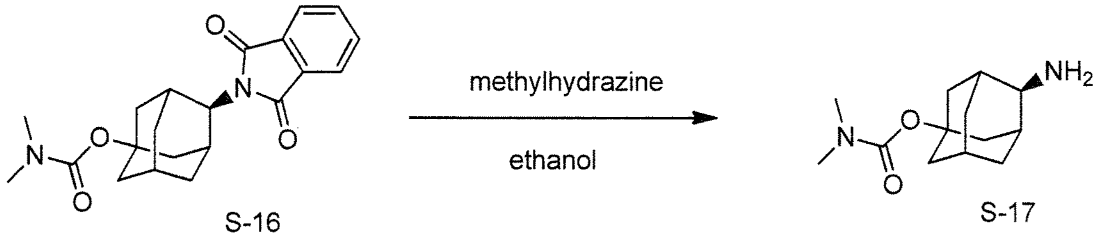


S-16 S-17

To a solution of Compound (S-16) (1.0g) in ethanol (10ml) was added methylhydrazine (361μl), the reaction mixture was refluxed for 20 hours. After the completion of the reaction, the solvent was removed under reduced pressure. To the residue were added 2N aqueous hydrochloric acid solution and ethyl acetate. The mixture was stirred and separated. The aqueous layer was washed with ethyl acetate, then concentrated under reduced pressure. The residue was alkalified with aqueous sodium carbonate solution. The obtained solid was collected by filtration and washed with water, then dried to give amine (S-17) (500 mg, 77.3%).

NMR(CDCl3); δ(ppm) 1.42-1.51 (m, 2H), 1.88-2.19 (m, 11H), 2.85 (s, 6H), 3.05-3.10 (br, 1H)

(Reference example 7)

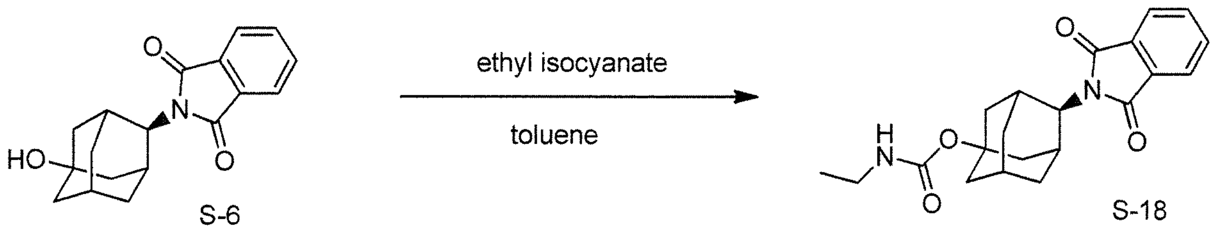


S-6 S-18

To a solution of Compound (S-6) (2.0g) in toluene (20ml) was added ethyl isocyanate (2.7ml), the reaction mixture was refluxed for 8.5 hours. After the completion of the reaction, the solvent was removed under reduced pressure. The obtained solid was washed with diisopropyl ether, then dried to give Compound (S-18) (2.15 g, 86.7%).

NMR(d6-DMSO); δ(ppm) 0.99 (t, J=7.2Hz, 3H), 1.47-1.57 (m, 2H), 2.04-2.32 (m, 9H), 2.76-2.84 (br, 2H), 2.88-3.00 (m, 2H), 4.17-4.22 (br, 1H), 6.84-6.91 (m, 1H), 7.83 (s, 4H)

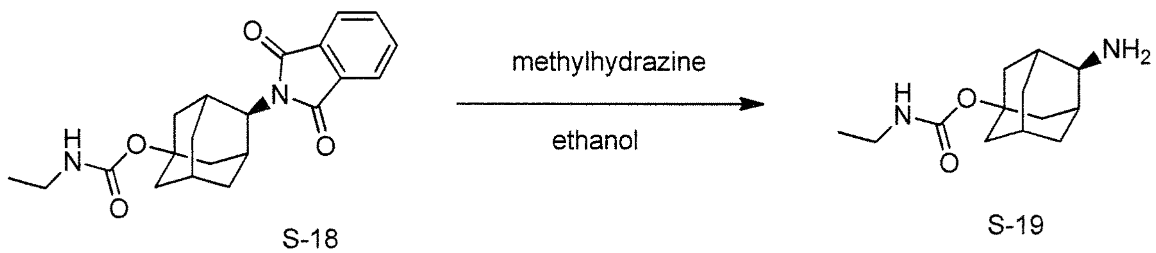


S-18 S-19

To a solution of Compound (S-18) (1.0g) in ethanol (10ml) was added methylhydrazine (361μ1), the reaction mixture was refluxed for 25 hours. After the completion of the reaction, the solvent was removed under reduced pressure. To the residue were added 2N aqueous hydrochloric acid solution and ethyl acetate. The mixture was stirred and separated. The aqueous layer was washed with ethyl acetate, then concentrated under reduced pressure. The residue was alkalified with aqueous sodium carbonate solution and extracted with chloroform. The organic layer was washed with brine and dried over sodium sulfate. The solvent was removed under reduced pressure. The obtained residue was

washed with diisopropyl ether, then dried to give amine (S-19) (475 mg, 73.4%).
NMR($CDCl_3$); δ(ppm) 1.11 (t, J=7.2Hz, 3H), 1.38-1.51 (m, 2H), 1.87-2.18 (m, 11H), 3.03-3.21 (m, 3H), 4.42-4.59 (br, 1H)

(Reference example 8)

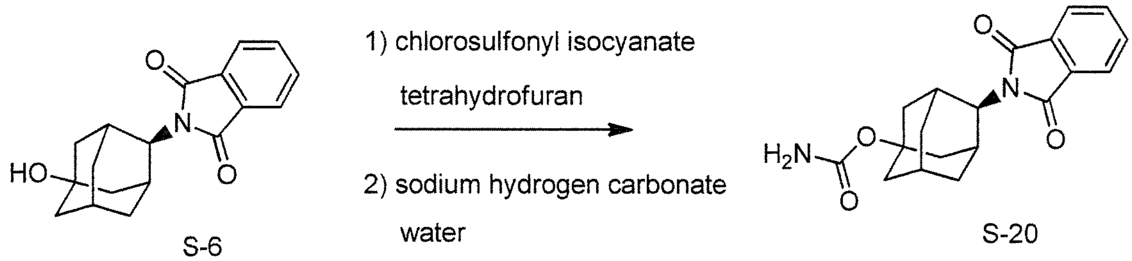


S-6 S-20

A solution of Compound (S-6) (2.5g) in tetrahydrofuran (50ml) was cooled to - 30 °C. To the solution was added chlorosulfonyl isocyanate (1.5ml), then the reaction mixture was stirred at - 30 °C for one hour. To the reaction mixture were added sodium hydrogen carbonate (3.5g) and water (1 ml), then the reaction mixture was stirred at room temperature for 16 hours. The reaction mixture was extracted with chloroform. The organic layer was washed with brine and dried over sodium sulfate. The solvent was removed under reduced pressure. The obtained residue was washed with diisopropyl ether, then dried to give Compound (S-20) (2.73 g, 95.5%).
NMR(d6-DMSO); δ(ppm) 1.46-1.58 (m, 2H), 2.02-2.30 (m, 9H), 2.76-2.84 (br, 2H), 4.16-4.22 (br, 1H), 6.10-6.35 (m, 1H), 7.82 (s, 4H)

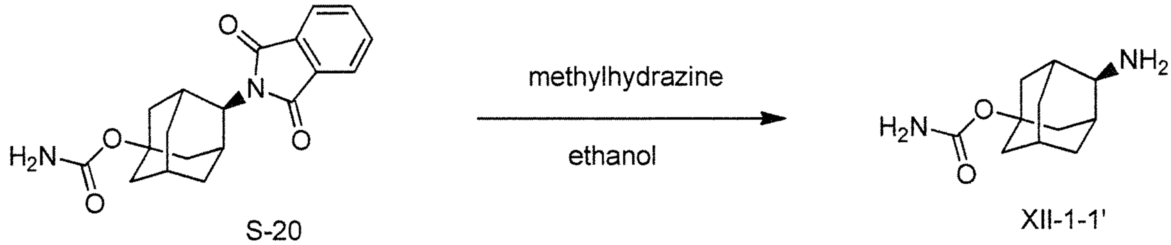


S-20 XII-1-1'

To a solution of Compound (S-20) (1.0g) in ethanol (10ml) was added methylhydrazine (391μl), the reaction mixture was refluxed for 21 hours. After the completion of the reaction, the solvent was removed under reduced pressure. To the residue were added 2N aqueous hydrochloric acid solution and ethyl acetate. The mixture was stirred and separated. The aqueous layer was washed with ethyl acetate, then concentrated under reduced pressure. The residue was alkalified with aqueous sodium carbonate solution. The obtained solid was collected by filtration and washed with water, then dried to give amine (XII-1-1') (468 mg, 75.7%).
NMR(d6-DMSO); δ(ppm) 1.40-1.51 (m, 2H), 1.91-2.22 (m, 11H), 3.28-3.34 (br, 1H), 6.06-6.42 (br, 2H), 8.08-8.34 (br, 2H)

[Industrial Applicability]

A compound represented by the Formula (III), a compound represented by the Formula (IV), a compound represented by the Formula (VI), a compound represented by the Formula (VIII), a compound represented by the Formula (IX), a compound represented by the Formula (X), a compound represented by the Formula (XI) and a compound represented by the Formula (XV) are useful as an intermediate to produce a compound represented by the Formula (XIII). Especially, a compound represented by the Formula (XI) is useful as an intermediate to produce a compound represented by the Formula (XIII).
The process of the present invention enables to produce with efficiency a compound represented by the Formula (XIII).

## Claims

**1.** A process for producing a compound represented by the Formula (VIII):

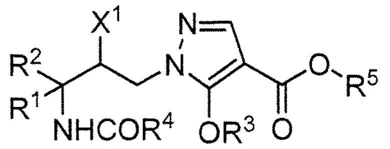

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $X^1$ is halogen;
which comprises reacting a compound represented by the Formula (VI):

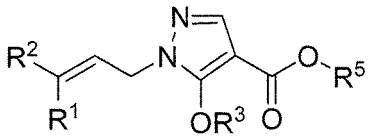

(VI)

wherein $R^1$, $R^2$, $R^3$ and $R^5$ are as defined above; with a compound represented by the Formula (VII): $R^4$-CN, wherein $R^4$ is as defined above; in the presence of a halogenating agent.

**2.** The process according to claim 1, wherein the halogenating agent is N-halogenosuccinimide, N-halogenoacetamide or halogen.

**3.** The process according to claim 2, wherein the halogenating agent is N-bromosuccinimide, N-bromoacetamide, N-chlorosuccinimide or I2.

**4.** The process according to any one of claims 1 to 3, wherein the reaction is performed in the presence of a Lewis acid.

**5.** The process according to claim 4, wherein the Lewis acid is boron trifluoride ether complex or metal halide.

**6.** The process according to claim 5, wherein the Lewis acid is boron trifluoride n-butyl ether complex, boron trifluoride diethyl ether complex or SnC14.

**7.** A process for producing a compound represented by the Formula (IX):

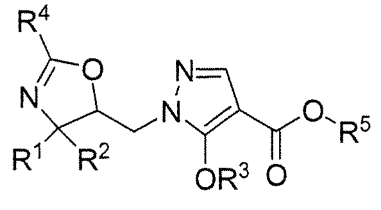

(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted

or unsubstituted heteroaryl or substituted or unsubstituted heterocyclyl; which comprises reacting a compound represented by the Formula (VIII):

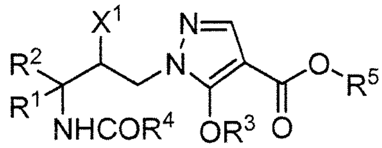

(VIII)

wherein $R^1$, $R^2$, $R^3$, $R^4$ and $R^5$ are as defined above and $X^1$ is halogen; with a base.

**8.** The process according to claim 7, wherein the base is an organic base.

**9.** The process for producing the compound represented by the Formula (IX) according to claim 7 or its salt, wherein the process comprises a process according to any one of claims 1 to 6.

**10.** A process for producing a compound represented by the Formula (X):

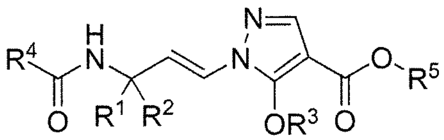

(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (IX):

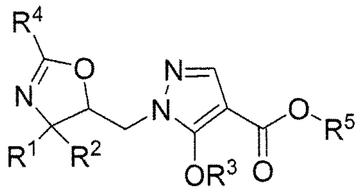

(IX)

wherein $R^1$, $R^2$, $R^3$, $R^4$ and $R^5$ are as defined above; with a base.

**11.** The process according to claim 10, wherein the base is a metal alkoxide.

**12.** The process for producing the compound represented by the Formula (X) according to claim 10 or its salt, wherein the process comprises a process according to any one of claims 1 to 9.

**13.** A process for producing a compound represented by the Formula (XI):

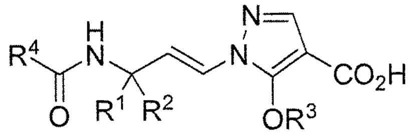 (XI)

or its salt, wherein and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (X):

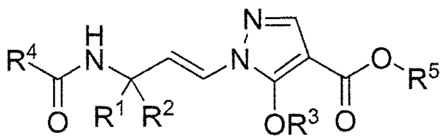 (X)

wherein $R^1$, $R^2$, $R^3$ and $R^4$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**14.** A process for producing a compound represented by the Formula (XV):

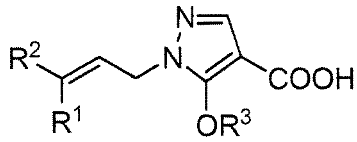 (XV)

or its salt, wherein and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises hydrolyzing a compound represented by the Formula (VI):

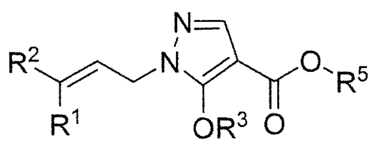 (VI)

wherein $R^1$, $R^2$ and $R^3$ are as defined above and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, provided that $R^3$ and $R^5$ are not the same group.

**15.** A process for producing a compound represented by the Formula (VI):

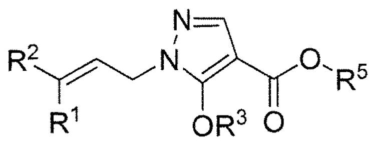

(VI)

or its salt, wherein and $R^2$ are each independently hydrogen or substituted or unsubstituted alkyl and $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (XV):

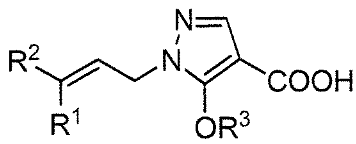

(XV)

wherein $R^1$, $R^2$ and $R^3$ are as defined above; with a compound represented by the Formula (XVII): $R^5$-OH, wherein $R^5$ is the same group as $R^3$.

16. The process according to claim 15, which comprises a step of producing a compound represented by the formula (XVI):

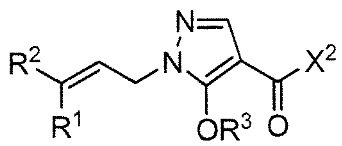

(XVI)

or its salt, wherein $R^1$, $R^2$ and $R^3$ are as defined in claim 15 and $X^2$ is halogen; by reacting a compound represented by the formula (XV):

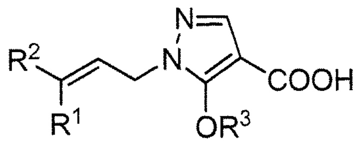

(XV)

wherein $R^1$, $R^2$ and $R^3$ are as defined above; with a halogenating agent.

17. The process according to claim 16, wherein the halogenating agent is selected from phosphorus oxyhalide, phosphorus pentahalide, oxalyl halide and thionyl halide.

18. The process for producing the compound represented by the Formula (VI) according to claim 15 or its salt, wherein the process comprises a process according to claim 14.

19. The process for producing the compound represented by the Formula (XI) according to claim 13 or its salt, wherein the process comprises a process according to any one of claims 1 to 18.

20. A process for producing a compound represented by the Formula (III):

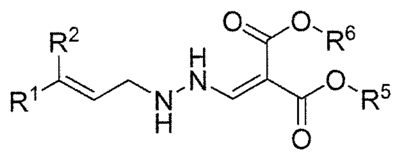

(III)

or its salt, wherein and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (I):

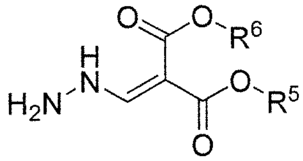

(I)

wherein $R^5$ and $R^6$ are as defined above; with a compound represented by the Formula (II):

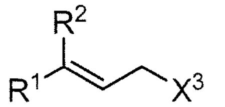

(II)

wherein and $R^2$ are as defined above and $X^3$ is a leaving group.

**21.** A process for producing a compound represented by the Formula (IV):

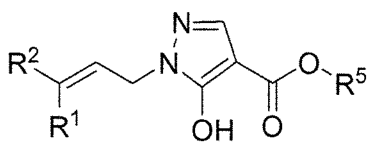

(IV)

or its salt, wherein and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (III):

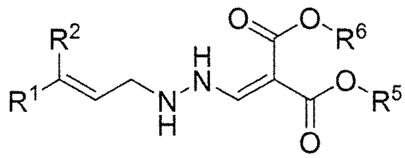

(III)

wherein $R^1$, $R^2$ and $R^5$ are as defined above and $R^6$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted

heterocyclyl; with a base.

**22.** The process for producing the compound represented by the Formula (IV) according to claim 21 or its salt, wherein the process comprises a process according to claim 20.

**23.** A process for producing a compound represented by the Formula (VI):

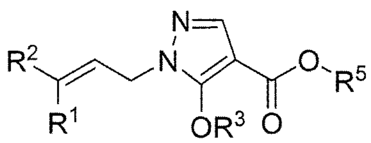

(VI)

or its salt, wherein and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
which comprises reacting a compound represented by the Formula (IV):

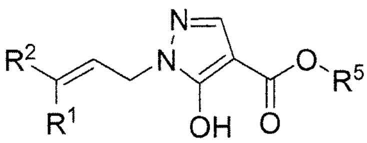

(IV)

wherein $R^1$, $R^2$ and $R^5$ are as defined above; with a compound represented by the Formula (V): $R^3$-$X^4$, wherein $R^3$ is as defined above and $X^4$ is a leaving group; in the presence of a base.

**24.** The process for producing the compound represented by the Formula (VI) according to claim 23 or its salt, wherein the process comprises a process according to any one of claims 20 to 22.

**25.** The process according to claim 19, wherein the process comprises a process according to claim 24.

**26.** The process according to any one of claims 1 to 25, wherein $R^1$ and $R^2$ are substituted or unsubstituted alkyl.

**27.** The process according to any one of claims 1 to 19 or 23 to 25, wherein $R^3$ is substituted or unsubstituted alkyl.

**28.** The process according to any one of claims 1 to 19 or 23 to 25, wherein $R^3$ and $R^5$ are the same substituted or unsubstituted alkyl.

**29.** A process for producing a compound represented by the Formula (XIII):

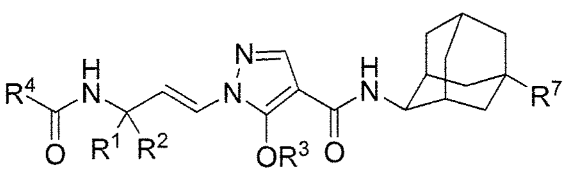

(XIII)

or its salt, wherein $R^1$, $R^2$, $R^3$ and $R^4$ are as defined in claim 13, $R^7$ is
a group represented by the Formula: -$OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or

unsubstituted cycloalkenyl, substituted or unsubstituted aryl, substituted or unsubstituted heteroaryl or substituted or unsubstituted heterocyclyl;
a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or
a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;
wherein the process comprises a process according to any one of claims 1 to 28.

**30.** A process for producing a compound represented by the Formula (XIII):

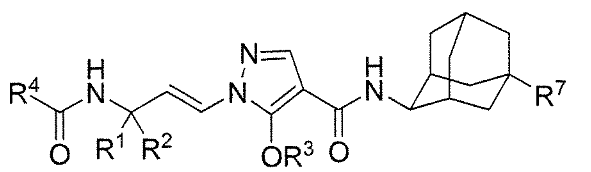
(XIII)

or its salt, wherein $R^1$, $R^2$, $R^3$ and $R^4$ are as defined in claim 13, $R^7$ is
a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;
a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or
a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;
which comprises obtaining a compound represented by the Formula (XI):

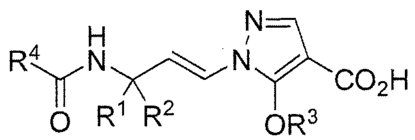
(XI)

wherein $R^1$, $R^2$, $R^3$ and $R^4$ are as defined above; by the process according to any one of claims 1 to 28, followed by reacting the obtained compound represented by the Formula (XI) with a compound represented by the Formula (XII):

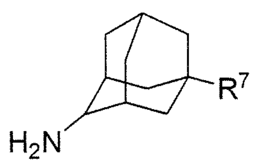
(XII)

wherein $R^7$ is as defined above.

**31.** The process according to claim 30, wherein the reaction is performed in the presence of a condensing agent.

**32.** The process according to claim 31, wherein the condensing agent is one or more condensing agent(s) selected from N,N'-dicyclohexylcarbodiimide, N,N'-diisopropylcarbodiimide and 1-ethyl-3-(3-dimethylamino propyl) carbodiimide hydrochloride.

**33.** The process according to claim 31 or 32, wherein the reaction is performed in the presence of one or more additive agent(s) selected from 1-hydroxybenzotriazole and N-hydroxy succinimide.

**34.** The process according to claim 30, which comprises a step of producing a compound represented by the formula (XIV):

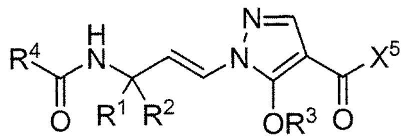

(XIV)

or its salt, wherein $R^1$, $R^2$, $R^3$ and $R^4$ are as defined in claim 13 and $X^5$ is halogen; by reacting a compound represented by the formula (XI):

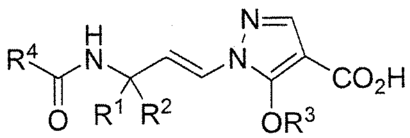

(XI)

wherein $R^1$, $R^2$, $R^3$ and $R^4$ are as defined above; with a halogenating agent.

**35.** The process according to claim 34, wherein the halogenating agent is selected from phosphorus oxyhalide, phosphorus pentahalide, oxalyl halide and thionyl halide.

**36.** A compound represented by the Formula (III):

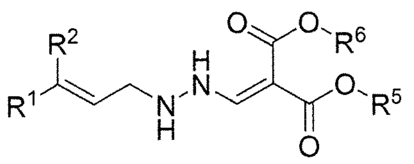

(III)

or its salt, wherein and $R^2$ are each independently hydrogen or substituted or unsubstituted alkyl, and $R^5$ and $R^6$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**37.** A compound represented by the Formula (IV):

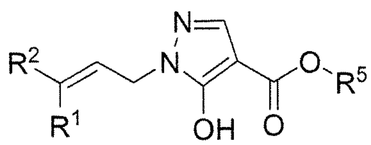

(IV)

or its salt, wherein $R^1$ and $R^2$ are each independently hydrogen or substituted or unsubstituted alkyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**38.** A compound represented by the Formula (VI):

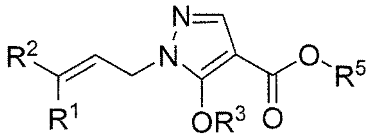

(VI)

or its salt, wherein and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^5$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**39.** A compound represented by the Formula (VIII):

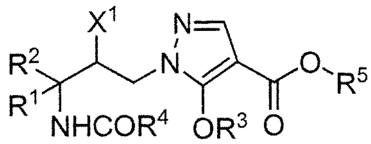

(VIII)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $X^1$ is halogen.

**40.** A compound represented by the Formula (IX):

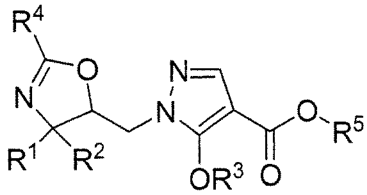

(IX)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**41.** A compound represented by the Formula (X):

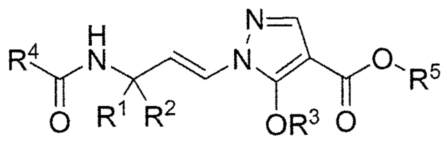

(X)

or its salt, wherein $R^1$ and $R^2$ are each independently hydrogen or substituted or unsubstituted alkyl, $R^3$ and $R^5$ are each independently substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

**42.** A compound represented by the Formula (XI):

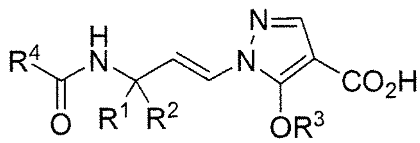

(XI)

or its salt, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, with the proviso that compounds wherein $R^3$ are isobutyl are excluded.

**43.** A compound represented by the Formula (XV):

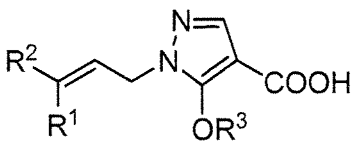

(XV)

or its salt, wherein $R^1$ and $R^2$ are each independently hydrogen or substituted or unsubstituted alkyl and $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl.

**44.** A process for producing a crystal of a non-solvate of a compound represented by the Formula (III):

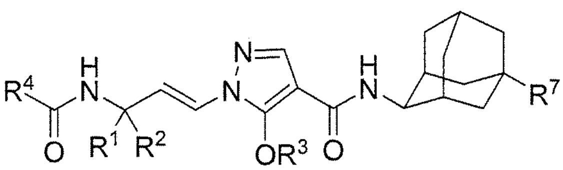

(XIII)

or a crystal of a non-solvate of a salt of the compound, wherein $R^1$ and $R^2$ are each independently hydrogen or

substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

which comprises crystallizing a compound represented by the Formula (XIII):

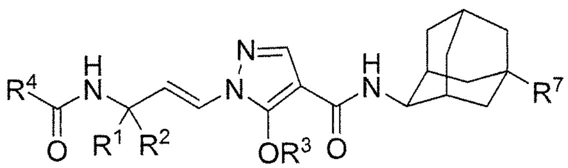

(XIII)

or its salt, wherein $R^1$, $R^2$, $R^3$ , $R^4$ and $R^7$ are as defined above; by using a solvent that is substantially free of methanol.

**45.** A process for producing a crystal of a non-solvate of a compound represented by the Formula (XIII):

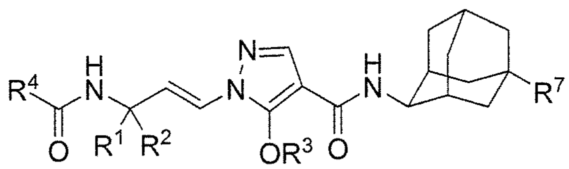

(XIII)

or a crystal of a non-solvate of a salt of the compound, wherein $R^1$ and $R^2$ are each independently hydrogen or substituted or unsubstituted alkyl, $R^3$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl, $R^4$ is substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl and is

a group represented by the Formula: $-OR^8$, wherein $R^8$ is hydrogen, substituted or unsubstituted alkyl, substituted or unsubstituted alkenyl, substituted or unsubstituted alkynyl, substituted or unsubstituted cycloalkyl, substituted or unsubstituted cycloalkenyl, substituted or unsubstituted aryl, substituted or unsubstituted heteroaryl or substituted or unsubstituted heterocyclyl;

a group represented by the Formula: $-(CR^9R^{10})m-NR^{11}-R^{12}$, wherein $R^{12}$ is substituted or unsubstituted acyl, substituted or unsubstituted alkylsulfonyl, substituted or unsubstituted alkyloxycarbonyl, substituted or unsubstituted carbamoyl or substituted or unsubstituted sulfamoyl, $R^{11}$ is hydrogen or substituted or unsubstituted alkyl, $R^9$ are each independently hydrogen, substituted or unsubstituted alkyl or halogen, $R^{10}$ are each independently hydrogen, substituted or unsubstituted alkyl or halogen and m is an integer of 0 to 3; or

a group represented by the Formula: $-(CR^9R^{10})m-O-C(O)-NR^{13}-R^{14}$, wherein $R^{13}$ and $R^{14}$ are each independently hydrogen or substituted or unsubstituted alkyl, and $R^9$, $R^{10}$ and m are as defined above;

which comprises transforming a crystal of a methanol solvate of a compound represented by the Formula (XIII):

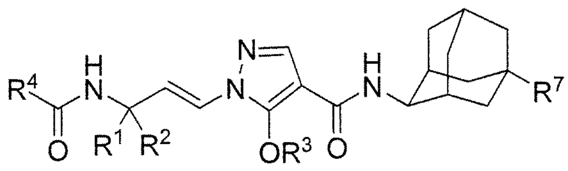

(XIII)

or a crystal of a methanol solvate of a salt of the compound, wherein $R^1$, $R^2$, $R^3$ , $R^4$ and $R^7$ are as defined above; by using a solvent that is free of methanol of more than 30%(V/V).

[Fig. 1]

## Raw data

| | | | | | |
|---|---|---|---|---|---|
| X ray | : Cu / 50 kV / 300mA | | | | |
| Goniometer | : TTRIII horizontal goniometer (Ts axis manual) | | | | |
| Attachment | : 6 sample changer | | | | |
| Sample number | : 1 | | | | |
| Sample rotation | : rotation | rate of rotation | : 120.000 rpm | | |
| Filter | : nonuse | Divergence slit | : 1.00 mm | First slit | : none |
| Incident monochro | : use | Divergence vertical limiting slit | : 10 mm | Second slit | : none |
| Counter-monochrometer | : nonuse | Scattering slit | : 1 mm | Entrance slit | : none |
| Counter | : scintillation counter | Light-receiving slit | : open | Light-receiving slit | : none |
| Scanning mode | : continuous | Scanning speed | : 5.000°/min | Sampling rage | : 0.0200° |
| Scan axis | : 2θ/θ | Scanning rage | : 4.0000 ～ 40.0000° | θ offset | : 0.0000° |
| Number of times of integrations : | 1 | | | | |

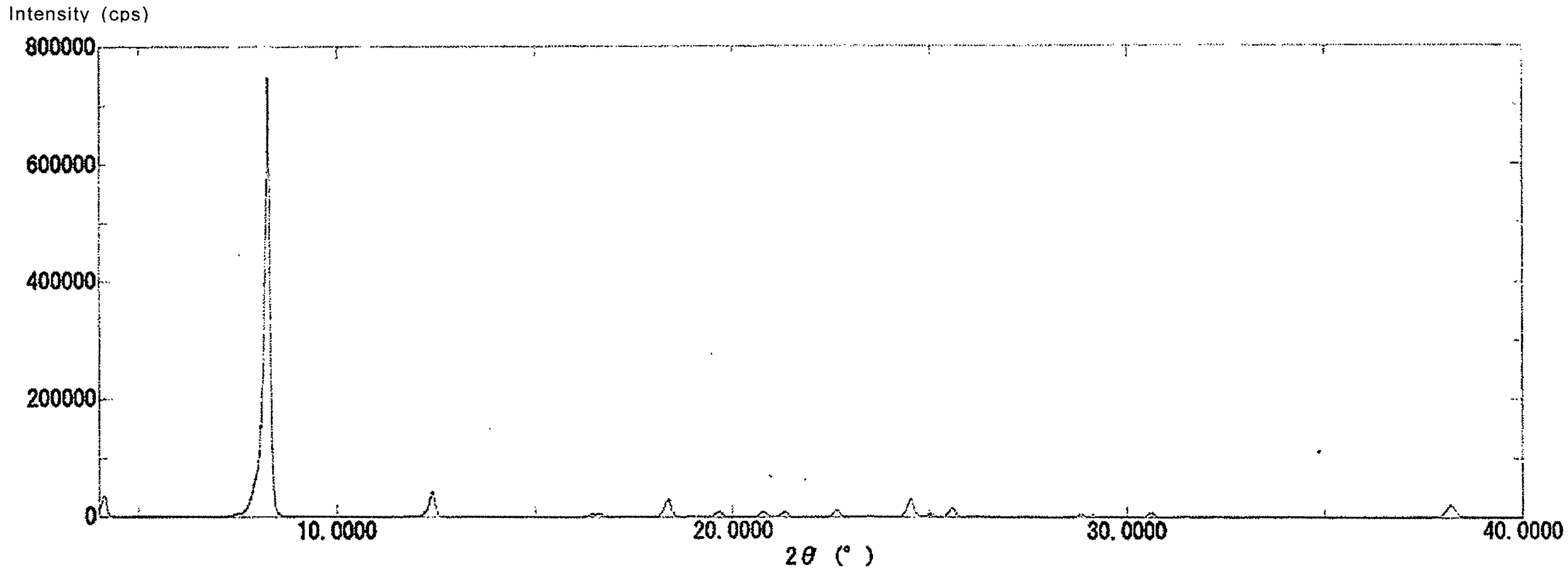

[Fig. 2]

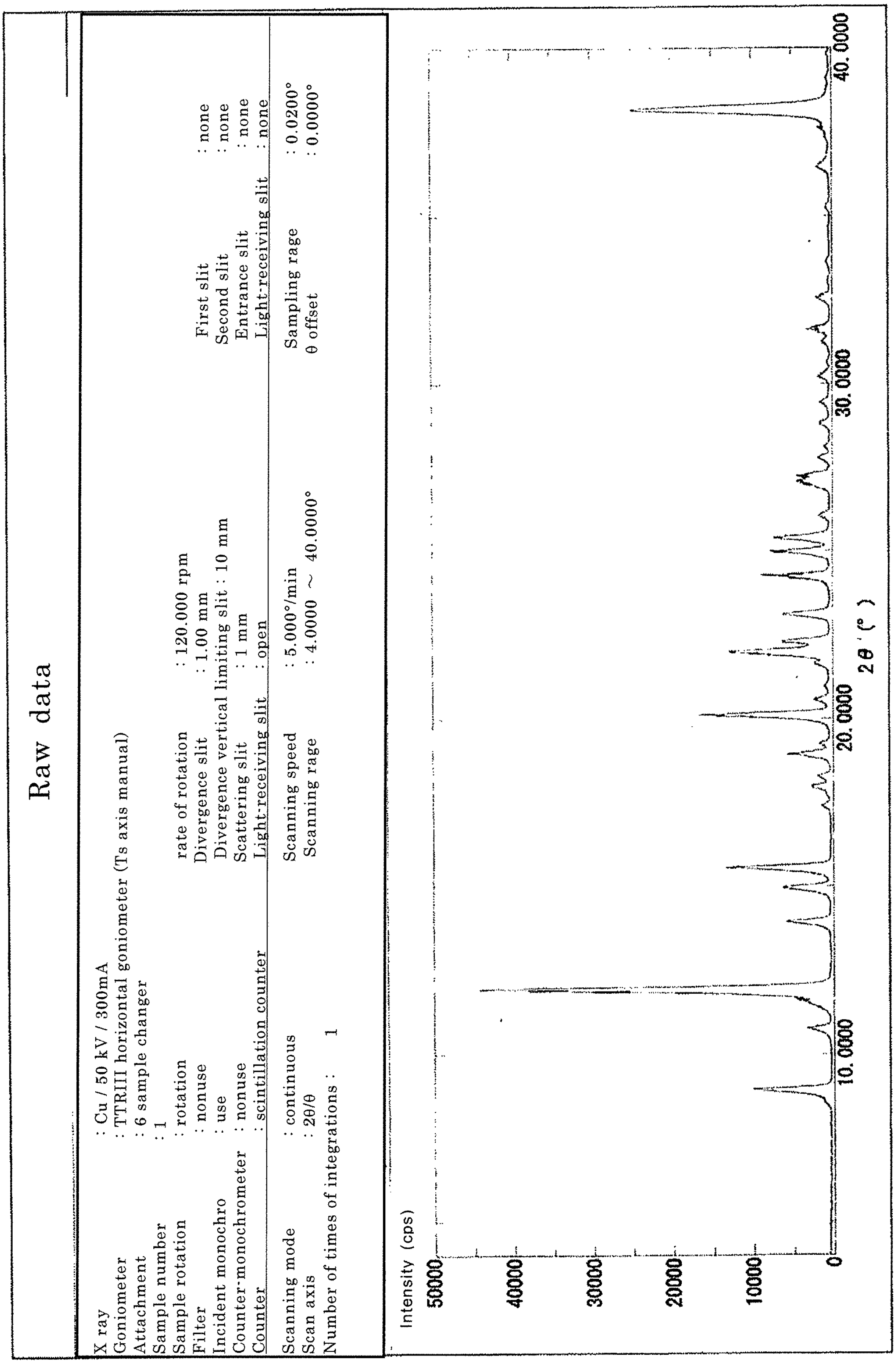
Raw data
X ray : Cu / 50 kV / 300mA
Goniometer : TTRIII horizontal goniometer (Ts axis manual)
Attachment : 6 sample changer
Sample number : 1
Sample rotation : rotation
rate of rotation : 120.000 rpm
Filter : nonuse
Divergence slit : 1.00 mm
First slit : none
Incident monochro : use
Divergence vertical limiting slit : 10 mm
Second slit : none
Counter-monochrometer : nonuse
Scattering slit : 1 mm
Entrance slit : none
Counter : scintillation counter
Light-receiving slit : open
Light-receiving slit : none
Scanning mode : continuous
Scanning speed : 5.000°/min
Sampling rage : 0.0200°
Scan axis : 2θ/θ
Scanning rage : 4.0000 ~ 40.0000°
θ offset : 0.0000°
Number of times of integrations : 1
Intensity (cps)
50000
40000
30000
20000
10000
0
10.0000
20.0000
30.0000
40.0000
2θ (°)

[Fig. 3]

## Raw data

| | | | | | |
|---|---|---|---|---|---|
| X ray | : Cu / 50 kV / 300mA | | | | |
| Goniometer | : TTRIII horizontal goniometer (Ts axis manual) | | | | |
| Attachment | : 6 sample changer | | | | |
| Sample number | : 2 | | | | |
| Sample rotation | : rotation | rate of rotation | : 120.000 rpm | | |
| Filter | : nonuse | Divergence slit | : 1.00 mm | First slit | : none |
| Incident monochro | : use | Divergence vertical limiting slit | : 10 mm | Second slit | : none |
| Counter-monochrometer | : nonuse | Scattering slit | : 1 mm | Entrance slit | : none |
| Counter | : scintillation counter | Light-receiving slit | : open | Light-receiving slit | : none |
| Scanning mode | : continuous | Scanning speed | : 5.000°/min | Sampling rage | : 0.0200° |
| Scan axis | : 2θ/θ | Scanning rage | : 4.0000 ～ 40.0000° | θ offset | : 0.0000° |
| Number of times of integrations : | 1 | | | | |

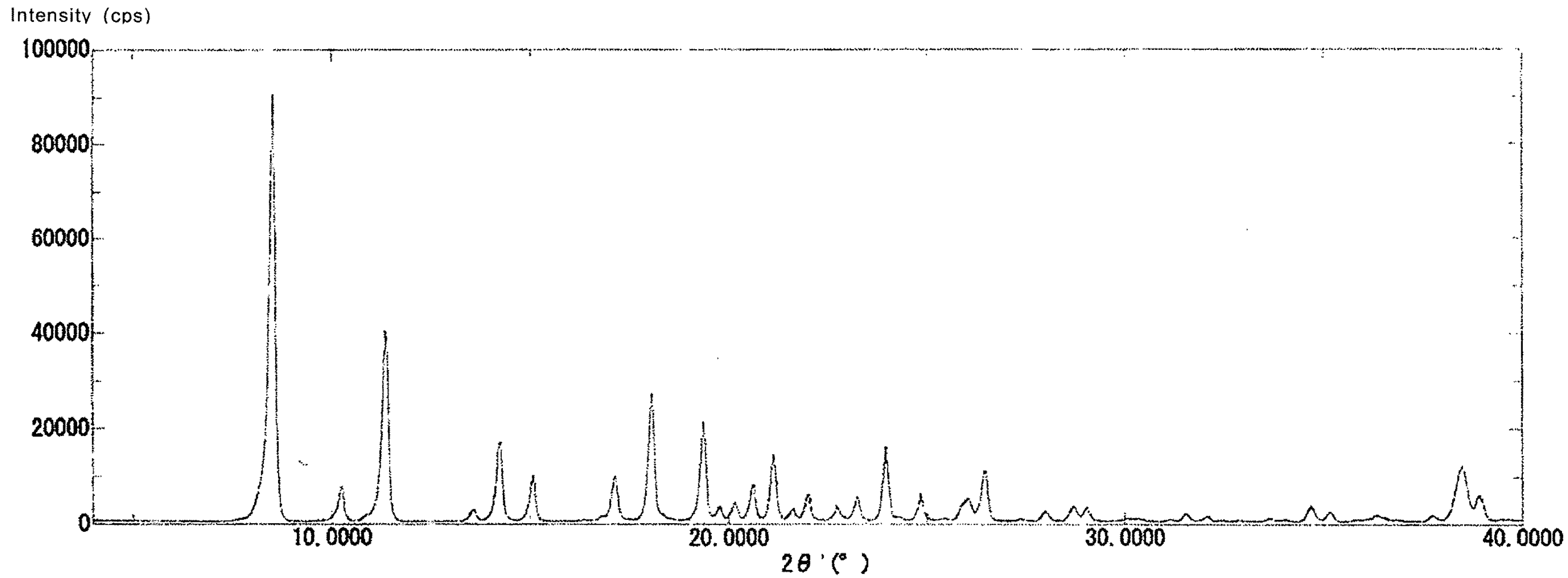

[Fig. 4]

## Raw data

| | | | | | |
|---|---|---|---|---|---|
| X ray | : Cu / 50 kV / 300mA | | | | |
| Goniometer | : TTRIII horizontal goniometer (Ts axis manual) | | | | |
| Attachment | : 6 sample changer | | | | |
| Sample number | : 3 | | | | |
| Sample rotation | : rotation | rate of rotation | : 120.000 rpm | | |
| Filter | : nonuse | Divergence slit | : 1.00 mm | First slit | : none |
| Incident monochro | : use | Divergence vertical limiting slit | : 10 mm | Second slit | : none |
| Counter-monochrometer | : nonuse | Scattering slit | : 1 mm | Entrance slit | : none |
| Counter | : scintillation counter | Light-receiving slit | : open | Light-receiving slit | : none |
| Scanning mode | : continuous | Scanning speed | : 5.000°/min | Sampling rage | : 0.0200° |
| Scan axis | : 2θ/θ | Scanning rage | : 4.0000 ~ 40.0000° | θ offset | : 0.0000° |
| Number of times of integrations : | 1 | | | | |

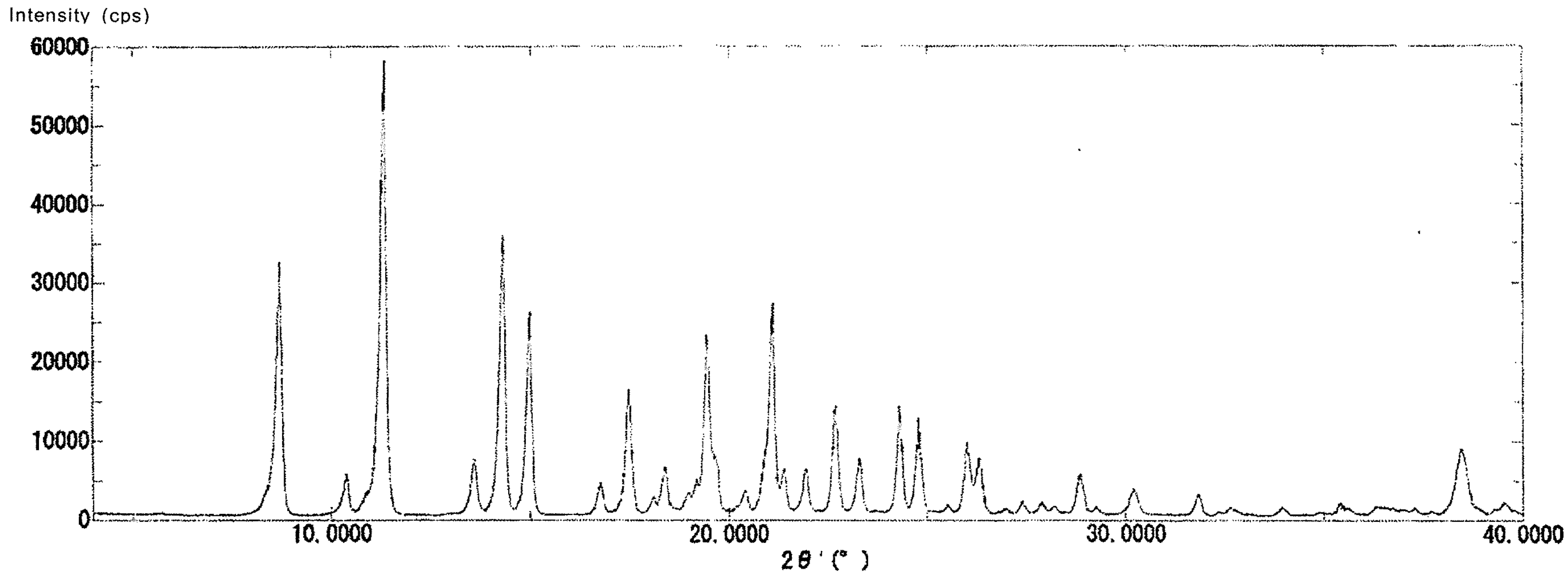

[Fig. 5]

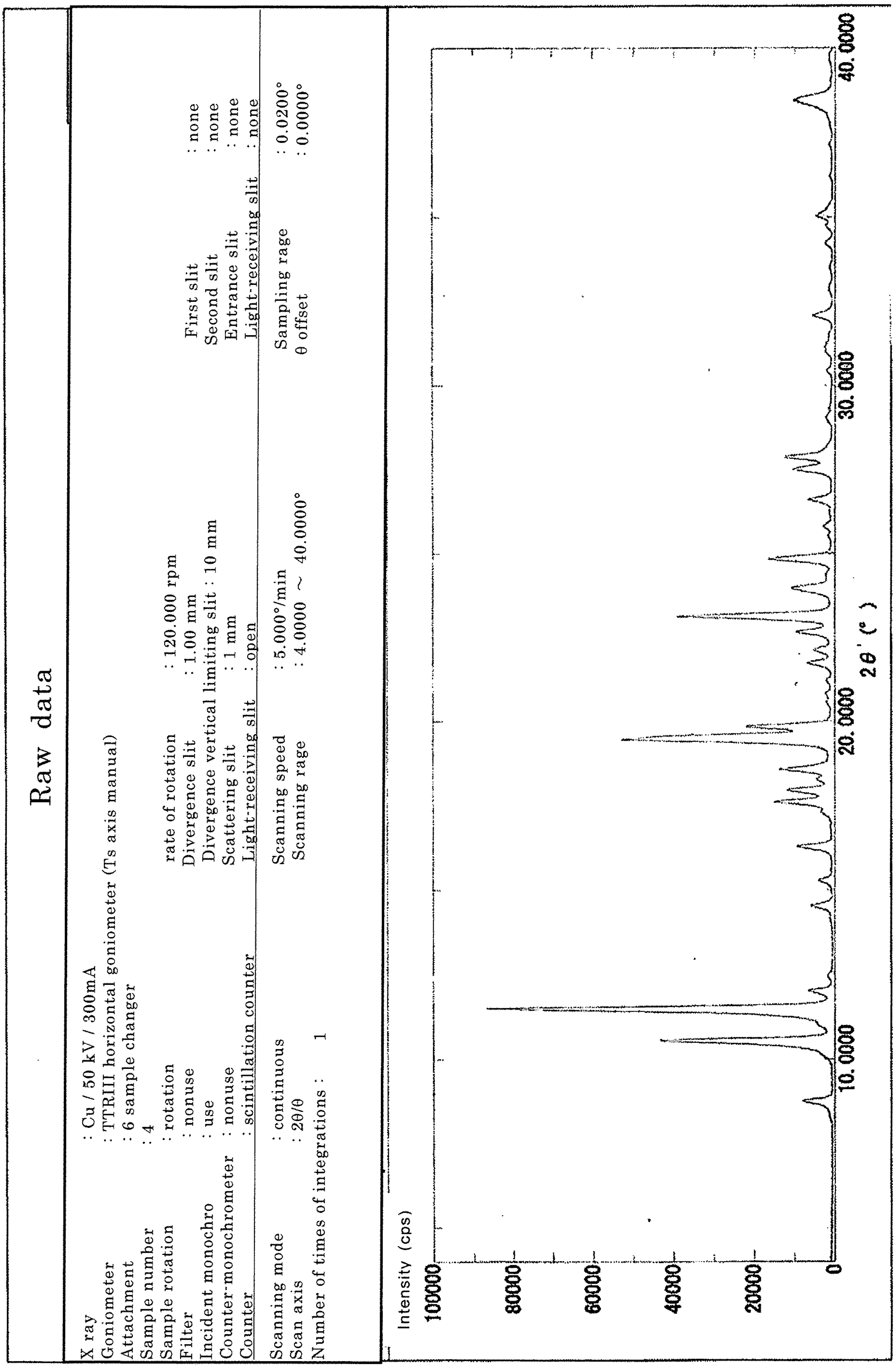
Raw data
X ray : Cu / 50 kV / 300mA
Goniometer : TTRIII horizontal goniometer (Ts axis manual)
Attachment : 6 sample changer
Sample number : 4
Sample rotation : rotation
rate of rotation : 120.000 rpm
Filter : nonuse
Divergence slit : 1.00 mm
First slit : none
Incident monochro : use
Divergence vertical limiting slit : 10 mm
Second slit : none
Counter-monochrometer : nonuse
Scattering slit : 1 mm
Entrance slit : none
Counter : scintillation counter
Light-receiving slit : open
Light-receiving slit : none
Scanning mode : continuous
Scanning speed : 5.000°/min
Sampling rage : 0.0200°
Scan axis : 2θ/θ
Scanning rage : 4.0000 ~ 40.0000°
θ offset : 0.0000°
Number of times of integrations : 1
Intensity (cps)
100000
80000
60000
40000
20000
0
10.0000
20.0000
30.0000
40.0000
2θ (°)

[Fig. 6]

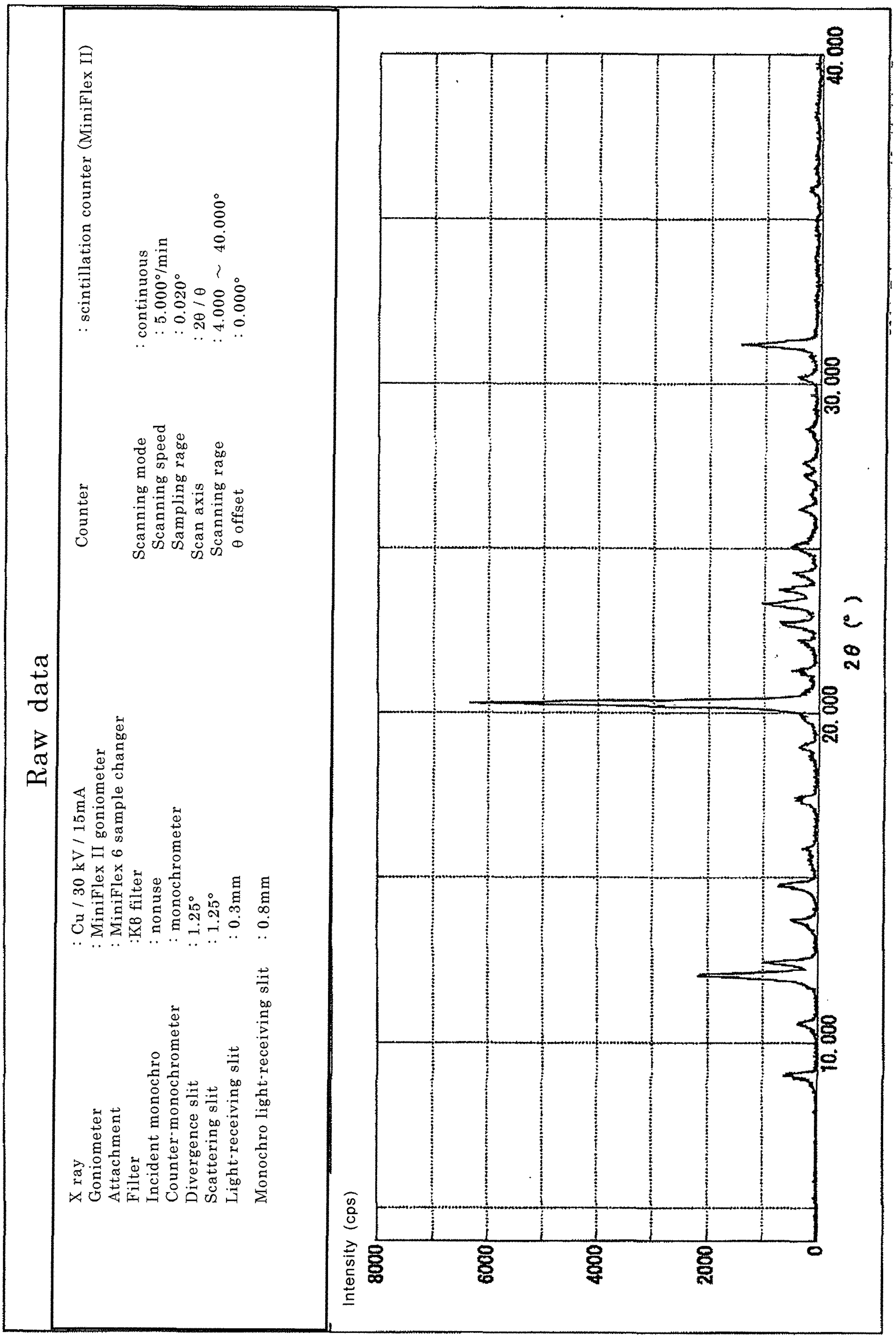
Raw data
X ray : Cu / 30 kV / 15mA
Goniometer : MiniFlex II goniometer
Attachment : MiniFlex 6 sample changer
Filter : Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 5.000°/min
Sampling rage : 0.020°
Scan axis : 2θ / θ
Scanning rage : 4.000 ~ 40.000°
θ offset : 0.000°
Intensity (cps)
8000
6000
4000
2000
0
10.000
20.000
30.000
40.000
2θ (°)

[Fig. 7]

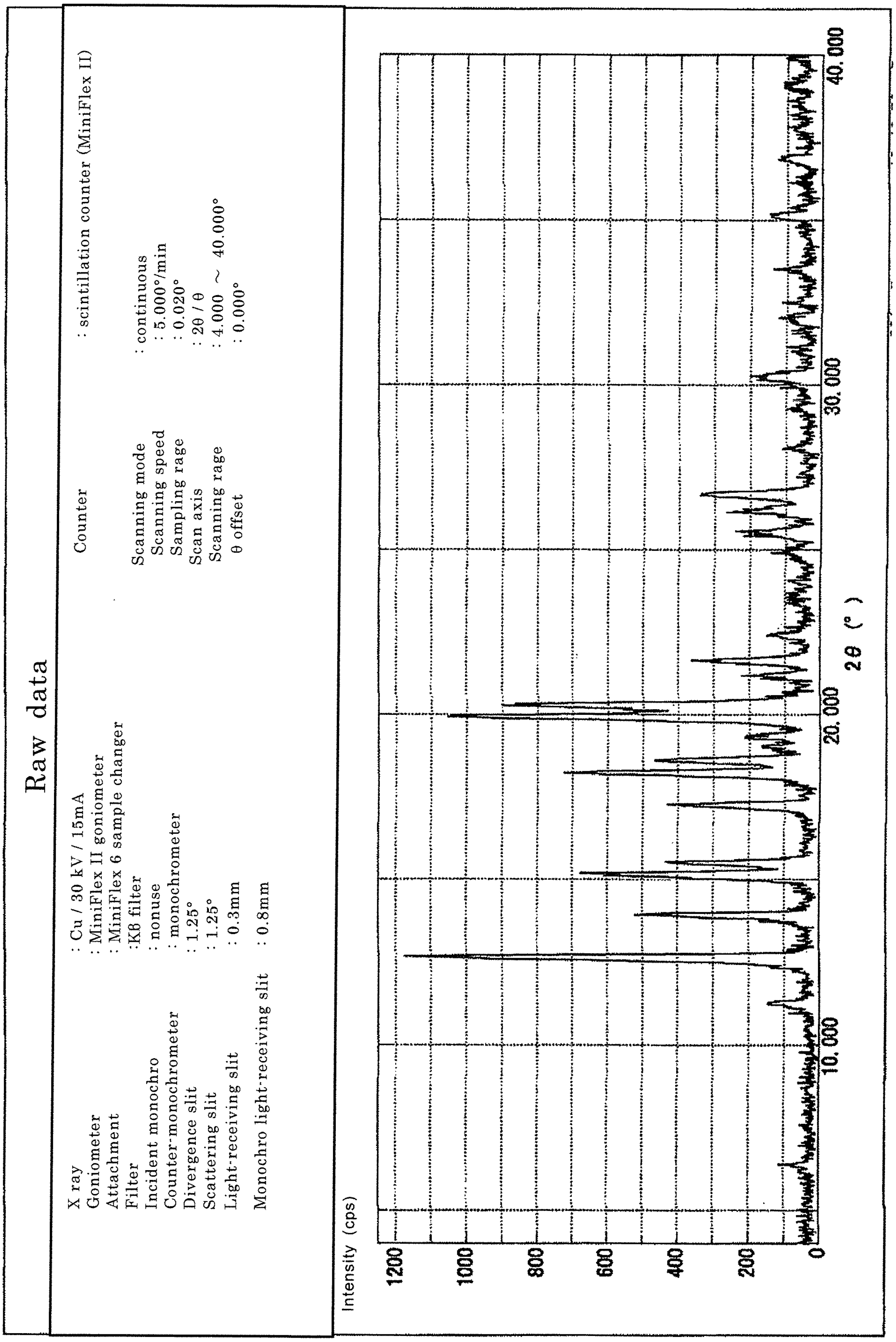
Raw data
X ray : Cu / 30 kV / 15mA
Goniometer : MiniFlex II goniometer
Attachment : MiniFlex 6 sample changer
Filter :Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 5.000°/min
Sampling rage : 0.020°
Scan axis : 2θ / θ
Scanning rage : 4.000 ~ 40.000°
θ offset : 0.000°
Intensity (cps)
0
200
400
600
800
1000
1200
10.000
20.000
30.000
40.000
2θ (°)

[Fig. 8]

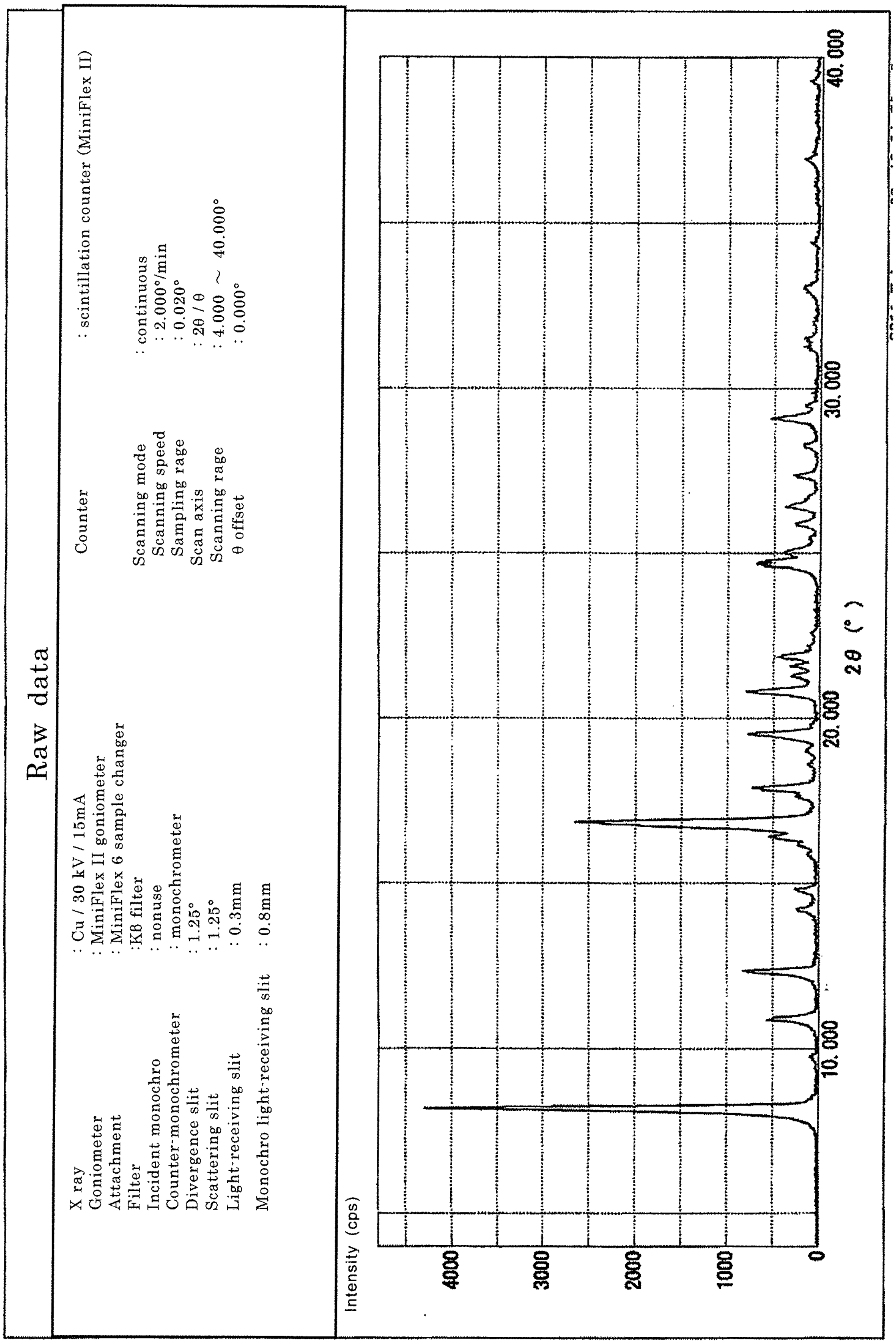
Raw data
X ray : Cu / 30 kV / 15mA
Goniometer : MiniFlex II goniometer
Attachment : MiniFlex 6 sample changer
Filter :Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 2.000°/min
Sampling rage : 0.020°
Scan axis : 2θ / θ
Scanning rage : 4.000 ~ 40.000°
θ offset : 0.000°
Intensity (cps)
4000
3000
2000
1000
0
10.000
20.000
30.000
40.000
2θ (°)

[Fig. 9]

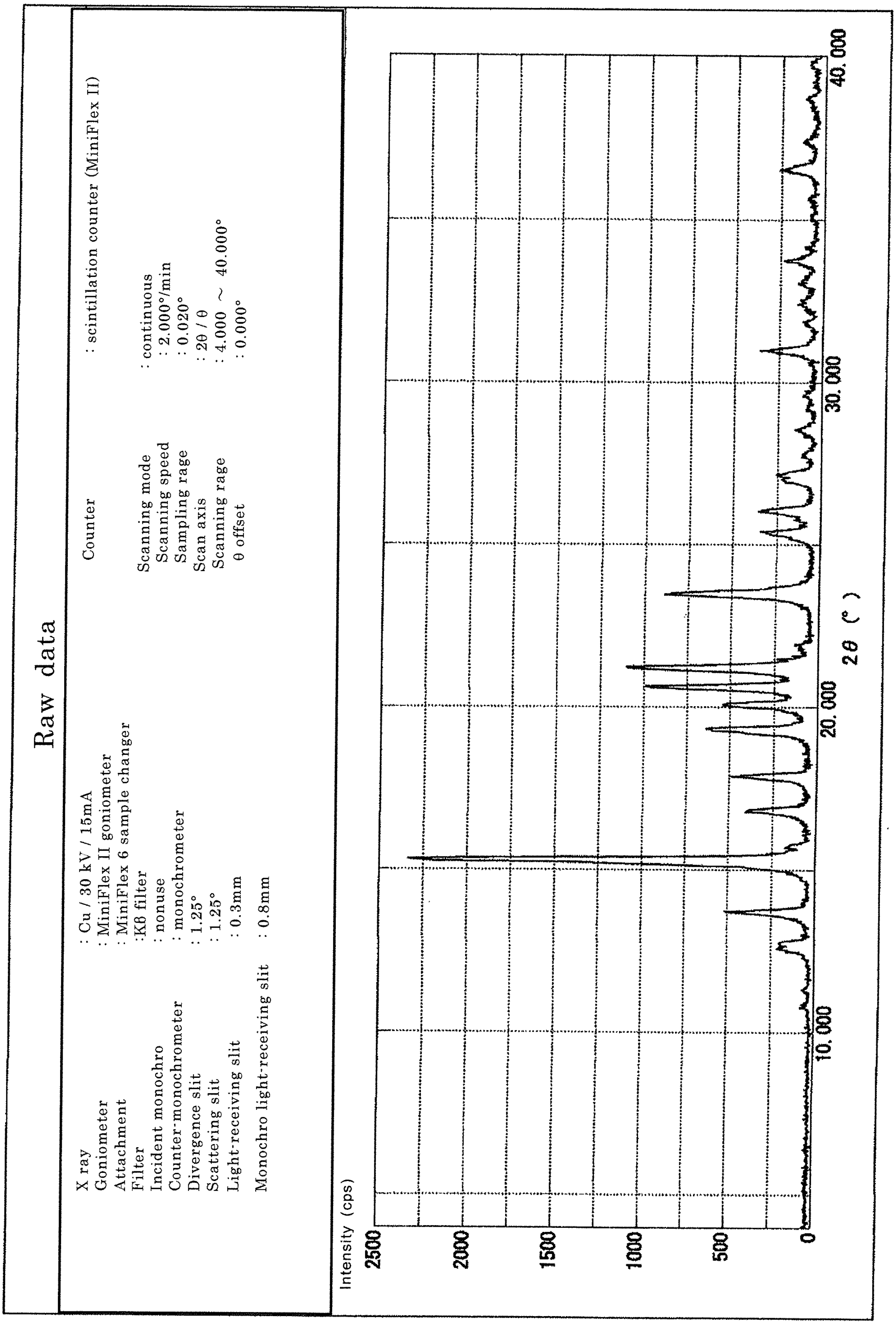

Raw data
X ray : Cu / 30 kV / 15mA
Goniometer : MiniFlex II goniometer
Attachment : MiniFlex 6 sample changer
Filter :Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 2.000°/min
Sampling rage : 0.020°
Scan axis : 2θ / θ
Scanning rage : 4.000 ~ 40.000°
θ offset : 0.000°
Intensity (cps)
2500
2000
1500
1000
500
0
10.000
20.000
30.000
40.000
2θ (°)

[Fig. 10]

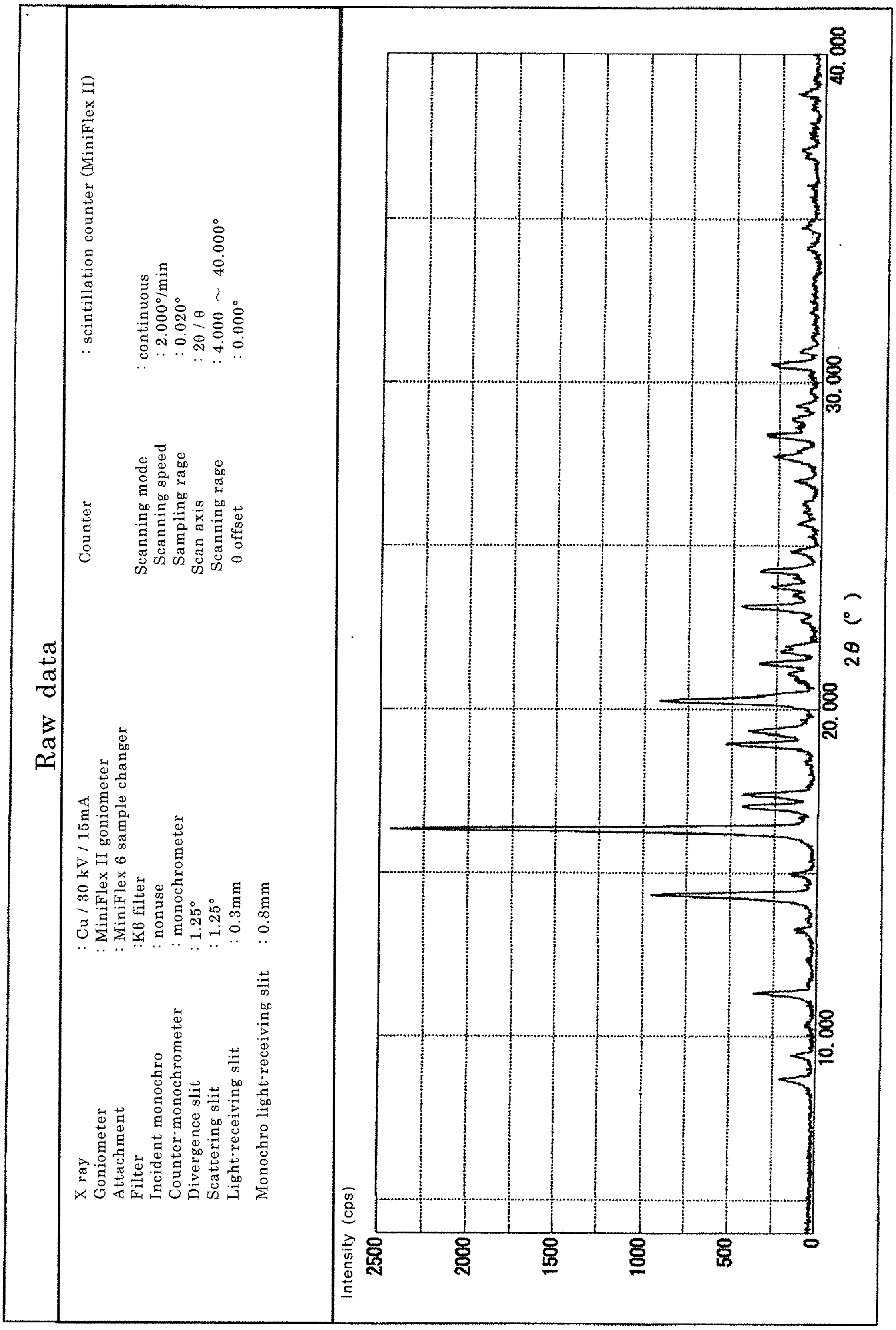
Raw data
X ray : Cu / 30 kV / 15mA
Goniometer : MiniFlex II goniometer
Attachment : MiniFlex 6 sample changer
Filter :Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 2.000°/min
Sampling rage : 0.020°
Scan axis : 2θ / θ
Scanning rage : 4.000 ~ 40.000°
θ offset : 0.000°
Intensity (cps)
2500
2000
1500
1000
500
0
10.000
20.000
30.000
40.000
2θ (°)

[Fig. 11]

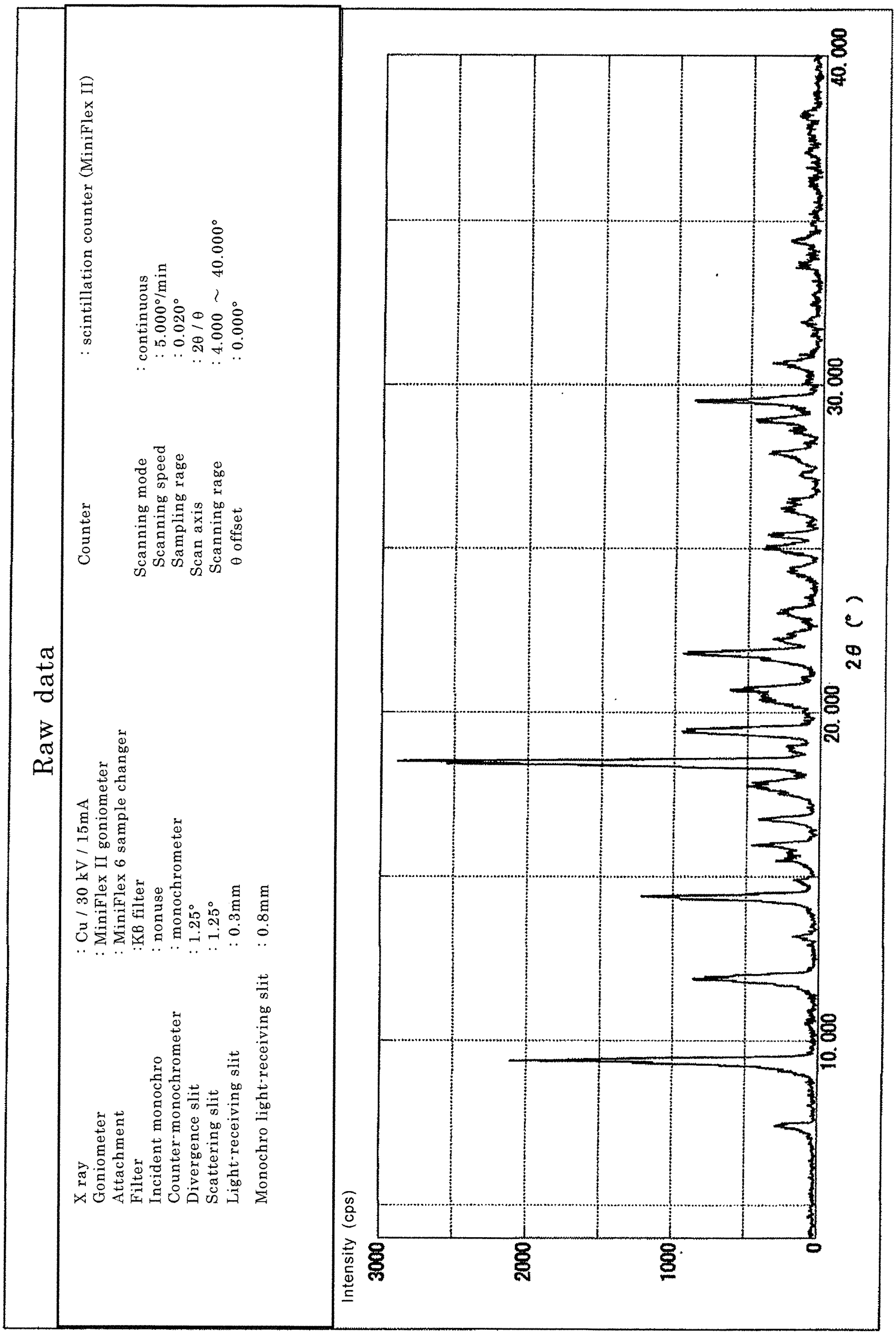
Raw data
X ray : Cu / 30 kV / 15mA
Goniometer : MiniFlex II goniometer
Attachment : MiniFlex 6 sample changer
Filter :Kβ filter
Incident monochro : nonuse
Counter-monochrometer : monochrometer
Divergence slit : 1.25°
Scattering slit : 1.25°
Light-receiving slit : 0.3mm
Monochro light-receiving slit : 0.8mm
Counter : scintillation counter (MiniFlex II)
Scanning mode : continuous
Scanning speed : 5.000°/min
Sampling rage : 0.020°
Scan axis : 2θ / θ
Scanning rage : 4.000 ~ 40.000°
θ offset : 0.000°
Intensity (cps)
3000
2000
1000
0
10.000
20.000
30.000
40.000
2θ (°)

## INTERNATIONAL SEARCH REPORT

International application No.
PCT/JP2012/056763

A. CLASSIFICATION OF SUBJECT MATTER
*C07D231/20*(2006.01)i, *C07C241/02*(2006.01)i, *C07C243/16*(2006.01)i

According to International Patent Classification (IPC) or to both national classification and IPC

B. FIELDS SEARCHED

Minimum documentation searched (classification system followed by classification symbols)
C07D231/20, C07C241/02, C07C243/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X/Y/A | WO 2007/058346 A1 (Shionogi & Co., Ltd.), 24 May 2007 (24.05.2007), example 30 & JP 4276280 B & US 2009/0170832 A1 & EP 1953145 A1 & KR 10-2008-0067353 A & CN 101312951 A | 13,19,26-33/ 44,45/1-12, 14-18,20-25, 34-43 |
| Y | WO 2008/142986 A1 (Shionogi & Co., Ltd.), 27 November 2008 (27.11.2008), entire text & JP 4671369 B & US 2010/0240659 A1 & EP 2163543 A1 & CN 101754954 A & KR 10-2010-0008382 A | 44,45 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 May, 2012 (07.05.12) | 22 May, 2012 (22.05.12) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2012/056763

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2006/131338 A1 (LEK PHARMACEUTICALS D. D.), 14 December 2006 (14.12.2006), pages 6 to 7 & JP 2008-545768 A & US 2009/0221646 A1 & EP 1907375 A | 44,45 |
| P,A | WO 2011/078101 A1 (Shionogi & Co., Ltd.), 30 June 2011 (30.06.2011), (Family: none) | 1-45 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007058346 A
- WO 2008142986 A
- WO 2011078101 A
- WO 2012020724 A
- WO 201107810 A
- WO 2007114125 A


### Non-patent literature cited in the description


- *Journal of the American Chemical Society,* 2006, vol. 128, 9644-9645
- **ALAN R. KATRISZLY et al.** *Comprehensive Heterocyclic Chemistry*
- **ALAN R. KATRISZLY et al.** *Comprehensive Heterocyclic Chemistry II*
- *RODD'S CHEMISTRY OF CARBON COMPOUNDS VOLUME IV HETEROCYCLIC COMPOUNDS,* vol. IV